(19) Europäisches Patentamt
European Patent Office
Office européen des brevets

(11) Numéro de publication: **0 360 701**
**A1**

(12) # DEMANDE DE BREVET EUROPEEN

(21) Numéro de dépôt: **89420320.7**

(22) Date de dépôt: **31.08.89**

(51) Int. Cl.5: **C 07 D 333/70**
C 07 D 307/85,
C 07 D 209/42,
C 07 D 213/78,
C 07 D 213/80,
C 07 D 213/82,
C 07 D 307/68,
C 07 D 207/34,
C 07 D 231/14,
C 07 D 239/28, C 07 D 295/18

(30) Priorité: **01.09.88 FR 8811665**
**25.04.89 FR 8905774**
**03.07.89 FR 8909150**
**13.07.89 FR 8909742**

(43) Date de publication de la demande:
**28.03.90 Bulletin 90/13**

(84) Etats contractants désignés:
**AT BE CH DE ES FR GB GR IT LI LU NL SE**

(71) Demandeur: **RHONE POULENC AGROCHIMIE**
**14-20 rue Pierre-Baizet**
**F-69009 Lyon (FR)**

(72) Inventeur: **Pepin, Régis**
**27 Montée Castellane**
**F-69140 Rilleux La Pape (FR)**

**Schmitz, Christian**
**Chemin de St Trys Pommiers**
**F-69480 Anse (FR)**

**Lacroix, Guy Bernard**
**332F Balmont La Duchère**
**F-69009 Lyon (FR)**

**Dellis, Philippe**
**14 Chemin des Rivières**
**F-69370 St Didier au Mont d'Or (FR)**

**Veyrat, Christine**
**21 Rue Nervieux**
**F-69450 St Cyr au Mont d'Or (FR)**

(74) Mandataire: **Brachotte, Charles et al**
**RHONE-POULENC AGROCHIMIE P.I.D. BP 9163**
**F-69263 Lyon Cedex 09 (FR)**

(54) **Antifongiques à base d'amides à groupe phényl.**

(57) Composés, utilisables notamment pour le traitement des atteintes des plantes, caractérisés en ce qu'ils ont la formule (I) dans laquelle :
* Le signe CYCLE entouré d'un demi-cercle, représente, avec les deux atomes de carbone auxquels ce demi-cercle est relié, un cycle carboné ou hétérocyclique comportant un moins une liaison insaturée à caractère éthylènique ou aromatique entre les 2 atomes de carbone porteurs du groupe
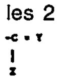
et du groupe phényle,
* Y est un atome d'oxygène ou de soufre.
* Z est un groupe $NR_1$ $R_2$.
* $R_1$ et $R_2$, identiques ou différents représentent :
- un radical alkyle ou alkoxy, ou cycloalkyle, ces radicaux étant éventuellement substitués
- un radical alkényle ou alkynyle .
* ou bien $R_1$ et $R_2$ forment ensemble, avec l'atome d'azote auquel ils sont rattachés, un hétérocycle, saturé ou insaturé,
* $R_3$, $R_4$ et $R_5$ identiques ou différents, représentent :
* un atome d'hydrogène ou d'halogène, l'un au moins de ces radicaux $R_3$, $R_4$ et $R_5$ étant autre qu'un atome d'hydrogène ou d'halogène
* un groupe amino éventuellement substitué
* un groupe alkyle ou alkoxy ou alkyl alkoxy ou alkylthio, éventuellement halogéné ou hydroxylé
* $R_3$ et $R_4$ (en position meta et para) peuvent former ensemble un radical unique divalent à 3 ou 4 chaînons comprenant 1 ou 2 atomes d'oxygène non vicinaux.
Les compositions contenant ces composés et leur utilisation comme fongicides.

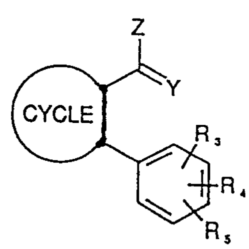

Formule I

EP 0 360 701 A1

## Description

### Antifongiques à base d'amides à groupe phényl

La présente invention a pour objet de nouveaux dérivés à groupes phényle et amides (ou leurs homologues) ainsi que leur fabrication, leur utilisation comme matière active pour la protection des plantes contre les atteintes fongiques, les produits intermédiaires pour leur fabrication et leur procédé de fabrication.

Dans la présente description, les diverses formules citées sont présentées ensemble à la fin du texte (avant les revendications). Le terme inférieur appliqué à des radicaux comprenant une chaîne carbonée signifie que cette chaîne carbonée a de un à quatre atomes de carbone (ou de 2 à 4 atomes de carbone dans des chaînes carbonées insaturées).

Les nouveaux produits selon l'invention sont des composés caractérisés en ce qu'ils ont pour formule (I) dans laquelle

* Le signe CYCLE entouré d'un demi-cercle, représente, avec les deux atomes de carbone auxquels ce demi-cercle est relié, un cycle carboné ou hétérocyclique, ayant de 4 à 7 chaînons (de préférence 5 ou 6), comportant au moins une liaison insaturée à caractère éthylènique ou aromatique entre les 2 atomes de carbone porteurs du groupe $-\underset{\underset{Z}{|}}{C}=Y$ et du groupe phényle, ledit cycle étant éventuellement substitué par des radicaux organiques ayant au plus 30 atomes de carbone,
* Y est un atome d'oxygène ou de soufre.
* Z est un atome de chlore, un groupe OW′, $NHR_1$ ou $NR_1$ $R_2$ (les composés dans la formule desquels Z est $NR_1$ $R_2$ sont préférés lorsqu'on cherche à combattre les maladies des plantes et les composés dans lesquels Z est un atome de chlore, un groupe OW′ ou un groupe $NHR_1$ sont principalement utiles comme intermédiaires chimiques)
* W′ est un atome d'hydrogène ; ou un radical alkyle inférieur ou un métal alcalin ou alcalinoterreux.
* $R_1$ et $R_2$, identiques ou différents représentent :

- un radical alkyle inférieur ou alkoxy inférieur (l'un au moins des radicaux $R_1$ et $R_2$ etant autre que alkoxy), ou cycloalkyle de 3 à 7 atomes de carbone, ces radicaux étant éventuellement substitués par au moins un atome d'halogène et/ou un groupe hydroxy ou alkoxy inférieur, ou alkoxy (inférieur)-alkyle (inférieur) ou acyloxy (inférieur) ou phényle ou un groupe hétérocyclique de 4 à 6 chaînons comprenant 1 ou 2 hétéroatomes (O, N ou S) ou un groupe amino mono-ou disubstitué par des alkyle inférieurs,
- un radical alkényle ou alkynyle ayant de 3 à 7 atomes de carbone.

* ou bien $R_1$ et $R_2$ forment ensemble, avec l'atome d'azote auquel ils sont rattachés, un hétérocycle, saturé ou insaturé, comprenant de 4 à 7 chaînons (de préférence 5 ou 6) et 1, 2 ou 3 hétéroatomes (O, N ou S), cet hétérocycle pouvant être substitué par des groupes alkyle inférieurs ou alkoxy inférieurs ou hydroxy ou un radical oxo ou un ou plusieurs atomes d'halogène ou un groupe alkyl (inférieur)-alkoxy (inférieur).
* $R_3$, $R_4$ et $R_5$ identiques ou différents, représentent :
* un atome d'hydrogène ou d'halogène, l'un au moins de ces radicaux $R_3$, $R_4$ et $R_5$ étant autre qu'un atome d'hydrogène ou d'halogène
* un groupe amino éventuellement substitué par un ou deux groupes alkyle inférieurs
* un groupe alkyle inférieur ou alkoxy inférieur ou alkyl (inférieur)-alkoxy inférieur ou alkylthio inférieur, éventuellement halogéné ou hydroxylé
* $R_3$ et $R_4$ (en position meta et para) peuvent former ensemble un radical unique divalent à 3 ou 4 chaînons comprenant 1 ou 2 atomes d'oxygène non vicinaux.

La présente invention concerne plus spécifiquement, à titre des composés utiles en particulier pour combattre les atteintes fongiques des végétaux, les composés tels que définis ci-avant dans lesquels Z est $NR_1$ $R_2$ et dans lesquels les divers radicaux et substituants sont choisis, dans leur nature et dans leur position, de manière telle que le logarithme (log P) décimal de leur coefficient de partage octanol/eau (P) soit compris entre 2 et 5, de préférence entre 2,5 et 4,5.

Le coefficient de partage octanol/eau P d'un produit chimique est déterminé selon des méthodes connues en soi.

D'une manière expérimentale, ce coefficient est le rapport des concentrations d'équilibre d'une substance dissoute dans un système à deux phases constitué des deux solvants immiscibles, l'octanol et l'eau, c'est-à-dire que:

$$P = \frac{C\ \text{octanol}}{C\ \text{eau}}$$

La mesure est faite à 20° C et à trois concentrations différentes et trois rapports de phase différents, étant entendu qu'on doit trouver la même valeur de P à ces trois concentrations et ces trois rapports de phase.

S'il s'agit d'un produit plutôt soluble dans l'eau, on prépare trois solutions aqueuses de ce produit à des

concentrations de 0,25 g/l, 0,5 g/l et 1 g/l, et chacune de ces solutions est mélangée à de l'octanol en quantité telle que le rapport volumique solution aqueuse du produit/octanol est respectivement 80/20, 90/10 et 95/5. Ces mélanges sont alors agités, décantés et l'on mesure dans chacun d'eux la quantité de produit dissous par chromatographie liquide à haute performance et le coefficient de partage est déterminé selon la formule indiquée plus haut.

Inversement, s'il s'agit d'un produit plutôt soluble dans l'octanol, on prépare trois solutions octanoliques de ce produit à des concentrations respectives de 0,25 g/l, 0,5 g/l et 1 g/l, et chacune de ces solutions est mélangée à de l'eau en quantité telle que le rapport volumique solution octanolique du produit/eau est respectivement 80/10, 90/10, 95/5.

Si le produit est trop peu soluble aussi bien dans l'eau que dans l'octanol, en sorte qu'on ne peut atteindre dans aucun d'eux des niveaux de concentration égaux à 0,25, 0,5 et 1 g/l, alors on opère à des gammes de concentrations plus basses par exemple 0,05, 0,1 et 0,2 g/l ou plus bas encore.

Quoi qu'il en soit, on obtient ainsi dans ces conditions trois valeurs de P qui doivent être identiques ( si elles ne le sont pas, on trouve des concentrations plus basses qui permettent d'arriver à trois valeurs égales ) et cette valeur unique commune aux trois mesures correspond au coefficient de partage P (ou à son logarithme log P) qui caractérise le produit.

Toutefois, dans la pratique, il est devenu commun de remplacer cette mesure du coefficient de partage par sa valeur calculée à partir de la structure de la molécule. Des indications sur les méthodes de calcul sont fournies par C. HANSCH et A. LEO dans le livre "Substituent Constants for Correlation Analysis in Chemistry and Biology" Ed. John WILEY, 1979, pages 18 à 43. Cette méthode évolue un peu au cours des années, mais dans le sens d'un affinement, c'est-à-dire d'une plus grande proximité en direction de la valeur réelle obtenue par mesure directe. C'est ainsi que la méthode de calcul du coefficient de partage a été perfectionnée pour tenir compte des facteurs de correction pour les interactions (A. LEO, J. Chem. Perkin Trans. II pages 825-838, 1983). Pour faciliter ces calculs, des programmes d'ordinateurs sont commercialisés, qui effectuent eux-mêmes directement le calcul du coefficient de partage (log P). C'est ainsi que l'on a utilisé dans le présent texte les valeurs fournies par le programme "Medchem" qui est décrit dans le "Medchem Software Manual, release 3.52" novembre 1987, édité par Pomona College à Claremont en Californie.

Même si un tel programme d'ordinateur est amené à évoluer dans le futur, son évolution va dans le sens d'une plus grande exactitude, c'est-à-dire d'un plus grand rapprochement vers la valeur expérimentale de log P.

Le principe de remplacement de la valeur mesurée par la valeur calculée de log P est tellement commun que cette méthode est acceptée par l'Environmental Protection Agency aux USA.

Dans le présent texte, on a donc utilisé les valeurs de log P calculées comme indiqué ci-avant.

Dans ce qui suit, lorsqu'on indique une signification de radicaux multiples, par exemple $(R)_n$, c'est-à-dire n fois le radical R, on entend d'une manière tout à fait générale que les divers radicaux R peuvent être identiques ou différents, il en est ainsi pour tous les types de radicaux et substituants.

Parmi la grande famille des composés de formule (I) diverses sous-familles sont particulièrement avantageuses.

C'est ainsi que, l'on préfère les composés ayant l'une ou l'autre ou plusieurs des caractéristiques suivantes :

- $R_1$ et $R_2$ représentent :

* un radical alkyle inférieur ; un radical cycloalkyle ayant de 3 à 7 atomes de carbone ; un radical alkènyle ou alkynyle ayant de 3 à 7 atomes de carbone ; un radical alkoxyalkyle ayant de 3 à 8 atomes de carbone

* ou bien $R_1$ et $R_2$ peuvent former ensemble, avec l'atome d'azote auquel ils sont reliés, un hétérocycle saturé ou insaturé comprenant 4 à 7 chaînons et 1 ou 2 hétéroatomes (O, N, S), cet hétérocycle pouvant être substitué par des radicaux alkyle de 1 à 3 atomes de carbone ou des groupes alkoxyalkyle de 2 à 4 atomes de carbone.

-$R_3$, $R_4$ et $R_5$ sont en position respective méta, para et méta' sur le groupe phényle substituant le CYCLE ( ces positions étant repérées par rapport au lien valentiel reliant ledit groupe phényle avec ledit CYCLE), et

* $R_3$, $R_4$ et $R_5$ représentent un atome d'hydrogène ou d'halogène (l'un au moins de ces 3 radicaux étant autre que l'atome d'hydrogène ou d'halogène) ; ou un radical alkyle inférieur ou un alkoxy inférieur ; ou un alkoxyalkyle ayant de 2 à 4 atomes de carbone ; ou un alkylthio inférieur ; ou un hydroxy ; ou un groupe amino éventuellement mono-substitué ou disubstitué par un radical alkyle inférieur, ou bien

* $R_3$ et $R_4$ (en position meta et para) peuvent former ensemble un radical unique divalent à 3 ou 4 chaînons comprenant 1 ou 2 atomes d'oxygène non vicinaux,

- le signe CYCLE, entouré d'un demi-cercle, représente un radical divalent $-K_6-K_7-K_8-K_9-$ ou $-K_6-K_7-K_{10}-$ de manière à constituer, avec les deux atomes de carbone auxquels il est relié un cycle à 5 ou 6 chaînons [comme représenté graphiquement dans les formules (Ia) et (Ib)], les groupes $K_6$, $K_7$, $K_8$, $K_9$ et $K_{10}$ étant tels que :

$K_6$ représente -N= ou $-C(R_6)-$

$K_7$ représente -N= ou $-C(R_7)-$

$K_8$ représente -N= ou $-C(R_8)-$

$K_9$ représente -N= ou $-C(R_9)-$

$K_{10}$ représente -N= ou $-C(R_{10})-$

et, en outre, dans le cas ou le signe CYCLE entouré d'un demi-cercle représente $K_6$, $K_7$, $K_{10}$, ces radicaux $K_6$, $K_7$ et $K_{10}$ peuvent encore être tels que :

$K_6$ représente $-N(R_6)-$ ou $-C(R_6)_2-$ ou -O- ou $-S(O)_n$
$K_7$ représente $-N(R_7)-$ ou $-C(R_7)_2-$ ou -O- ou $-S(O)_n$
$K_{10}$ représente $-N(R_{10})-$ ou $-C(R_{10})_2-$ ou -O- ou $-S(O)_n$
ou -CO-
n étant un nombre entier égal à 0, 1 ou 2,
étant entendu que, dans les deux cas où le signe CYCLE représente $-K_6-K_7-K_8-K_9$ et $-K_6-K_7-K_{10}-$, l'un au moins des groupes $K_6$ et $K_7$ représente $-C(R_6)=$ ou $-C(R_6)_2-$ ou $-C(R_7)=$ ou $-C(R_7)_2-$ et que, de plus, ces radicaux divalents $-K_6-K_7-K_8-K_9-$ et $-K_6-K_7-K_{10}-$ ne contiennent dans leur chaîne principale que 0, 1 ou 2 hétéroatomes (N, O ou S) et aucun enchaînement -O-O- ou -S-S-.
(On remarque que $-K_6-K_7-K_8-K_9-$ forment en pratique un cycle à caractère aromatique).
$-R_6$, $R_7$, $R_8$, $R_9$ ou $R_{10}$ chacun étant identique ou différent des autres, représentent :
* un atome d'hydrogène ou d'halogène, l'un au moins des radicaux $R_6$ ou $R_7$ étant autre que l'atome d'hydrogène,
* un groupe cyano, nitro, thiocyanato, hydroxy ou carboxy,
* un alkyle, cycloalkyle, alkényle, alkynyle, alkyl-$S(O)_n$ (avec $n = 0, 1$ ou 2), alkoxy, cycloalkoxy de 3 à 8 atomes de carbone, alkényloxy, alkynyloxy ; ces radicaux ont avantageusement de 1 à 8 atomes de carbone et peuvent éventuellement être substitués par un ou plusieurs atomes d'halogène,
* un radical phényle, naphtyle, phényl-$S(O)_n$ (avec $n = 0, 1$ ou 2), phénoxy, phénylalkyle (alkyle inférieur), phénylalkyl-S(O)n (alkyle inférieur ; $n = 0, 1$ ou 2), phénylalkoxy (alkoxy inférieur) ou encore un reste d'un hétérocycle éventuellement substitué, saturé ou insaturé, ayant dans le cycle 5 à 6 chaînons avec 1 à 3 hétéroatomes choisis dans le groupe comprenant l'oxygène, le soufre et l'azote, le noyau phényle ou hétérocyclique de chacun de ces radicaux pouvant être substitué par au moins un substituant choisi dans le groupe comprenant :
* un atome d'halogène,
* un groupe nitro, cyano, carboxy, hydroxy, mercapto, thiocyanato, alkoxy (inférieur)-carbonyle, -CO-NR′R″, NR′R″, -N(R′)-CO-R″, -O-CO-R′, -O-CO-NR′R″ ou,
* un radical alkyle inférieur, alkoxy inférieur, alkyl (inférieur)-$S(O)_n$ (avec $n = 0, 1$ ou 2), cycloalkyle (de 3 à 7 atomes de carbone), phényle, phénoxy, phényl-$S(O)_n$ (avec $n = 0, 1$ ou 2), phénylalkyle, phénylalkoxy, phénylalkyl-$S(O)_n$ (avec $n = 0, 1$ ou 2), ces radicaux étant éventuellement halogénés et ayant 1 à 4 atomes de carbone pour la partie alkyle.
* Un groupe alkoxy (inférieur)-carbonyle, CO-NR′R″, NR′R″, N(R′)-CO-R″, O-CO-R′, O-CO-NR′R″.
* R′ et R″ identiques ou différents sont chacun un atome d'hydrogène; un radical alkyle inférieur ; cycloalkyle de 3 à 7 atomes de carbone ; phényle éventuellement halogéné ; phénylalkyle (alkyle inférieur) éventuellement halogéné ; un alkényle ou alkynyle de 3 à 7 atomes de carbone ; alkoxyalkyle de 3 à 8 atomes de carbone.
* Lorsque $K_6$ et $K_7$ représentent $-C(R_6)=$ ou $-C(R_6)_2-$ou $-C(R_7)=$ ou $-C(R_7)_2-$, alors $R_6$ avec $R_7$ ou l'un des radicaux $R_6$ avec l'un des radicaux $R_7$, peuvent former, avec les deux atomes de carbone portant $R_6$ et $R_7$, un cycle carboné ou hétérocyclique, saturé ou insaturé, comprenant 5 ou 6 chaînons et 0,1 ou 2 hétéroatomes (O, N ou S) non vicinaux.
* Lorsque $K_6$ ou $K_7$ ou $K_8$, $K_9$ ou $K_{10}$ ont l'une des significations $-N(R_6)-$, $-N(R_7)-$, $-N(R_8)-$, $-N(R_9)-$, $-N(R_{10})-$, alors le radical correspondant , $R_6$, $R_7$, $R_8$, $R_9$ ou $R_{10}$, a l'une des significations suivantes (pourvu que l'un au moins des radicaux $R_6$ ou $R_7$ soit autre que l'atome d'hydrogène) :
l'atome d'hydrogène ; un radical alkyle de 1 à 6 atomes de carbone ; un radical phényle ou phénylalkyle (alkyle inférieur) éventuellement substitués par un ou plusieurs atomes d'halogène ou par un groupe cyano ou nitro ou par un radical alkyle inférieur ou alkoxy inférieur.

Selon ce qui vient d'être exposé, les composés de formule (Ia) et (Ib) constituent des familles particulières dans l'invention.

Parmi les divers composés de formule (I), (Ia) et (Ib) faisant partie de l'invention, différentes sous-familles particulières présentent un intérêt particulier, notamment sur le plan des propriétés des produits obtenus et sur le plan de l'accessibilité. Ces sous-familles sont définies avec plus de précision ci-après, notamment par le moyen des formules (II), (III), (IV), (V), (VI) et (VII).

Dans ces sous-familles, lorsqu'il est fait référence aux définitions générales de radicaux données ci-avant, cela se rapporte aussi bien aux définitions les plus générales, que, d'une manière préférentielle, aux définitions préférées.

De la même manière, en ce qui concerne les produits utiles, notamment pour la lutte contre les atteintes fongiques des végétaux, on préfère utiliser les composés ayant une valeur de log P telle que définie ci-avant.

Une <u>première sous-famille</u> de produits selon l'invention est constituée des <u>produits de formule (II)</u> dans laquelle :
* $K_{110}$ est un atome d'oxygène, un groupe $S(O)_{n1}$ dans lequel $n_1$ est un entier égal à zéro, 1 ou 2, ou un groupe $NR_{110}$ ou $C(R_{110})_2$ ou un groupe oxo.
* $Z_1$ est un atome d'halogène ou un groupe $OW_1$ (dans lequel $W_1$ a l'une des significations données pour W′) ou un groupe $NHR_{11}$ ou $NR_{11}R_{12}$, dans lequel $R_{11}$ et $R_{12}$, identiques ou différents, ont l'une des significations données respectivement pour $R_1$ et $R_2$
* $R_{13}$ à $R_{15}$, identiques ou différents, ont l'une des significations données pour $R_3$, $R_4$ et $R_5$
* $R_{13}$ et $R_{14}$ peuvent également former ensemble un groupe méthylènedioxy ou éthylènedioxy, les carbones de ces motifs pouvant eux-mêmes être substitués par des radicaux alkyle inférieur ou des atomes d'halogène

* $R_{16}$ à $R_{19}$, identiques ou différents, ont également l'une des significations données pour $R_6$ à $R_9$,
* si $K_{110}$ est $=C(R_{110})$- ou $-C(R_{110})_2$-, alors $R_{110}$ représente un atome d'hydrogène ou un radical alkyle inférieur ou un radical benzyle ou un hydroxy, ou un radical alcanoyloxy inférieur (par exemple acétyloxy) ou un alkoxy inférieur,
* si $K_{110}$ est $-N(R_{110})$-, alors $R_{110}$ représente un atome d'hydrogène ou un radical alkyl inférieur ou un radical benzyle.

Lorsque $Z_1$ est un groupe $NR_{11}R_{12}$, les dérivés ont la formule (II') et lorsque $Z_1$ est un groupe $OW_1$, les dérivés ont la formule (II").

Parmi les dérivés de formule (II), on préfère ceux dans la formule desquels :
* $K_{110}$ est un atome d'oxygène, de soufre, ou représente $-N(R_{110})$- ou $-C(R_{110})_2$-,
* $Z_1$ est un radical $OW_1$ tel que défini ci-dessus, un groupe $NHR_{11}$ ou $NR_{11}R_{12}$, où $R_{11}$ et $R_{12}$ sont des alkyle inférieurs tels que $R_{11}$ et $R_{12}$ comportent au plus six carbones au total, ou bien forment ensemble un groupe morpholino ou thiomorpholino,
* $R_{13}$ à $R_{15}$ ont la même signification que $R_3$, $R_4$ et $R_5$.

Sont encore plus particulièrement préférés les dérivés de formule (II) dans laquelle :
* $Z_1$ est un groupe $OW_1$ tel que défini ci-dessus, ou un groupe $NR_{11}R_{12}$ qui est un groupe morpholino, thiomorpholino, N-méthyléthylamino ou N-diéthylamino,
* $R_{13}$ à $R_{15}$, identiques ou différents, sont un atome d'hydrogène ou d'halogène, un alkyle inférieur, un alkoxy inférieur, un alkylthio inférieur,
* $R_{16}$, $R_{18}$, $R_{19}$ sont un atome d'hydrogène ou de fluor,
* $R_{17}$ est un atome d'hydrogène ou d'halogène ; un radical alkyle inférieur, alkoxy inférieur, alkylthio inférieur, halogénoalkyle inférieur, halogénoalkoxy inférieur, halogénoalkylthio inférieur ; un groupe nitro, cyano ; un radical alkényle inférieur, un alkynyle inférieur ou un alkoxy (inférieur)-carbonyle.

Par ailleurs, sont particulièrement préférés, pour les applications pesticides, les produits ci-dessus pour lesquels log P est compris entre 2,5 et 4.

Parmi les divers composés de formule (I) faisant partie de l'invention, une <u>seconde sous-famille</u> présentant un intérêt tout particulier est constituée de phénylamides de <u>formule (III)</u>
dans laquelle :
* $Z_2$ représente :
- un atome d'halogène, ou
- un groupe $OW_2$ (dans lequel $W_2$ a l'une des significations données pour W'), ou un groupe $NHR_{21}$, ou
- un groupe $NR_{21}R_{22}$ dans lequel $R_{21}$ et $R_{22}$, identiques ou différents, ont l'une des significations données respectivement pour $R_1$ et $R_2$.
* $R_{23}$ à $R_{25}$ identiques ou différents ont l'une des significations données respectivement pour $R_3$, $R_4$ et $R_5$.
* $R_{23}$ et $R_{24}$ peuvent également former ensemble un groupe méthylènedioxy ou éthylènedioxy, les carbones de ces motifs pouvant eux-mêmes être substitués par des radicaux alkyle inférieur ou des atomes d'halogène.
* $R_{26}$ et $R_{27}$, identiques ou différents, ont l'une des significations données respectivement pour $R_6$, $R_7$ sous réserve que $R_{26}$ et $R_{27}$ ne sont pas simultanément un atome d'hydrogène.
* $R_{28}$ et $R_{29}$, identiques ou différents, peuvent être un atome d'hydrogène ou d'halogène ; le groupe hydroxy ; un radical alkyle inférieur ; alkoxy inférieur ; alkoxy (inférieur)-alkyle (inférieur); alkoxy (inférieur)-carbonyle.

Parmi les dérivés de formule (III), on préfère ceux dans la formule desquels :
* $Z_2$ est un radical $OW_2$ (dans lequel $W_2$ a l'une des significations données pour W'), un groupe $NHR_{21}$ ou un groupe $NR_{21}$ $R_{22}$ où $R_{21}$ et $R_{22}$ sont des alkyle inférieurs tels que $R_{21}$ et $R_{22}$ comportant au plus six carbones au total, ou bien forment ensemble un groupe morpholino ou thiomorpholino.
* $R_{23}$ à $R_{25}$, identiques ou différents, ont la même signification que $R_3$, $R_4$ et $R_5$.
* $R_{28}$ et $R_{29}$ identiques ou différents, peuvent être un atome d'hydrogène ou d'halogène ou un groupe hydroxy.

Sont particulièrement préférés les dérivés de formule (III) dans laquelle :
* $Z_2$ est un groupe $OW_2$ tel que défini ci-dessus ou un groupe $NR_{21}$ $R_{22}$ qui est un groupe morpholino, thiomorpholino, N-méthyl éthylamino ou N-diéthylamino.
* $R_{23}$ à $R_{25}$, identiques ou différents, sont un atome d'hydrogène ou d'halogène un alkyle inférieur, un alkoxy inférieur, un alkylthio inférieur.
* $R_{26}$ à $R_{27}$ identiques ou différents, sont un atome d'hydrogène ou d'halogène, un groupe alkyle inférieur, un groupe alkényle inférieur, un groupe alkoxy inférieur, un groupe alkylthio inférieur, un groupe halogénoalkyle inférieur, un groupe halogénoalkoxy inférieur ou halogénoalkythio inférieur.
* $R_{28}$ et $R_{29}$, identiques ou différents, sont un atome d'hydrogène ou de fluor.

Par ailleurs, en ce qui concerne les produits pour applications pesticides, on préfère les produits ci-dessus pour lesquels log P est compris entre 2,5 et 4.

Parmi les composés de formule (I) faisant partie de l'invention, une <u>troisième sous-famille</u> présentant un intérêt tout particulier est constituée des dérivés nicotiniques de <u>formule (IV)</u>.
dans laquelle :
* $Z_3$ représente :
- un atome d'halogène, ou
- un groupe $OW_3$ (dans lequel $W_3$ a l'une des significations données pour W') ou un groupe $NHR_{31}$ ou

- un groupe $NR_{31}$ $R_{32}$, dans lequel $R_{31}$ et $R_{32}$, identiques ou différents, ont l'une des significations données respectivement pour $R_1$ et $R_2$,

* $R_{33}$ à $R_{35}$, identiques ou différents, ont l'une des significations données respectivement pour $R_3$, $R_4$ et $R_5$.

* $R_{33}$ et $R_{34}$ peuvent également former ensemble un groupe méthylènedioxy ou éthylènedioxy, les carbones de ces groupes pouvant eux-mêmes être substitués par des alkyle inférieurs ou des atomes d'halogène.

* $R_{37}$ a l'une des significations données pour $R_7$ sous réserve que $R_7$ ne soit pas un atome d'hydrogène.

* $R_{38}$ et $R_{39}$, identiques ou différents, peuvent être un atome d'hydrogène ou d'halogène, un radical alkyle inférieur, alkoxy inférieur, alkoxy (inférieur)-alkyle (inférieur) ou alkoxycarbonyle inférieur ou un groupe cyano. De plus, lorsque $R_{37}$ est un phényle, $R_{38}$ peut former, avec l'atome de carbone de $R_{37}$ en ortho de celui relié au noyau pyridinique, une chaîne $Y_3$-$(CH_2)_{n3}$, dans laquelle $Y_3$ est un atome d'oxygène ou de soufre ou un groupe méthylène et $n_3$ un nombre égal à 0, 1 ou 2.

Parmi les composés de formule (IV), on préfère ceux dans la formule desquels $R_{33}$ à $R_{35}$, identiques ou différents, sont un atome d'hydrogène ou d'halogène (l'un au moins de ces trois radicaux étant autre que l'atome d'hydrogène ou d'halogène) ou un alkyle inférieur, un alkoxy inférieur ou un alkylthio inférieur.

Parmi les composés de formule (I) faisant partie de l'invention, une <u>quatrième sous-famille</u> présentant un intérêt tout particulier est constituée des dérivés de <u>formule (V)</u> dans laquelle :

- $Z_4$ représente :

* un atome d'halogène, ou

* un groupe $OW_4$ (dans lequel $W_4$ a l'une des significations données pour W′), un groupe $NHR_{41}$ ou un groupe $NR_{41}$ $R_{42}$ dans lequel $R_{41}$ et $R_{42}$, identiques ou différents, ont l'une des significations données respectivement pour $R_1$ et $R_2$.

* $R_{43}$, $R_{44}$, $R_{45}$, identiques ou différents, ont l'une des significations données respectivement pour $R_3$, $R_4$ et $R_5$.

* $R_{47}$ a une des significations données pour $R_7$, mais est autre que l'atome d'hydrogène.

* $R_{49}$ peut être un atome d'hydrogène ou un radical alkyle inférieur.

Parmi les dérivés de formule (V), on préfère ceux dans la formule desquels :

* $Z_4$ est un radical $OW_4$ (dans lequel $W_4$ a l'une des significations données pour W′), un groupe $NHR_{41}$ ou un groupe $NR_{41}$ $R_{42}$ dans lesquels $R_{41}$ et $R_{42}$ sont des alkyle inférieurs tels que $R_{41}$ et $R_{42}$ comportent au plus six carbones au total, ou bien forment ensemble un groupe morpholino ou thiomorpholino.

* $R_{43}$ à $R_{45}$, identiques ou différents, sont un atome d'hydrogène ou d'halogène, un alkyle inférieur, un alkoxy inférieur, un alkylthio inférieur.

* $R_{49}$ est un atome d'hydrogène.

Une <u>cinquième sous-famille</u> de produits selon la formule (I) est constituée par les produits de formule (VI) dans laquelle :

* $Z_5$ représente un atome d'halogène ou un groupe $OW_5$ (lequel $W_5$ a l'une des significations données pour W′) ou un groupe $NHR_{51}$ ou un groupe $NR_{51}R_{52}$ dans lequel $R_{51}$ et $R_{52}$, identiques ou différents, ont l'une des significations données respectivement pour $R_1$ et $R_2$.

* $R_{53}$, $R_{54}$, $R_{55}$, identiques ou différents, ont l'une des significations données respectivement pour $R_3$, $R_4$, $R_5$.

* $R_{53}$ et $R_{54}$ peuvent également former ensemble en groupe méthylènedioxy ou éthylènedioxy, les carbones de ces groupes pouvant eux-mêmes être substitués par des alkyle inférieurs ou des atomes d'halogène.

* $R_{56}$ et $R_{57}$ ont l'une des significations données pour $R_6$ et $R_7$ sous réserve que $R_6$ et $R_7$ ne sont pas simultanément un atome d'hydrogène.

* $R_{59}$ peut être un atome d'hydrogène ou d'halogène, un radical alkyle inférieur, un radical hydroxy, alkoxy inférieur ou alkoxyalkyle inférieur, ces radicaux pouvant être halogénés.

Parmi les composés de formule (VI), on préfère ceux dans la formule desquels $R_{53}$ à $R_{55}$, identiques ou différents, sont un atome d'hydrogène ou d'halogène ou un alkyle inférieur, un alkyloxy inférieur ou un alkylthio inférieur.

$R_{59}$ est un atome d'hydrogène ou un alkyle inférieur.

Une <u>sixième sous-famille</u> de produits selon la formule générale (I) est constituée par les produits de <u>formule (VII)</u> dans laquelle :

* $Z_6$ représente :

- un atome d'halogène,

- un groupe $OW_6$ (dans lequel $W_6$ a l'une des significations données pour W′), un groupe $NHR_{61}$ ou un groupe $NR_{61}$ $R_{62}$ dans lequel $R_{61}$ et $R_{62}$, identiques ou différents, ont l'une des significations données respectivement pour $R_1$ et $R_2$.

* $K_{66}$ est un atome d'azote substitué -N($R_{66}$)-, ou -N=, ou un atome de soufre ou d'oxygène ou un atome de carbone substitué =C($R_{66}$)-.

* $K_{67}$ est un atome de soufre ou d'oxygène ou un atome d'azote substitué -N($R_{67}$)-, ou -N=, ou un atome de carbone substitué =C($R_{67}$)-, étant entendu que $K_{66}$ et $K_{67}$ ne peuvent pas simultanément être -N($R_{66}$)- et -N($R_{67}$)-.

* $K_{610}$ est un atome d'azote substitué -N($R_{610}$)-, ou -N=, ou un atome de soufre ou d'oxygène, ou un atome de carbone substitué =C($R_{610}$)-, étant entendu que $K_{610}$ et $K_{67}$ ne peuvent pas simultanément être l'un -N($R_{610}$) et l'autre N($R_{67}$)-.

* $R_{63}$, $R_{64}$, $R_{65}$, identiques ou différents, ont l'une des significations données respectivement pour $R_3$, $R_4$, $R_5$.

* $R_{66}$, $R_{67}$, et $R_{610}$, identiques ou différents, ont l'une des significations données respectivement pour $R_6$, $R_7$

et $R_{10}$.

Parmi les dérivés de formule VII, on préfère ceux dans la formule desquels :

* $Z_6$ est un radical $OW_6$ (dans lequel $W_6$ a l'une des significations données pour W′), un groupe $NHR_{61}$ ou un groupe $NR_{61}$ $R_{62}$ dans lesquels $R_{61}$ et $R_{62}$ comportent au plus six carbones au total, ou bien forment ensemble un groupe morpholino ou thiomorpholino.
* $K_{66}$ est un atome d'azote substitué -N($R_{66}$)-.
* $K_{67}$ est un atome d'azote -N= ou un atome de carbone substitué =C($R_{67}$)-.
* $K_{610}$ est un atome de carbone =CH-.
* $R_{63}$, $R_{64}$, $R_{65}$, identiques ou différents, sont un atome d'hydrogène ou d'halogène, ou un alkyle inférieur, un alkoxy inférieur, un alkylthio inférieur.
* $R_{66}$ et $R_{67}$ sont un atome d'hydrogène, un groupe alkyle, phényle, phénylalkyle éventuellement substitués par des halogènes ou des groupements halogénoalkyles étant bien entendu que $R_{66}$ et $R_{67}$ ne peuvent pas simultanément être un atome d'hydrogène.

Les dérivés selon l'invention sont particulièrement intéressants comme intermédiaires et en raison de leurs remarquables propriétés fongicides, notamment en agriculture ainsi que comme intermédiaires pour la synthèse de dérivés, notamment d'autres dérivés de formule (I).

Dans les diverses formules de composés selon l'invention, décrits ci-avant, on estime que les produits couverts sont nouveaux. Toutefois , en ce qui concerne l'Allemagne Fédérale seulement, les produits suivants sont exclus des revendications :

* les produits de formule (Ia) dans laquelle Y est l'atome d'oxygène, et en même temps $K_8$ et $K_9$ sont, ou bien -CH=, ou bien l'un est -N= et l'autre -CH=, et en même temps $K_6$ et $K_7$ forment ensemble en cycle carboné insaturé accolé au cycle à 5 chaînons comprenant $K_{10}$.
* les produits de formule (Ib) dans laquelle Y est l'atome d'oxygène et en même temps $K_{10}$ est O, S ou SO ou $SO_2$ ou $NR_{10}$ ($R_{10}$ ayant la signification donnée ci-avant) ou $CH_2$ ou CO et en même temps $K_6$ et $K_7$ forment ensemble un cycle carboné insaturé, accolé au cycle à 5 chaînons comprenant $K_{10}$.

Procédés de préparation :

Les dérivés selon l'invention peuvent être préparés selon plusieurs procédés.

On décrit ci-après la préparation des composés de formule (II).

Selon un premier procédé (procédé A) convenant particulièrement aux composés de formule (II) dans lesquels $K_{110}$ est -O-, -S- ou $NHR_{110}$, on part d'une benzophénone de formule (XII′) dans laquelle $R_{13}$ à $R_{19}$ ont les mêmes significations que précédemment (y compris pour les significations préférées), et $A_1$ est un atome d'halogène substituable (tel que le brome ou, de préférence, le chlore ou le fluor) ou un groupe nitro, que l'on fait réagir avec un dérivé de formule :

$H-K_{110}-CH_2-CO-Z_1$

dans laquelle $K_{110}$ et $Z_1$ ont la même signification que précédemment, pour donner un dérivé de formule (II) par remplacement de $A_1$ et cyclisation.

Cette réaction s'effectue avantageusement en solvant alcoolique (alkanol inférieur, par exemple) en présence de l'alkanolate correspondant (par exemple $CH_3ONa$, $C_2H_5ONa$, $C_3H_7ONa$) ou en présence d'une base organique telle qu'une amine de préférence tertiaire (triéthylamine), ou une base minérale telle que les hydroxydes, carbonates ou hydrogénocarbonates, cette base étant dérivée des métaux alcalins ou alcalino-terreux tels que par exemple le sodium, le potassium ou le calcium.

La réaction peut également être effectuée en présence des mêmes bases dans un solvant dipolaire aprotique tel que le diméthylsulfoxyde, le diméthylformamide, le N, N-diméthylacétamide, la N-méthyl pyrrolidone, ou l'hexaméthylphosphorotriamide.

Dans le cas des dérivés de formule (II″) ceux-ci peuvent être transformés (procédé G) en dérivés de formule (II′) par chauffage, à une température comprise entre 100° C et 200° C et éventuellement sous pression, avec un excès d'amine $HNR_{11}R_{12}$ selon le schéma :

$(II'') + HNR_{11}R_{12} \rightarrow (II') + W_1OH$

Lorsque, dans la formule (II″), $W_1$ est un atome d'hydrogène, ces dérivés doivent d'abord subir une activation de la fonction acide par un agent tel que le chlorure de thionyle ($SOCl_2$), le chlorure de phosphoryle ($POCl_3$), le trichlorure ou le pentachlorure de phosphore ($PCl_3$, $PCl_5$), le dicyclohexylcarbodiimide, le diimidazole carbonyle, le chloroformiate d'éthyle ou l'anhydride trifluoroacétique, avant traitement par l'amine $HNR_{11}R_{12}$ à une température comprise entre 0° C et 50° C dans un solvant tel que le THF (tétrahydrofurane) ou un solvant chloré (notamment un hydrocarbure aliphatique ou aromatique), de préférence en présence d'un excès d'amine $HNR_{11}R_{12}$ ou en présence d'une base minérale ou organique telle que la triéthylamine.

A partir des dérivés de formule (II′), il est possible de préparer d'autres dérivés de formule (II′) par les techniques classiques d'aménagement fonctionnel de la chimie organique.

Par exemple dans le cas où un dérivé nitré (formule (II) dans laquelle $R_{16}$ ou $R_{17}$ ou $R_{18}$ ou $R_{19}$ est $NO_2$) est obtenu il peut être réduit (procédé B) en dérivé aminé par un réducteur tel qu'un métal (fer, étain) ou un sel de ces métaux (chlorure d'étain) en milieu acide minéral (HCl, $H_2SO_4$) ou organique (alcool éthylique ou un acide carboxylique, notamment acétique). Comme réducteur, on peut également employer l'hydrogène (éventuellement sous pression), ou ses précurseurs (borohydrure de sodium, dérivés de l'acide formique, cyclohexène) en présence d'un métal de transition tel que le palladium. Dans ce cas, on utilise avantageusement comme solvant, l'acétate d'éthyle, l'acide acétique ou un alcool tel que l'alcool éthylique.

Le dérivé aminé obtenu (formule (II) dans laquelle $R_{16}$ ou $R_{17}$ ou $R_{18}$ ou $R_{19}$ sont $NH_2$) peut lui-même être soumis à des réactions d'acylation (procédé C) ou de diazotation. La réaction d'acylation s'effectue à l'aide de dérivés d'acide carboxylique (chlorure ou anhydride d'acide) en présence d'une base organique (triéthylamine, pyridine) ou minérale (hydroxyde, carbonate ou hydrogénocarbonate des métaux alcalins) au sein d'un solvant aprotique tel qu'un solvant chloré (chlorure de méthylène, chloroforme) ou aromatique (benzène toluène) ou un éther aliphatique (diéthyloxyde, tétrahydrofuranne, 1,2- diméthoxy éthane).

La diazotation (procédé D) est réalisée en traitant une solution du dérivé aminé (formule (II) dans laquelle $R_{16}$ ou $R_{17}$ ou $R_{18}$ ou $R_{19}$ sont $NH_2$) dans un acide minéral fort (acide chlorhydrique, bromhydrique ou sulfurique) par un nitrite alcalin (nitrite de sodium), à une température comprise entre -10°C et +15°C. Le sel de diazonium ainsi obtenu est alors décomposé à des températures comprises entre + 5°C et +100°C en présence de différents agents tels que le chlorure cuivreux, le bromure cuivreux, l'iodure de potassium, l'oxyde cuivreux ou l'éthylthioxanthate de potassium pour fournir respectivement des dérivés, dans lesquels le groupe amino a été remplacé par un atome de chlore, un atome de brome, un atome d'iode, un groupe hydroxyle ou un groupe mercapto.

Dans le cas où un atome de brome est introduit, on peut réaliser une réaction de couplage arylique (procédé E) avec un acide boronique $R_{17}B(OH)_2$ dans lequel $R_{17}$ peut être un hétérocycle ou un phényl ou un groupe vinyle (ces groupes peuvent être substitués comme indiqué dans la définition de $R_7$) pour conduire à d'autres dérivés selon l'invention.

$R_{17}B(OH)_2$

(II"') ——————> (II"")

Base

catalyseur

La réaction s'effectue aussi bien avec $Z_1 = OW_1$ ($W_2$ = H ou alkyle, ou métal alcalin) qu'avec $Z_1 = NR_{11}R_{12}$

Comme catalyseur, on peut utiliser des complexes du palladium commerciaux Pd $[(P(C_6H_5)_3]_4$, palladium tétrakistriphénylphosphine ou préparés in situ à partir d'un sel de palladium tel que l'acétate de palladium et d'une phosphine telle que la triphénylphosphine ou la tri-o-tolylphosphine. Comme base, on peut utiliser une base minérale telle que les carbonates et hydrogènocarbonates des métaux alcalins ($Na_2CO_3$, $NaHCO_3$...) ou une base organique telle qu'une amine (triéthylamine). La réaction s'effectue avec une base minérale en milieu hétérogène comprenant une phase organique qui peut être un solvant hydrocarboné tel que le benzène ou le toluène ou un éther à point d'ébullition élevé tel que le glyme ou ses dérivés supérieurs diglyme, triglyme, et une phase aqueuse basique. La réaction peut également s'effectuer avec une base organique en milieu homogène en présence d'un solvant tel que le diméthylformamide ou la N-méthylpyrrolidone. Les acides boroniques utilisés peuvent être des composés commerciaux ou peuvent être préparés par interaction d'un dérivé organométallique $R_{17}M_1$ où $R_{17}$ a la même signification que précédemment, ($M_1$ = Li ou MgX avec X = Cl, Br) avec un borate d'alkyle B $(OR)_3$, (avec R′ = $CH_3$, $C_2H_5$, $C_3H_7$, i-$C_3H_7$, $C_4H_9$) suivie d'une hydrolyse acide selon les modes opératoires décrits dans Organic Synthesis coll. vol. 4 page 68 ou dans Journal of organic chemistry 49 pages 5237 - 5243 (1984). Les réactifs sont utilisés dans les proportions suivantes :

| | |
|---|---|
| acide boronique $R_{17}B(OH)_2$ | : 1,05 à 1,50 équivalents |
| base | : 4,00 à 6,00 équivalents |
| catalyseur | : 0,005 à 0,03 équivalents |
| substrat de formule II"' | : 1,00 équivalent |

La réaction s'effectue à une température comprise entre 50°C et 150°C, de préférence au reflux du mélange réactionnel, pendant une durée allant de deux à quarante huit heures. Le dérivé de formule (II"") est isolé par les méthodes habituelles, extraction ou précipitation par adjonction d'eau au mélange réactionnel, et peut être purifié par recristallisation ou chromatographie dans un solvant approprié.

Dans un autre procédé (procédé F), qui est une variante du procédé D, on fait réagir (à chaud à une température comprise entre 40°C et la température d'ébullition du solvant) un composé de formule (II) dans lequel un des substituants $R_{16}$ à $R_{19}$ est un groupe amino non substitué et $Z_1$ et $R_{13}$ à $R_{15}$ ont la signification déjà indiquée avec un nitrite d'alkyle inférieur tel que le nitrite de t-butyle au sein d'un solvant tel que le tétrahydrofurane ou le diméthylformamide pour obtenir un dérivé de formule (II) dans lequel le groupe amino est remplacé par un atome d'hydrogène et où $Z_1$ et $R_{13}$ à $R_{19}$ ont la signification déjà indiquée

Les dérivés de formule (XII′) peuvent être préparés par une réaction de Friedel-Crafts selon le schéma :

(XII‴) + (XII″) → (XII′)
dans lequel $A_1$ et $R_{13}$ à $R_{19}$ ont la même signification que précédemment, les composés (XII‴) et (XII″) étant en soi connus.

Cette réaction s'effectue en présence d'un acide de Lewis en quantité catalytique ou stoéchiométrique ($FeCl_3$, $AlCl_3$, $ZnCl_2$, $SnCl_4$) en présence ou en l'absence de solvant (nitrobenzène, solvants chlorés aliphatiques, disulfure de carbone). La réaction est conduite entre -10°C et 200°C suivant les conditions expérimentales employées.

Selon un autre procédé (procédé G) convenant particulièrement aux composés de formule (II) dans laquelle $K_{110}$ est un groupe $C(R_{110})_2$ et où $R_{13}$ à $R_{19}$ ont les mêmes significations que précédemment (y compris pour les significations préférées), on fait réagir une benzophénone de formule (XXII′) dans laquelle $Z_{11}$ = OH et $R_{13}$ à $R_{19}$ ont les mêmes significations que précédemment (y compris pour les significations préférées) avec du chlorure de thionyle ($SOCl_2$), du chlorure de phosphoryle ($POCl_3$), du trichlorure de phosphore ou du pentachlorure de phosphore ($PCl_3$ ou $PCl_5$) pour obtenir une benzophénone de formule (XXII′) dans laquelle $Z_1$ = Cl et $R_{13}$ à $R_{19}$ ont les mêmes significations que précédemment.

De façon générale, le composé ainsi obtenu peut ne pas être isolé ; il est mis à réagir avec un malonate d'alkyle inférieur en présence d'une base qui est préférentiellement un alkanoate de magnésium, tel que l'éthylate de magnésium. Le composé de formule (XXII″), dans laquelle $W_1$ est un alkyle inférieur, ainsi obtenu est ensuite traité par une base tel qu'un alkanoate d'un métal alcalin ou alcalino terreux ou une base minérale tels que les hydroxydes et carbonates des métaux alcalins en milieu aqueux ou alcoolique ou au sein d'un solvant dipolaire aprotique tel que le diméthylsulfoxyde, le diméthylformamide, la N-méthylpyrrolidone, pour fournir un composé de formule (II) dans laquelle $K_{110}$ est un groupe oxo et où $R_{13}$ à $R_{19}$ ont les mêmes significations que précédemment et $Z_1$ est un groupe $OW_1$ où $W_1$ est un alkyle inférieur.

Ce composé traité à chaud à une température comprise entre 100°C et 200°C éventuellement sous pression avec un excès d'une amine $HNR_{11}R_{12}$, en présence ou non d'un solvant, fournit un composé de formule (II) dans lequel $K_{110}$ est un groupe oxo et où $R_{13}$ à $R_{19}$ ont la même signification que précédemment et $Z_1$ est un groupe $NR_{11}$ $R_{12}$ tel que défini. Ce dernier composé peut être réduit par un réducteur approprié, tel que le borohydrure de sodium en présence de trichlorure de cérium au sein d'un alkanol tel que l'éthanol, pour fournir un composé de formule (II) dans lequel $K_{110}$ est un groupe CH(OH) et $Z_1$ est un groupe $NR_{11}$ $R_{12}$ tel que défini.

Ces deux derniers produits peuvent subir les réactions classiques de la chimie organique (addition d'un organométallique pour le premier, acylation ou alkylation pour le deuxième) pour fournir d'autres dérivés de formule (II).

Selon un autre procédé (procédé H) convenant particulièrement aux composés de formule (II) dans laquelle $K_{110}$ est un groupe $C(R_{110})_2$ et où $R_{13}$ à $R_{19}$ ont les mêmes significations que précédemment (y compris les significations préférées), on fait réagir un dérivé de formule (XXXII′), dans lequel $Z_1$ est un groupe $OW_1$ ou $NR_1R_2$ avec un halogénure de benzyle de formule (XXXII′) où $R_{16}$ à $R_{110}$ ont la même signification que précédemment et où Hal désigne un atome d'halogène tel que le chlore ou le brome. La réaction s'effectue en présence d'une base qui est de préférence un alkanoate d'un métal alcalin ou alcalinoterreux ou en présence d'une base minérale telle que les hydroxydes des métaux alcalins en milieu alkanol ou au sein d'un solvant dipolaire aprotique tel que le diméthylsulfoxyde, le diméthylformamide ou la N-méthylpyrrolidone pour fournir un composé de formule (XXXII‴) dans lequel $R_{13}$ à $R_{19}$ et $Z_1$ ont les significations précédentes. Ce composé traité par un acide organique ou minéral, de préférence l'acide polyphosphorique en présence ou non d'un solvant (toluène, xylène) à une température comprise entre 0°C et 150°C, fournit un dérivé de formule (II) dans lequel $K_{110}$ est un groupe $C(R_{110})_2$ et $R_{13}$ à $R_{19}$ ont la signification précédente. Si le dérivé $Z_1$ = $OW_1$ est obtenu, avec $W_1$ alkyle inférieur ou l'atome d'hydrogène, on peut le transformer en dérivé où $Z_1$ = $NR_1R_2$ par les techniques classiques de la chimie organique indiquées dans le premier procédé.

Selon un autre procédé (procédé I, c'et-à-dire i majuscule) convenant particulièrement aux composés de formule (II) dans laquelle $K_{110}$ est un groupe $C(R_{110})_2$ et où $R_{13}$ à $R_{19}$ ont les mêmes significations que précédemment (y compris les significations préférées), on fait réagir un dérivé de formule (XXXII′) dans lequel $Z_1$ est un groupe $OW_1$ ou $NR_{11}$ $R_{12}$ avec un benzaldéhyde substitué par des radicaux $R_{16}$, $R_{17}$, $R_{18}$ ou $R_{19}$. La réaction s'effectue au sein d'un solvant qui peut être un hydrocarbure aromatique ou un acide carboxylique en présence d'un catalyseur qui est préférentiellement une amine disubstituée telle que la pipéridine, on obtient ainsi un composé de formule (XXXXII′) dans lequel les groupes $R_{13}$ à $R_{19}$ et $Z_1$ ont les mêmes significations que précédemment. Ce composé est ensuite réduit par un réducteur approprié, tel que le borohydure de sodium en présence de chlorure de cérium décrit ci-dessus dans le deuxième procédé. L'alcool allylique obtenu est cyclisé par traitement par un acide organique ou minéral en quantité catalytique ou supérieure à la stoéchiométrie en présence ou non d'un solvant (toluène, xylène) à une température comprise entre 0°C et 150°C pour forunir un dérivé de formule (II) dans lequel $K_{110}$ est un groupe $CH_2$ et $R_{13}$ à $R_{19}$ ont la signification précédente. Si le dérivé $Z_1$ = $OW_1$ est obtenu, avec $W_1$ alkyle inférieur ou l'atome d'hydrogène, on peut le transformer en $Z_1$ = $NR_1R_2$ par les techniques classiques de la chimie organique indiquées dans le premier procédé.

On décrit ci-après la préparation des composés de formule (III):

La préparation de composés de formule (III) s'effectue, selon un procédé qui fait aussi partie de l'invention, par une réaction de couplage arylique entre un acide boronique $R_{27}B(OH)_2$ [dans lequel $R_{27}$ peut être un hétérocycle, un phényle ou un groupe vinyle ( ces groupes pouvant être substitués comme indiqué dans la

définition de $R_7$)] et un dérivé bromé de formule (XIII'), étant entendu qu'aucun des autres substituants ne peut être simultanément un atome de brome ou d'iode. Le schéma réactionnel de ce couplage arylique est :

$$(XIII') + R_{27}\ B(OH)_2 \xrightarrow[\text{catalyseur}]{\text{base}} (III)$$

La réaction s'effectue aussi bien avec $Z_2 = OW_2$ ($W_2$ = H ou alkyle, ou métal alcalin) qu'avec $Z_2 = NR_{21}R_{22}$

Comme catalyseur, on peut utiliser des complexes du palladium commerciaux Pd $[(P(C_6H_5)_3]_4$, palladium tétrakistriphénylphosphine ou préparés in situ à partir d'un sel de palladium tel que l'acétate de palladium et d'une phosphine telle que la triphénylphosphine ou la tri-o-tolylphosphine. Comme base, on peut utiliser une base minérale telle que les carbonates et hydrogènocarbonates des métaux alcalins ($Na_2CO_3$, $NaHCO_3$...) ou une base organique telle qu'une amine (triéthylamine). La réaction s'effectue avec une base minérale en milieu hétérogène comprenant une phase organique qui peut être un solvant hydrocarboné tel que le benzène ou le toluène ou un éther à point d'ébullition élevé tel que le glyme ou ses dérivés supérieurs diglyme, triglyme, et une phase aqueuse basique. La réaction peut également s'effectuer avec une base organique en milieu homogène en présence d'un solvant tel que le diméthylformamide ou la N-méthylpyrrolidone. Les acides boroniques utilisés peuvent être des composés commerciaux ou peuvent être préparés par interaction d'un dérivé organométallique $R_{27}M_2$ où $R_{27}$ a la même signification que précédemment, ($M_2$ = Li ou MgX avec X = Cl, Br) avec un borate d'alkyle B $(OR)_3$ (avec R' = $CH_3$, $C_2H_5$, $C_3H_7$, i-$C_3H_7$, $C_4H_9$) suivie d'une hydrolyse acide selon les modes opératoires décrits dans Organic Synthesis coll. vol. 4 page 68 ou dans Journal of organic chemistry 49 pages 5237 - 5243 (1984). Les réactifs sont utilisés dans les proportions suivantes :

| | |
|---|---|
| acide boronique $R_{27}$ B $(OH)_2$ | : 1,05 à 1,50 équivalents |
| base | : 4,00 à 6,00 équivalents |
| catalyseur | : 0,005 à 0,03 équivalents |
| substrat de formule XIII' | : 1,00 équivalent |

La réaction s'effectue à une température comprise entre 50° C et 150° C, de préférence au reflux du mélange réactionnel, pendant une durée allant de deux à quarante huit heures. Le dérivé de formule (III) est isolé par les méthodes habituelles, extraction ou précipitation par adjonction d'eau au mélange réactionnel, et peut être purifié par recristallisation ou chromatographie dans un solvant approprié.

Le dérivé bromé de formule (XIII') peut être obtenu par diazotation de l'amine de formule (XIII")

$$(XIII'') \xrightarrow[\text{CuBr}]{NaNO_2,\ HBr} (XIII')$$

La réaction s'effectue aussi bien avec les dérivés dans la formule desquels $Z_2 = OW_2$ ($W_2$ = H ou alkyle) qu'avec les dérivés dans la formule desquels $Z_2 = NR_{21}R_{22}$. La réaction est réalisée en traitant une solution du dérivé aminé (XIII') dans un acide minéral fort (acide sulfurique ou bromhydrique) contenant éventuellement un acide organique (acide acétique) par un nitrite alcalin (nitrite de sodium) à une température comprise entre -10° C et + 10° C. Le sel de diazonium ainsi obtenu est alors décomposé à des températures comprises entre + 5° C et +90° C, en présence d'acide bromhydrique et de bromure cuivreux.

Le produit de formule (XIII') est isolé par les méthodes habituelles, extraction ou précipitation par adjonction d'eau au mélange réactionnel et peut être purifié par recristallisation ou chromatographie dans un solvant approprié.

L'amine (XIII") est obtenue par une réaction de couplage arylique entre un acide phénylboronique de formule ($A_2$) convenablement substitué par $R_{23}$, $R_{24}$, $R_{25}$ et un dérivé halogéné (bromé ou iodé) de formule (XIII'''), selon le schéma:

$$(XIII''') + (A_2) \xrightarrow[\text{catalyseur}]{\text{base}} (XII'')$$

dans lesquelles formules les radicaux $Z_2$, $R_{26}$, $R_{28}$, et $R_{29}$, sous réserve que $R_{26}$, $R_{28}$ et $R_{29}$ ne soient pas

simultanément un atome de brome ou d'iode, ont la même signification que celles indiquées précédemment et Hal représente un atome d'halogène, en particulier brome ou iode.

La réaction s'effectue aussi bien avec $Z_2$ = $OW_2$ ($W_2$ = H, ou alkyle/ou métal alcalin) qu'avec $Z_2$ = $NR_{21}R_{22}$. Les conditions réactionnelles sont identiques à celles décrites précédemment pour passer de (XIII') à (III).

L'amine (XIII''') peut être obtenue par réduction d'un dérivé nitré de formule (XIII'''').

La réaction s'effectue aussi bien avec les dérivés dans la formule desquels $Z_2$ = $OW_2$ ($W_2$ = H, ou alcoyle) qu'avec les dérivés dans la formule desquels $Z_2$ = $NR_{21}R_{22}$. Comme réducteur on peut utiliser un métal (par exemple : fer, étain) ou un sel de ces métaux (par exemple : chlorure d'étain) en milieu acide minéral (HCl, $H_2SO_4$) ou organique (acide acétique). On peut également utiliser comme réducteur l'hydrogène (éventuellement sous pression) ou ses précurseurs (borohydrure de sodium, dérivés de l'acide formique, cyclohexène) en présence d'un métal de transition tel que le palladium ou le platine. Dans ce cas, on utilise avantageusement comme solvant l'acétate d'éthyle, l'acide acétique ou un alcool tel que l'alcool éthylique. Le produit de formule (XIII''') est isolé par les méthodes habituelles : extraction ou précipitation par adjonction d'eau ou mélange réactionnel, et peut être purifié par recristallisation ou chromatographie dans un solvant approprié.

Les dérivés du type (XIII'''') sont des composés connus.

Pour les composés de formule (III'') et (XIII'), si le dérivé dans la formule desquels $Z_2$ = $OW_2$ est obtenu, il est possible de le transformer en dérivé dans la formule desquels $Z_2$ = $NR_{21}R_{22}$ par les techniques classiques d'aménagement fonctionnel de la chimie organique. Ainsi, lorsque $Z_2$ est $OW_2$ avec $W_2$ = alkyle, il est possible de le transformer en $Z_2$ = $NR_{21}R_{22}$ par chauffage à une température comprise entre 100° C et 200° C, éventuellement sous pression, avec un excès d'une amine $HNR_{21}R_{22}$ selon le schéma suivant :

Composé (III') [composé (III) à groupe CO-$OW_2$] + $HNR_{21}R_{22}$

→

Composé (III') [composé (III) à groupe CO-$NR_{21}R_{22}$] + $W_2$ OH

A partir de composés dans la formule [(III''), (XIII') ou (XIII'''')] desquels $Z_2$ = $OW_2$ avec $W_2$ = alkyle, on peut également obtenir des composés dans la formule desquels $Z_2$ = $OM_2$ où $M_2$ est un métal alcalin, par une réaction de saponification avec une base minérale telle que la soude ou la potasse alcoolique. Ces composés traités par un acide minéral tel que l'acide chlorhydrique ou sulfurique fournissent respectivement des dérivés (III), (XIII') ou (XIII'''') avec $Z_2$ = OH.

A partir du dérivé dans la formule [(III), (XIII') ou (XIII'''')] duquel $Z_2$ = OH, on peut également obtenir le composé dans la formule duquel $Z_2$ = $NR_{21}R_{22}$ après activation de la fonction acide carboxylique par un agent tel que le chlorure de thionyle ($SOCl_2$), le chlorure de phosphoryle ($POCl_3$), le trichlorure ou le pentachlorure de phosphore ($PCl_3$, $PCl_5$), le dicyclohexylcarbodiimide, le diimidazole carbonyle, les chloroformiates d'alcoyle ou l'anhydride trifluoracétique et réaction avec l'amine $HNR_{21}R_{22}$ en présence d'une base organique ou minérale au sein d'un solvant organique tel qu'un solvant chloré ou aromatique ou un éther tel que le TMF.

On décrit ci-après la préparation de composés de formule (IV) :

La préparation de composés de formule (IV) peut s'effectuer par exemple par cyclisation d'un dérivé de formule (XIV')

$$R_{37}-CO-\underset{}{\overset{R_{38}\,|}{CH}}-\overset{R_{39}\,|}{CH}-\overset{CO-Z_3\,|}{CH}-CO-Ar_3 \qquad (XIV')$$

dans laquelle $Ar_3$ est un groupe de formule (XIV'') c'est-à-dire phényle substitué par $R_{33}$ $R_{34}$ et $R_{35}$, et $Z_3$, $R_{33}$ à $R_{39}$ ont la même signification que précédemment,
à chaud en présence d'un donneur d'ammoniac, en milieu acide.

Les dérivés intermédiaires de formule (XIV') sont des composés nouveaux faisant partie de l'invention. Il peuvent être préparés selon un procédé caractérisé en ce qu'on effectue la réaction d'une 3-propiocétone et d'un benzoyl-acétate ou acétamide convenablement substitué selon le schéma suivant :

$$R_{37}-CO-\overset{R_{38}\,|}{CH}-\overset{R_{39}\,|}{CH}-T_3 + Z_3-CO-CH_2-CO-Ar_3$$

$$\longrightarrow (XIV')$$

dans lesquelles formules $Z_3$ et $R_{33}$ à $R_{39}$ ont les mêmes significations que pour la formule (XIV'), $T_3$ désigne un groupe partant tel qu'un atome d'halogène ou un ammonium quaternaire, en présence d'une base minérale forte et d'un solvant.

Dans le cas où le groupe $T_3$ est un ammonium quaternaire, on fait alors réagir un composé, dérivé d'une

base de Mannich, c'est-à-dire un dérivé de formule

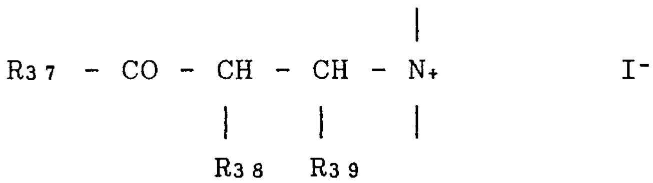

sur un dérivé de formule
$Ar_3 - CO - CH_2 - CO - Z_3$
$AR_3 - CO - CH_2 - CO - Z_3$
en milieu alcalin, en présence d'un solvant de type alcoolique.

Ces derniers dérivés sont en soi connus.

Les dérivés de formule (IV), dans laquelle $Z_3$ est le groupe OH, c'est-à-dire les dérivés de formule (IV") avec $W_3$ = H, peuvent encore être préparés selon un procédé caractérisé en ce que l'on saponifie un ester de formule (IV"), dans laquelle $W_3$ est un radical alkyle inférieur, puis que l'on acidifie.

Enfin on peut passer des dérivés de formule (IV") avec $W_3$ = alkyl inférieur aux dérivés de formule (IV'), dans laquelle $Z_3$ est le groupe $NR_{31}R_{32}$ par saponification, comme indiqué au paragraphe précédent, puis halogénation de ces acides en halogénures d'acides correspondants et réaction de ces halogénures avec une amine de formule $HNR_{31}R_{32}$ dans laquelle $R_{31}$ et $R_{32}$ ont la signification indiquée précédemment.

On décrit ci-après la préparation de produits de formule (V):

Selon un premier procédé, on fait réagir une énone de formule (XV')

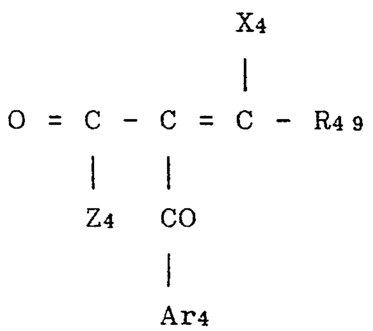
(XV')

dans laquelle
. $Ar_4$ a la formule (XV")
. $R_{43}$, $R_{44}$, $R_{45}$, $R_{49}$ et $Z_4$ ont la même signification que dans la formule (V) et
. $X_4$ est un groupe dialkyl (inférieur)-amino ou alkoxy (inférieur),
avec une amidine de formule

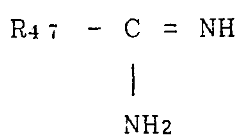

ou un de ses sels de formule

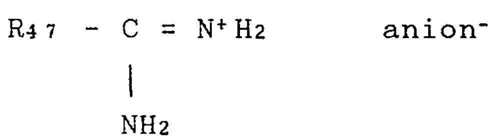

dans lesquelles $R_{47}$ a la même signification que précédemment, en présence d'un solvant en milieu basique. Cette réaction s'effectue avantageusement en solvant organique en particulier alcoolique par exemple le méthanol, l'éthanol, le propanol, en présence de l'alcoolate correspondant ou d'une base organique telle qu'une amine de préférence tertiaire ou d'une base minérale telle qu'un hydroxyde, carbonate ou hydrogénocarbonate, d'un métal alcalin ou alcalino terreux tel que par exemple sodium, potassium ou calcium.

La réaction peut également être effectuée dans l'eau en présence des bases minérales citées ci-dessus.

Dans le cas des dérivés de formule (V'), c'est à dire des dérivés de formule (V) dans laquelle $Z_4$ est un groupe $NR_{41}R_{42}$ tel que défini ci-dessus, on fait réagir un dérivé de formule (V''), c'est à dire un dérivé de formule (V), dans laquelle $Z_4$ est un groupe $OW_4$ tel que défini ci-dessus, avec un amine de formule $HNR_{41}R_{42}$ et élimination de $W_4OH$. Lorsque dans la formule (V''), $W_4$ est un atome d'hydrogène, ces dérivés doivent d'abord subir une activation de la fonction acide par un agent tel que le dicyclohexylcarbodiimide, le diimidazole carbonyle, le chloroformiate d'éthyle ou l'anhydride trifluoroacétique , le chlorure de thionyle ($SOCl_2$), le chlorure de phosphoryle ($POCl_3$), le trichlorure ou le pentachlorure de phosphore ($PCl_3$, $PCl_5$) avant traitement par l'amine $HNR_{41}R_{42}$ en présence d'une base organique au sein d'un solvant organique tel qu'un solvant chloré ou aromatique (notamment un hydrocarbure) ou un éther tel que le THF.

A partir des dérivés de formule (V'), il est possible de préparer d'autres dérivés de formule (V') par les techniques classiques d'aménagement fonctionnel de la chimie organique.

Les dérivés de formule (XV') peuvent être préparés selon un procédé, dans lequel on fait réagir un dérivé de formule (XV''')

$$\begin{array}{l} Ar_4 - CO - CH_2 \\ \qquad\qquad\quad | \\ \qquad\qquad\quad C = O \\ \qquad\qquad\quad | \\ \qquad\qquad\quad Z_4 \end{array} \qquad (XV''')$$

dans laquelle $Ar_4$, $R_{43}$, $R_{44}$ et $R_{45}$, et $Z_4$ ont les significations indiquées ci-dessus avec un dérivé de formule (XV'''').

$$\begin{array}{c} OR_{410} \\ | \\ R_{49} - C - X_4 \\ | \\ OR_{410} \end{array} \qquad (XV'''')$$

dans lequel $R_{49}$ et $X_4$ ont la même signification que ci-dessus, et $R_{410}$ est un radical alkyle inférieur.

Selon un second procédé de préparation des dérivés de formule (V), dans le cas où $R_{47}$ n'est pas un radical alkyl-ou benzyl-sulfinyle ou sulfonyle, on fait réagir un dérivé de formule (XXV') dans laquelle $R_{43}$ à $R_{45}$, $R_{49}$ et $Z_4$ ont les significations indiquées ci-dessus et $A_4$ est un groupe alkyl(inférieur)sulfonyle ou benzylsulfonyle, avec un dérivé de formule $R_{47}H$ dans lequel $R_{47}$ est un radical ayant même signification que $R_7$ sous réserve que le lien valentiel le reliant à l'atome d'hydrogène soit également lié dans $R_{47}$ à un hétéroatome tel que O, S ou $NR_{47}$.

Cette réaction s'effectue en milieu solvant et alcalin avantageusement en solvant protique ou aprotique, en présence d'une base organique ou minérale telle que les hydroxydes, carbonates ou hydrogénocarbonates, des métaux alcalins ou alcalino-terreux tels que par exemple sodium, potassium ou calcium.

Des dérivés de formule (XXV') peuvent être préparés par oxydation d'un dérivé de formule (V) dans laquelle $R_{47}$ est un groupe alkylthio inférieur ou benzylthio, au moyen d'un agent oxydant comme par exemple l'eau oxygénée ou des peracides tels que l'acide peracétique, l'acide métachloroperbenzoïque ou le perphthalate de magnésium.

On décrit ci-après la <u>préparation des composés de formule (VI)</u>.

Selon ce procédé, on fait réagir (Réaction de Michael) une phénylcétone 2-éthylénique de formule (XVI') avec un ester ou un amide β-cétonique, de formule $R_{59}$-CO-$CH_2$-$COZ_5$, dans lesquels $R_{53}$ à $R_{59}$ et $Z_5$ ont la signification précédente, en présence d'une base organique ou minérale, comme par exemple la baryte partiellement deshydratée au sein d'un alkanol inférieur tel que l'alcool éthylique et à une température comprise entre 0°C et la température d'ébullition du solvant utilisé. Le composé de formule (XVI'') obtenu, dans la formule duquel $R_{53}$ à $R_{59}$ et $Z_5$ ont la signification précédente, peut être cyclisé par simple chauffage de 40°C au reflux du solvant en milieu acide carboxylique, de préférence en milieu acide acétique, en présence d'un réactif donneur d'azote tel que l'ammoniac ou un sel d'ammonium organique ou minéral. On obtient ainsi un produit de formule (VI'') ou (VI') dans lequel $R_{53}$ à $R_{59}$ ont même signification que precédemment et avec respectivement $Z_5 = OW_5$ et $Z_5 = NR_{51}R_{52}$.

Si le dérivé de formule (VI'') est obtenu, on peut le transformer en dérivé de formule (VI') par les techniques classiques de la chimie organique, déjà mentionnées dans les procédés précédents.

On décrit ci-après la <u>préparation de composés de formule (VII)</u>.

Les composés selon la formule (VII) dans laquelle $K_{66}$ est 0 et $K_{67}$ est -C($R_{67}$)=, et $K_{610}$ est -C($R_{610}$)= se préparent à partir d'esters ou d'amides β,gammadicétoniques de formule (XVII') par cyclisation dans un alcool

ou un acide carboxylique, tel que l'acide acétique, par simple chauffage du réactif en présence d'une quantité catalytique (0,1 à 5 %) d'acide fort (minéral ou organique, par exemple les acides chlorhydrique ou sulfurique) à une température comprise entre 40 et 120° C, selon la méthode générale décrite par KRAFF et DAAL, Chem. Ber. 21, page 3053 (1888) et reprise par TREBAUL et TESTE, Bull. Soch. Chim Fr page 2272 (1970).

Les composés selon la formule (VII) dans laquelle $K_{66}$ est $NR_{66}$, $K_{67}$ est $-C(R_{67})=$, $K_{610}$ est $-C(R_{610})=$ se préparent à partir d'esters ou d'amides β,gammadicétoniques de formule (XVII') par cyclisation en milieu acide à chaud en présence d'une amine de formule $NHR_{66}$ dans laquelle $R_{66}$ a la signification précédente. Comme solvant on peut utiliser un acide carboxylique tel que l'acide acétique qui joue aussi le rôle de catalyseur. On peut également ajouter des quantités catalytiques d'un acide fort minéral comme par exemple l'acide chlorhydrique ou sulfurique. La réaction s'effectue à une température comprise entre 40° C et la température d'ébullition du solvant utilisé.

Dans le cas où la réaction est conduite avec l'ammoniaque, le produit obtenu a la formule (VII) dans laquelle $K_{66}$ est NH, $K_{67}$ est $-C(R_{67})=$, $K_{610}$ est $-C(R_{610})=$. Ce composé peut être traité par un dérivé halogéné $R_{66}$ Hal où Hal est un halogène (qui peut être un atome de chlore, de brome ou d'iode) en présence d'une base minérale ou organique au sein d'un solvant tel que le diméthylsulfoxyde, le diméthylformamide ou la N-méthylpyrrolidone pour fournir un dérivé de formule (VII) dans laquelle $K_{66}$ est $NR_{66}$, $K_{67}$ est $-C(R_{67})=$ et $K_{610}$ est $-C(R_{610})=$.

Les esters et amides dicétoniques de formule (XVII') peuvent être préparés selon un procédé caractérisé en ce qu'on effectue la réaction d'une halogénocétone (éventuellement substituée par des radicaux $R_{67}$ et $R_{610}$) de formule (XVII'') avec un benzoylacétate ou acétamide convenablement substitué selon le schéma suivant :

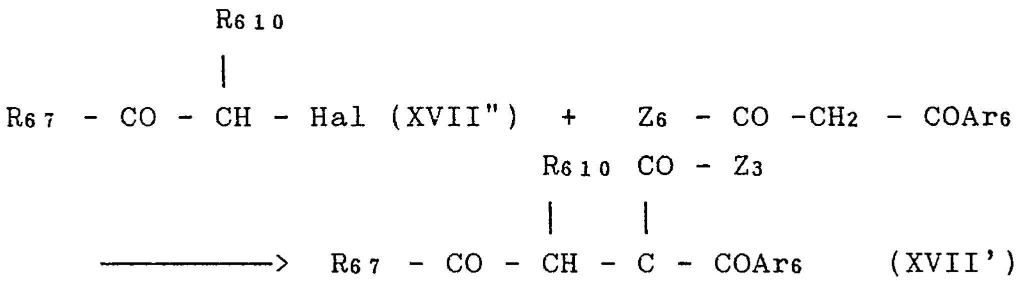

(XVII'') (XVII')

dans laquelle $Ar_6$ est un groupe de formule (XVII'''), c'est-à-dire un phényle substitué par $R_{63}$, $R_{64}$ et $R_{65}$ dans lesquelles formules $Z_6$ et $R_{63}$ à $R_{610}$ ont les mêmes significations que précédemment, Hal désigne un groupe partant tel qu'un atome d'halogène.

Les composés selon la formule (VII) dans laquelle $K_{66}$ est -N = ou-$N(R_{66})$- et $K_{67}$ est -N = ou -$N(R_{67})$-, sous réserve que $K_{66}$ et $K_{67}$ ne soient pas simultanément -$N(R_{66})$- et $N(R_{67})$-, se préparent par réaction d'une énone de formule (XXVII')

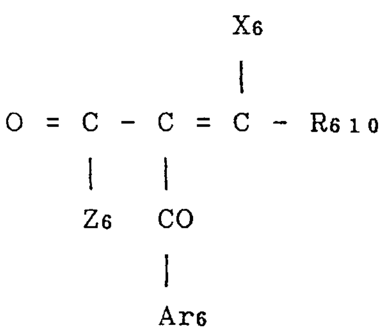

(XXVII')

dans laquelle $Ar_6$ est un groupe de formule (XVII''') c'est-à-dire un phényl substitué par $R_{63}$, $R_{64}$ et $R_{65}$ ; $Z_6$, $R_{610}$ ont la même signification que précédemment et $X_6$ est un groupe dialkyl(inférieur) amino ou alkoxy avec une hydrazine éventuellement substituée par au plus un radical $R_{66}$. La réaction s'effectue au sein d'un alkanol tel que l'alcool éthylique en présence d'une base minérale ou organique telle que la triéthylamine.

Dans le cas où $R_{66}$ est un groupe alkyle inférieur ou aralkyle inférieur, il est possible d'obtenir deux isomères de position de formule (VII') et (VII'') dans lesquels respectivement
$K_{66}$ est -N= et $K_{67}$ est -$N(R_{67})$-
ou
$K_{66}$ est -$N(R_{66})$- et $K_{67}$ est -N=
ce dernier étant généralement majoritaire.

L'énone utilisé comme matière de départ est préparée comme indiqué précédemment dans le procédé de préparation des composés de formule (V).

Pour tous les composés de formule (VII), si le dérivé dans la formule desquels $Z_6 = OW_6$ est obtenu, il est possible de le transformer en dérivé dans la formule desquels $Z_6 = NR_{61}R_{62}$ par les techniques classiques d'aménagement fonctionnel de la chimie organique. Ainsi, lorsque $Z_6$ est $OW_6$ avec $W_6$ = alkyle inférieur, il est

possible de le transformer en $Z_6 = NR_{61}R_{62}$ par chauffage à une température comprise entre 100°C et 200°C, éventuellement sous pression, avec un excès d'une amine $HNR_{61}R_{62}$.

A partir de composés dans la formule (VII) desquels $Z_6$ est O-alkyle, on peut également obtenir des composés dans la formule desquels $Z_6$ est $OM_6$ où $M_6$ est un métal alcalin, par une réaction de saponification avec une base minérale telle que la soude ou la potasse alcoolique. Ce composé, traité par un acide minéral tel que l'acide chlorhydrique ou sulfurique, fournit le dérivé (VII) avec $Z_6$ = OH.

A partir du dérivé dans la formule (VII) duquel $Z_6$ est OH, on peut également obtenir le composé dans la formule duquel $Z_6$ est $NR_{61}$ $R_{62}$ après activation de la fonction acide carboxylique par un agent tel que le chlorure de thionyle ($SOCl_2$), le chlorure de phosphoryle ($POCl_3$), le trichlorure ou le pentachlorure de phosphore ($PCl_3$, $PCl_5$), le dicyclohexylcarbodiimide, le diimidazole carbonyle, les chloroformiates d'alcoyle ou l'anhydride trifluoracétique puis une réaction avec l'amine $HNR_{61}$ $R_{62}$ en présence d'une base organique ou minérale au sein d'un solvant organique tel qu'un solvant chloré ou aromatique (notamment un hydrocarbure) ou un éther tel que le THF.

Les exemples suivants, donnés à titre non limitatif, illustrent l'invention et montrent comment elle peut être mise en oeuvre.

La structure de ces divers exemples a été confirmée par spectrographie de résonance magnétique nucléaire.

Dans ces divers exemples, on a utilisé les abréviations suivantes : PF est le point de fusion exprimé en °C (degré celsius). Lorsque log P n'est pas donné pour un produit, cela signifie que le produit est un intermédiaire chimique et non pas un produit utile contre les atteintes fongiques des plantes.

Les exemples 101 à 170 concernent des produits de la sous-famille de formule (II).

Exemple 101 : 2-(4-Morpholino carbonyl) 5-iodo 3-(3,4-diméthoxy phényl) benzothiophène - (Procédé D ; composé n° 101).

Dans un ballon de 100 ml, on introduit 1,47 g (0,0037 mole) de 2-(4-morpholino carbonyl) 5-amino 3-(3,4-diméthoxy phényl) benzothiophène, 10 ml d'eau distillée et 1,0 ml d'acide sulfurique concentré à 98 %. En même temps qu'on maintient la température du mélange inférieure ou égale à 50°C, on coule progressivement une solution contenant 0,27 g (0,0039 mole) de nitrite de sodium dans 5 ml d'eau. On poursuit l'agitation pendant 1 h, puis verse le mélange réactionnel dans une solution contenant 10 ml d'eau et 0,65 g (0,0039 mole) d'iodure de potassium. Le mélange obtenu est alors progressivement chauffé à 60°C et maintenu à cette température pendant 1 heure. Après refroidissement, le mélange réactionnel est extrait par 3 fois avec 50 ml de chlorure de méthylène chaque fois ; la phase organique est lavée à l'eau, séchée sur sulfate de magnésium et évaporée. La purification s'effectue par chromatographie sur silice et fournit 0,9 g de 2-(4-morpholino carbonyl) 5-iodo 3-(3,4-diméthoxy phényl) benzothiophène fondant à 214°C (rendement 48 % ; composé n° 101).

Exemple 102 : 2-(4-Morpholino carbonyl) 5-(4-chloro benzoylamino) 3-(3,4- diméthoxy phényl) benzothiophène - (Procédé C ; composé n° 102).

Dans un ballon de 100 ml, on introduit successivement 2,0 g (0,005 mole) de 2-(4-morpholino carbonyl) 5-amino 3-(3,4-diméthoxy phényl) benzothiophène, 50 ml de dichlorométhane, 0,74 ml (o,oo52 mole) de triéthylamine et O,70 ml (o,oo52 mole) de chlorure de 4-chloro benzoyle.

Après agitation à 20°C pendant 1 h, on évapore le solvant, lave à l'eau, sèche à l'air puis rince le solide obtenu par 20 ml de dichlorométhane. On obtient ainsi 1,0 g (37 %) de 2-(4-morpholino carbonyl) 5-(4-chlorobenzoylamino) 3-(3,4- diméthoxy phényl) benzothiophène fondant à 247°C. (composé n° 102).

Exemple 103 : 2-(4-morpholino carbonyl) 5-amino 3-(3,4 diméthoxy phényl) benzothiophène - (Procédé B ; composé n° 103).

Dans un ballon de 250 ml, on introduit successivement 10,7 g (0,025 mole) de 2-(4- morpholino carbonyl) 5-nitro 3-(3,4-diméthoxy phényl) benzothiophène, 100 ml d'éthanol absolu, 3 ml d'acide chlorhydrique concentré et 4,2 g (0,075 mole) de poudre de fer. Le mélange réactionnel est porté au reflux deux heures puis versé, après refroidissement, dans 200 ml d'une solution de bicarbonate de sodium saturée.

Après 6 extractions par 100 ml d'acétate d'éthyle, la phase organique est séchée sur sulfate de magnésium, filtrée puis évaporée pour fournir 9,2 g (rendement : 92 %) de 2-(4-morpholino carbonyl) 5-amino 3-(3,4-diméthoxy-3,4 phényl) benzothiophène fondant à 213°C (composé n° 103).

Exemple 104 : 2-(4-morpholino carbonyl) 5-nitro 3-(3,4-diméthoxyphényl) benzothiophène - (Procédé A ; composé n° 104).

Dans un ballon, on introduit successivement 9,0 g (O,OO56 mole) d'alpha-mercaptoacétomorpholide, 13,8 g (O,1 mole) de carbonate de potassium, 14,8 g (O,O46 mole) de 2-chloro 3′, 4′-diméthoxy 5-nitro benzophénone et 200 ml d'éthanol. Le mélange est chauffé au reflux sous agitation pendant deux heures puis refroidi à température ambiante et versé dans 1 l d'eau ; le précipité jaune qui se forme est filtré, rincé à l'eau, puis séché sous courant d'air. On obtient ainsi 18,1 g (92 %) de 2-(4-morpholinocarbonyl) 5-nitro 3-(3,4-diméthoxyphényl) benzothiophène fondant à 144,5°C (composé n° 104).

Exemple 105 : 2-(4-morpholinocarbonyl) 5-(propen-2-yl) 3-(3,4-diméthoxyphényl) benzothiophène (PROCEDE E ; composé n° 105).

Dans un ballon sous atmosphère inerte, on introduit successivement 2,0 g (0,005 mole) de 2-(4-morpholinocarbonyl) 5-bromo 3-(3,4-diméthoxyphényl) benzothiophène préparé selon le procédé A, 50 ml de 1,2-diméthoxy éthane, 0,2 g de palladium tétrakistriphénylphosphine, 0,5 g (0,006 mole) d'acide propen-2-yl boronique et 20 $cm^3$ de carbonate de sodium aqueux 2M. Le mélange est porté au reflux huit heures puis versé dans un mélange d'eau et de glace. Le produit beige obtenu par précipitation est séparé par filtration, rincé à l'eau et séché à l'air. Par chromatographie sur silice, on obtient 1,5 g (rendement = 81,5 %) de composé n° 105 fondant à 135°C.

Exemple 106 : 2-(4-morpholinocarbonyl) 3-(4-méthoxyphényl) benzothiophène (PROCEDE F ; composé n° 106).

Dans un ballon sous atmosphère inerte, on introduit 1,0 g (0,0027 mole) de 5-amino 2-(4-morpholinocarbonyl) 3-(4-méthoxyphényl) benzothiophène et 50 ml de tétrahydrofuranne (THF) distillé. Le mélange est porté au reflux, puis on coule goutte à goutte 0,7 ml (0,0054 mole) de nitrite de tertiobutyle dissous dans 20 ml de THF. Après trois heures, le mélange réactionnel est traité à l'eau, extrait par $CH_2Cl_2$, lavé à l'eau et séché sur sulfate de magnésium. Après évaporation et chromatographie, on isole 0,5 g (rendement = 53 %) de composé n° 106 fondant à 131°C.

Exemple 107 : 2-(N,N-diéthylcarboxamide) 3-(3,4-diméthoxyphényl) benzothiophène (PROCEDE G ; composé n° 107).

Dans un ballon, on introduit 25 g (0,08 mole) d'acide 3-(3,4-diméthoxyphényl) benzothiophène 3-carboxylique préparé selon le procédé D, 300 ml de 1,2-dichloroéthane, 12 ml (0,16 mole) de chlorure de thionyle et 1 ml de DMF. Le mélange est progressivement chauffé au reflux ; le solide se dissout peu à peu pour fournir une solution limpide jaune. Après quatre heures, les solvants sont évaporés pour fournir 24,7 g (rendement = 96 %) de 2-(chlorocarbonyl) 3-(3,4-diméthoxyphényl) benzothiophène.

2,5 g (0,0075 mole) de ce produit sont immédiatement mis à réagir avec 4 $cm^3$ (0,0375 mole) de N,N-diéthylamine dans 100 ml de THF pour fournir après traitement par l'eau et extraction par l'acétate d'éthyle 2,5 g (rendement = 90 %) de 2-(N,N-diéthylcarboxamide) 3-(3,4-diméthoxyphényl) benzothiophène qui cristallise par triturage dans le pentane. PF = 113°C (composé n° 107).

Exemple 108 : 3-(3,4-diméthoxy-phényl) 2-éthoxycarbonyl indénone (procédé G) :

a) A 15 g d'acide 2-(3,4-diméthoxybenzoyl) benzoïque, on ajoute 25 g de chlorure de thionyle et on chauffe à 50°C jusqu'à fin de dégagement gazeux. A la fin de la réaction, on ajoute du toluène et évapore pour éliminer le $SOCl_2$ en excès. Le chlorure d'acide obtenu est un solide orange.

b) Le chlorure d'acide est mis en réaction avec un léger excès d'énolate magnésien du malonate d'éthyle dans le toluène (préparé à partir de malonate d'éthyle et d'éthylate de magnésium). On coule le mélange réactionnel dans l'acide sulfurique dilué et extrait à l'acétate d'éthyle. Après lavage à l'eau et séchage, on évapore le solvant et obtient une huile rouge qui est utilisée telle quelle dans l'étape suivante.

c) L'huile obtenue précédemment est chauffée à reflux avec 300 ml de solution de carbonate de sodium à 5 % pendant 35 minutes. Il se forme un solide rouge. On refroidit, élimine la phase aqueuse par décantation et rajoute 300 ml d'eau ; on chauffe à reflux de nouveau 35 minutes. On refroidit, filtre, lave à l'eau, sèche. On obtient l'indénone sous forme de solide orange fondant à 130°C avec 49 % de rendement (composé n° 108).

Exemple n° 109 : 3-(3,4 diméthoxy phényl)-2-morpholinocarbonyl indénone :

On chauffe 0,9 g de l'indénone obtenue dans l'exemple 108 avec 10 ml de morpholine pendant 1 heure. On coule le mélange réactionnel dans une solution d'acide chlorhydrique dilué et extrait à l'acétate d'éthyle, lave à l'eau et concentre. Le produit est obtenu avec 98 % de rendement (PF = 71,5° ; composé n° 109).

Exemple n°110 : 1-(3,4-diméthoxy phényl) 2-morpholinocarbonyl-3-hydroxy indène :

0,5 g de l'indénone de l'exemple 2 sont réduits par 0,05 g de $NaBH_4$ et 0,5 g de chlorure de cérium dans 20ml de méthanol. La réaction est terminée après 10 minutes et le mélange réactionnel est coulé dans l'eau. On amène le pH à 5 au moyen d'HCl dilué et extrait à l'acétate d'éthyle. On lave à l'eau et évapore le solvant. On obtient 0,49 g d'indénol fondant à 134°C (rendement : 99 % ; composé n° 110).

Exemple n° 111 : 2-morpholinocarbonyl 3-(3,4-diméthoxy phényl)-6-méthoxy indène (procédé H) :

a) Dans un réacteur, on fait réagir 0,8 g de sodium sur 100 ml d'éthanol absolu. On ajoute ensuite 10 g de 3,4-diméthoxy benzoyl acétomorpholide et agite 15 minutes. On ajoute ensuite 5,3 g de chlorure de 3-méthoxy benzyle. On porte au reflux pendant 1 heure 30 minutes et concentre le mélange réactionnel à pression réduite. On reprend par l'eau et HCl dilué et extrait à l'acétate d'éthyle. On obtient une huile

orange.

b) A 5,1 g de cette huile on ajoute 53 g d'acide polyphosphorique et chauffe à 70° C pendant 6 heures. On verse de l'eau dans le mélange réactionnel et agite jusqu'à dissolution puis extrait à l'acétate d'éthyle. On purifie par chromatographie (éluant : acétate d'éthyle). Rendement : 25 %. PF = 115° C (composé n° 111).

Exemple n° 112 : 2-morpholinocarbonyl 3-(3,4-diméthoxy phényl)-5,6-méthoxy indène (procédé I) :

a) On condense le 3,4-(diméthoxy-benzoyl) acétomorpholide avec le vératraldéhyde dans l'acide acétique en présence de pipéridine et d'anhydride acétique. On chauffe à 100° C, coule dans l'eau, extrait à l'acétate d'éthyle et lave la phase organique au $K_2CO_3$ saturé. On purifie sur silice en éluant avec un mélange heptane/acétate d'éthyle 10/90. On obtient un solide rouge fondant à 150° C.

b) La cétone éthylènique est alors réduite par $NaBH_4$ en présence de chlorure de cérium dans le méthanol. On obtient ainsi l'alcool allylique avec 83 % de rendement (fusion 110° C).

c) Dans 80 ml de toluène, on place 2,2 g de l'alcool allylique obtenu précédemment et de l'acide paratoluènesulfonique en quantité catalytique. On porte au reflux pendant 5 heures en éliminant l'eau formée dans la réaction. On coule dans l'eau et décante, lave au bicarbonate de potassium dilué puis encore à l'eau. On obtient 1,9 g de poudre marron clair (composé n° 112).

Les composés suivants ont été préparés en utilisant l'un des procédés précédents. Leur structure et leurs caractéristiques physicochimiques et le procédé d'obtention sont indiqués dans le tableau (I) ci-après.

Ce tableau regroupe en outre les structures et propriétés des produits des exemples 101 à 112. Ces composés possèdent la formule (II) dans laqueLle $R_{15}$ est H et $NR_{11}R_{12}$ est un radical 4-morpholino. Les structures sont données dans le tableau (I) ci-après par le moyen des radicaux $K_{110}$, $R_{13}$, $R_{14}$, $R_{16}$, $R_{17}$, $R_{18}$ et $R_{19}$ de la formule (II).

TABLEAU I

| COMPOSE N° | $K_{110}$ | $R_{13}$ | $R_{14}$ | $R_{16}$ | $R_{17}$ | $R_{18}$ | $R_{19}$ | PF | logP | Procédé |
|---|---|---|---|---|---|---|---|---|---|---|
| 101 | S | $OCH_3$ | $OCH_3$ | H | I | H | H | 214 | 4,4 | D |
| 113 | S | $OCH_3$ | $OCH_3$ | H | Cl | H | H | 166,5 | 3,9 | D |
| 114 | S | $OCH_3$ | $OCH_3$ | H | Br | H | H | 180 | 4,1 | D |
| 102 | S | $OCH_3$ | $OCH_3$ | H | 4-Cl-$C_6H_4$CONH | H | H | 247 | 4,8 | C |
| 115 | S | $OCH_3$ | $OCH_3$ | H | $CH_3$CONH | H | H | 140 | 2,4 | C |
| 116 | S | $OCH_3$ | $OCH_3$ | H | $CF_3$CONH | H | H | 198 | 3,5 | C |
| 103 | S | $OCH_3$ | $OCH_3$ | H | $NH_2$ | H | H | 213 | * | B |
| 104 | S | $OCH_3$ | $OCH_3$ | H | $NH_2$ | H | H | 144,5 | 3 | A |
| 117 | S | $OCH_3$ | $OCH_3$ | H | F | H | H | 146 | 3,4 | A |
| 118 | S | $OCH_3$ | $OCH_3$ | H | H | H | H | 123,5 | 3,3 | A |
| 119 | S | $OCH_3$ | $OCH_3$ | H | H | H | $NO_2$ | 269 | 3 | A |
| 120 | S | $OCH_3$ | $OCH_3$ | H | $C_6H_{50}C_0NH$ | H | H | 173 | 4,5 | C |
| 121 | S | $OCH_3$ | $OCH_3$ | H | H | H | $NO_2$ | 205 | * | B |
| 122 | S | $OCH_3$ | $OCH_3$ | H | H | H | F | 150 | 3,4 | A |
| 123 | S | $OCH_3$ | $OCH_3$ | H | H | H | Br | 205 | 4,1 | D |
| 124 | S | $OCH_3$ | $OCH_3$ | H | H | H | Cl | 186 | 3,9 | A |
| 125 | S | $OCH_3$ | $OCH_3$ | H | H | Cl | H | 192 | 3,9 | A |
| 126 | S | $OCH_3$ | $OCH_3$ | H | H | H | $C_6H_5$ | 191 | 5,0 | E |
| 127 | S | $OCH_3$ | $OCH_3$ | H | H | H | $CH_3$ | 167 | 3,8 | A |
| 128 | S | $OCH_3$ | $OCH_3$ | Cl | H | H | H | 151 | 3,9 | E |
| 129 | S | $OCH_3$ | $OCH_3$ | F | H | H | H | 167 | 3,4 | A |
| 130 | S | $OCH_3$ | $OCH_3$ | CH3 | H | H | H | 150 | 3,8 | E |
| 131 | S | $OCH_3$ | $OCH_3$ | H | H | H | $NHCOCF_3$ | 226 | 3,5 | C |
| 132 | S | $OCH_3$ | $OCH_3$ | H | H | H | $NHCOCH_3$ | 210 | 2,4 | C |
| 133 | S | $OCH_3$ | $OCH_3$ | H | $CF_3$ | H | H | 164 | 4,1 | A |
| 105 | S | $OCH_3$ | $OCH_3$ | H | $H_2C=C(CH_3)$ | H | H | 135 | 4,3 | E |
| 134 | S | $OCH_3$ | $OCH_3$ | H | thienyl-3 | H | H | 188 | 4,7 | E |
| 135 | S | $OCH_3$ | $OCH_3$ | H | $C_6H_5$ | H | H | 208 | 5,0 | E |
| 136 | O | $OCH_3$ | $OCH_3$ | H | $NO_2$ | H | H | 152 | 2,5 | A |
| 137 | O | $OCH_3$ | $OCH_3$ | H | H | H | H | 153 | 2,7 | F |
| 138 | O | $OCH_3$ | $OCH_3$ | H | Cl | H | H | 135 | 3,4 | D |
| 139 | O | $OCH_3$ | $OCH_3$ | H | Br | H | H | 133 | 3,6 | D |
| 140 | O | $OCH_3$ | $OCH_3$ | H | $NH_2$ | H | H | 198 | * | B |
| 141 | $NCH_3$ | $OCH_3$ | $OCH_3$ | H | $NH_2$ | H | H | 190 | 2,7 | A |
| 142 | $NCH_3$ | $OCH_3$ | $OCH_3$ | H | $NH_2$ | H | H | 184 | * | B |
| 143 | S | H | $OCH_3$ | H | $NH_2$ | H | H | 106 | 3,5 | A |
| 144 | S | H | $OCH_3$ | H | $NH_2$ | H | H | 210 | * | B |
| 106 | S | H | $OCH_3$ | H | H | H | H | 131 | 3,7 | F |
| 145 | O | H | $OCH_3$ | H | $NO_2$ | H | H | 141 | 3 | A |
| 146 | O | H | $OCH_3$ | H | $NO_2$ | H | H | 196 | * | B |
| 147 | O | H | $OCH_3$ | H | H | H | H | | 3,2 | F |
| 111 | $CH_2$ | $OCH_3$ | $OCH_3$ | H | H | $OCH_3$ | H | 115 | 2,5 | H |
| 112 | $CH_2$ | $OCH_3$ | $OCH_3$ | H | $OCH_3$ | $OCH_3$ | H | 175 | 2,1 | I |
| 110 | CHOH | $OCH_3$ | $OCH_3$ | H | H | H | H | 134 | 2,0 | G |
| 148 | CHOAc | $OCH_3$ | $OCH_3$ | H | H | H | H | 110 | 2,7 | C |
| 149 | $CH_2$ | H | $OCH_3$ | H | $OCH_3$ | $OCH_3$ | H | 162,4 | 2,5 | I |

* intermédiaires de synthèse

Ac est $CH_3$-CO-

Le tableau (II) regroupe de plus les structures et propriétés physicochimiques de composés de formule (II) dans lesquels $K_{110}$ est un atome de soufre, $R_{13}$ et $R_{14}$ sont des radicaux méthoxy, $R_{15}$, $R_{16}$, $R_{18}$ et $R_{19}$ sont des atomes d'hydrogène ; $R_{17}$ et $NR_{11}R_{12}$ sont indiqués dans le tableau (II). Les composés ont été préparés selon le procédé G.

TABLEAU (II)

| Composé N° | $R_{17}$ | $NR_{11}R_{12}$ | PF | Log P |
|---|---|---|---|---|
| 150 | H | N-méthyléthylamino | 95 | 3,6 |
| 151 | H | bis(méthoxyéthyl)amino | 90 | 3,7 |
| 107 | H | N-diethylamino | 113 | 4,1 |
| 152 | H | 2,6-diméthylmorpholino | 150 | 4,2 |
| 153 | H | piperidino | 110 | 4,3 |
| 154 | H | pyrrolidino | 119 | 3,7 |
| 155 | H | N-diméthylamino | 128 | 3,1 |
| 156 | H | N-méthylhydroxyéthylamino | 84 | 2,8 |
| 157 | H | 2-hydroxyméthyl-piperidino | 60 | 3,8 |
| 158 | H | N-méthyl méthoxyéthylamino | miel | 3,4 |
| 159 | H | 2-méthoxyméthylpiperidino | 123 | 4,5 |
| 160 | H | 2-hydroxyméthylpyrrolidino | 74 | 3,3 |
| 161 | H | 4-hydroxypiperidino | 166 | 2,2 |
| 162 | H | 4-oxopiperidino | 148 | 3,0 |
| 163 | H | 2-méthoxyméthylpyrrolidino | 60 | 3,9 |
| 164 | H | 3-hydroxypiperidino | 90 | 3,1 |
| 165 | H | 4-méthoxypiperidino | 139 | 2,8 |
| 166 | H | 3-méthoxypiperidino | 60 | 3,8 |
| 167 | F | N-méthyléthylamino | 103 | 3,8 |
| 168 | F | 2,6-diméthylmorpholino | 134 | 4,4 |
| 169 | F | N-méthylméthoxyéthylamino | miel | 3,6 |
| 170 | F | bis(diméthoxyéthyl)amino | 99 | 3,9 |

Exemple 171 à 184 : 2-chloro 3′, 4′-diméthoxy 5-nitro benzophénone (produit intermédiaire pour la préparation des produits de formule (II) dans lesquels $K_{110}$ est O, S ou -N($R_{110}$).

Dans un ballon, on introduit successivement 11 g (0,05 mole) de chlorure de 2-chloro 5-nitro benzoyle, 20ml (0,15 mole) de 1,2-diméthoxy benzène et 0,5 g de chlorure ferrique anhydre. Le mélange est chaufée progressivement à 150°C et maintenu à cette température pendant 1 heure. Après refroidissement, le mélange réactionnel est versé dans 200 ml HCl 1N puis extrait par 3 fois à l'aide de 200 ml de dichlorométhane chaque fois.

Après lavage par une solution de bicarbonate de sodium, la phase organique est séchée sur $MgSO_4$ puis concentrée sous pression réduite pour fournir un huile brune. Par trituration avec 300 ml de pentane, on isole 12,5 g (rendement : 78 %) de 2-chloro 3′, 4′-diméthoxy 5-nitro benzophénone sous forme d'un solide beige fondant à 138°C (composé n° 171).

Les composés qui suivent on été obtenus dans des conditions analogues : leurs structures et leurs caractéristiques physico-chimiques sont indiquées dans le tableau ci-après. Ces composés sont des produits de formule (XII′) dans laquelle $R_{15}$ est H, $R_{14}$ est un radical méthoxy et $A_1$, $R_{13}$, $R_{16}$ $R_{17}$ $R_{18}$ et $R_{19}$ ont les significations indiquées dans le tableau (III) figurant plus bas.

Les exemples 201 à 253 concernent des produits de la sous-famille (II) de formule (III).

Exemple 201 : 2-(3,4-diméthoxy phényl) 4-(4-fluorophényl) 1-morpholinocarbonyl benzène :

Dans un ballon de 100 ml, on introduit 1,6 g (0,00454 mole) d'acide 2-(3,4-diméthoxy phényl) 4-(4-fluoro phényl) benzoïque (composé 204), 50 ml de tétrahydrofuranne et 0,92 g (0,0057 mole) de diimidazolecarbonyle. On agite à 20°C pendant 2 heures. On ajoute alors 1 ml (0,0113 mole) de morpholine et maintient sous agitation pendant six heures. Le mélange réactionnel est versé sur 150 ml d'eau distillée, le précipité est filtré, lavé avec 100 ml d'eau puis séché à l'air. On obtient ainsi 1,3 g (rendement : 68,4 %) de 2-(3,4-diméthoxy phényl) 4-(4-fluorophényl) 1-morpholinocarbonyl benzène sous forme d'un solide blanc fondant à 153°C (composé 201).

On a obtenu de façon identique les composés n° 201 à 231. Ces composés ont pour formule la formule (III) dans laquelle $R_{23}$ et $R_{24}$ sont des groupes méthoxy, $R_{25}$, $R_{28}$ et $R_{29}$ sont l'atome d'hydrogène et $R_{26}$, $R_{27}$ et $Z_2$ ont les significations données ci-après dans les tableaux (IV) et (IV′) figurant plus bas.

Un autre sous-ensemble de produits ont pour formule la formule (III) dans laquelle $R_{23}$ et $R_{24}$ constituent ensemble un radical

TABLEAU III

| Composé N° | $R_{16}$ | $R_{13}$ | $A_1$ | $R_{17}$ | $R_{18}$ | $R_{19}$ | F°C |
|---|---|---|---|---|---|---|---|
| 171 | H | $CH_3O$ | Cl | $NO_2$ | H | H | 138 |
| 172 | H | $CH_3O$ | Cl | H | $NO_2$ | H | 140 |
| 173 | H | $CH_3O$ | F | H | H | H | 84 |
| 174 | H | $CH_3O$ | F | F | H | H | 125 |
| 175 | H | $CH_3O$ | Cl | H | H | $NO_2$ | 139,5 |
| 176 | H | $CH_3O$ | F | H | H | F | 189 |
| 177 | H | $CH_3O$ | F | H | H | Cl | 167 |
| 178 | H | $CH_3O$ | F | H | Cl | H | 134 |
| 179 | H | $CH_3O$ | F | H | $NO_2$ | H | 140 |
| 180 | H | $CH_3O$ | F | H | H | $CH_3$ | 110 |
| 181 | F | $CH_3O$ | F | H | H | H | 102 |
| 182 | H | $CH_3O$ | F | $CF_3$ | H | H | 79 |
| 183 | H | H | Cl | H | $NO_2$ | H | 103 |
| 184 | F | $CH_3O$ | F | F | F | F | 77 |

TABLEAU (IV)

| N° | $Z_2$ | $R_{27}$ | PF | log P |
|---|---|---|---|---|
| 201 | morpholino | 4-fluorophényl | 153 | 4,1 |
| 202 | morpholino | phényl | 130 | 4,0 |
| 203 | morpholino | 4-méthylphényl | 129 | 4,6 |
| 204 | $N(CH_3)CH_2CH_2OCH_3$ | Br | miel | 3,2 |
| 205 | $N(C_2H_5)_2$ | Br | 82 | 4,0 |
| 206 | $N(CH_3)C_2H_5$ | Br | 85 | 3,4 |
| 207 | pyrrolidino | Br | 96 | 3,5 |
| 208 | morpholino | Cl | 108 | 2,9 |
| 209 | $N(CH_3)C_2H_5$ | Cl | 68 | 3,3 |
| 210 | $N(CH_3)CH_2CH_2OCH_3$ | Cl | miel | 3,1 |
| 211 | $N(C_2H_5)_2$ | Cl | 54 | 3,8 |
| 212 | $N(CH_3)C_2H_5$ | propen-2-yl | 98 | 3,6 |
| 213 | $N(CH_3)CH_2CH_2OCH_3$ | propen-2-yl | 78 | 3,4 |
| 214 | $N(C_2H_5)_2$ | propen-2-yl | 68 | 4,1 |
| 215 | $N(CH_3)C_2H_5$ | p-tolyl | 130 | 5,0 |
| 216 | $N(CH_3)CH_2CH_2OH$ | p-tolyl | 130 | 4,2 |
| 217 | $N(CH_3)_2$ | p-tolyl | 109 | 4,5 |
| 218 | $N(CH_3)C_2H_5$ | $CH_3$ S | 110 | 3,2 |
| 219 | $N(C_2H_5)_2$ | $CH_3S$ | 73 | 3,7 |
| 220 | morpholino | $CH_3S$ | 132 | 2,8 |
| 221 | $N(CH_3)CH_2CH_2OCH_3$ | $CH_3S$ | 63 | 3,0 |
| 222 | morpholino | Br | 123 | 3,0 |

TABLEAU (IV')

| N° | $Z_2$ | $R_{16}$ | PF | log P |
|---|---|---|---|---|
| 223 | morpholino | Br | 55 | 3,0 |

divalent méthylènedioxy $R_{25}$, $R_{26}$, $R_{28}$ et $R_{29}$ sont l'atome d'hydrogène et $R_{27}$ et $Z_2$ ont les significations données ci-après dans le tableau (V).

TABLEAU (V)

| N° | $Z_2$ | $R_{27}$ | PF | log P |
|---|---|---|---|---|
| 224 | morpholino | Br | 145°C | 2,9 |
| 225 | $N(CH_3)C_2H_5$ | Br | miel | 3,4 |
| 226 | $N(CH_3)CH_2CH_2OCH_3$ | Br | miel | 3,2 |
| 227 | $N(C_2H_5)_2$ | Br | miel | 3,9 |
| 228 | $N(CH_3)C_2H_5$ | 4-fluorophényl | 98°C | 4,5 |
| 229 | morpholino | 4-fluorophényl | 170°C | 4,0 |
| 230 | $N(CH_3)C_2H_5$ | p-totyl | 148°C | 5,0 |
| 231 | morpholino | p-totyl | 137°C | 4,5 |

Exemple 232 : acide 2-(3,4-diméthoxy phényl) 4-(4-fluoro phényl) benzoïque (composé 232) :

Dans un ballon de 100 ml, on introduit 2,8 g (0,074 mole) de 2-(3,4-diméthoxy phényl) 4-(4-fluoro phényl) benzoate d'éthyle, 50 ml d'éthanol absolu et 2 ml de soude 10 N. Le mélange est porté au reflux pendant deux heures puis versé sur 150 ml d'acide chlorhydrique 1N. Le précipité est filtré sur verre fritté, lavé à l'eau, puis séché à l'air. On obtient ainsi 2,1 g (rendement : 81 %) d'acide 2-(3,4-diméthoxy phényl) 4-(4-fluoro phényl) benzoïque sous forme d'un solide blanc fondant à 210°C.

Exemple 233 : 2-(3,4-diméthoxy phényl) 4-(4-fluoro phényl) benzoate d'éthyle (composé 233) :

Dans un ballon tricol de 250 ml, on introduit 30 ml de 1,2-diméthoxyéthane dégazé par l'argon puis ajoute 3,65 g (0,010 mole) de 4-bromo 2-(3,4-diméthoxy phényl) benzoate d'éthyle (composé 209), 0,1 g de tétrakis (triphényl) phosphine palladium, 1,7 g (0,012 mole) d'acide 4-fluorophényl boronique et 20 ml de carbonate de sodium 2M. Après 8 heures de reflux, le mélange réactionnel est versé sur 100 ml d'eau distillée. On effectue ensuite une extraction par 2 fois 100 ml de dichlorométhane chaque fois, un lavage par 100 ml d'eau, un séchage sur sulfate de magnésium et passage sur une couche de silice, puis une évaporation. On obtient ainsi 3,4 g (rendement: 89,5 %) de 2-(3,4-diméthoxy phényl) 4-(4-fluoro phényl) benzoate d'éthyle fondant à 127°C (composé 233).

On a obtenu de façon identique les composés de formule (III) dans laquelle $R_{23}$ et $R_{24}$ sont des groupes méthoxy, et $R_{25}$, $R_{26}$ $R_{28}$ $R_{29}$ sont l'atome d'hydrogène ; $R_{27}$ et $Z_2$ ont les significations indiquées dans le tableau (VI) ci-après.

TABLEAU (VI)

| Composé N° | $Z_2$ | $R_{27}$ 1 | PF | Log P |
|---|---|---|---|---|
| 233 | $OC_2H_5$ | 4-F-phényl | 127 | * |
| 234 | morpholino | 3,4-diméthoxy phényl | 189 | 3,4 |
| 235 | morpholino | $(CH_3)_2C=CH-$ | 97 | 3,8 |
| 236 | morpholino | $H_2C=C(CH_3)-$ | 128 | 3,2 |
| 237 | morpholino | 4-chloro phényl | 144 | 4,7 |
| 238 | morpholino | 3-thiényl | 151,5 | 3,6 |
| 239 | morpholino | 4-pipéronyl | 138 | 3,4 |
| 240 | $OC_2H_5$ | 4-méthoxyphényl | 125 | * |
| 241 | OH | p-tolyl | 215 | * |
| 242 | $OC_2H_5$ | p-tolyl | 84 | * |
| 243 | OH | 4-méthoxyphényl | 202 | * |

* intermédiaire de synthèse

On a également obtenu les composés de formule (III) dans laquelle $R_{23}$ et $R_{24}$ constituent ensemble un radical divalent méthylène dioxo, $R_{25}$, $R_{26}$, $R_{28}$ et $R_{29}$ sont l'atome d'hydrogène et $R_{27}$ et $Z_2$ ont les significations données ci-après dans le tableau (VII).

TABLEAU (VII)

| N° | $Z_2$ | $R_{27}$ | PF |
|---|---|---|---|
| 244 | OH | Br | 194 |
| 245 | $OC_2H_5$ | 4-fluoro-phényl | 72 |
| 246 | $OC_2H_5$ | p-tolyl | 98 |
| 247 | OH | 4-fluorophényl | 177 |
| 248 | OH | p-tolyl | 180 |
| 249 | OH | phényl | 177 |

Exemple 250 : 4-bromo 2-(3,4-diméthoxyphényl) benzoate d'éthyle (composé n° 250) :

Dans un ballon tricol de 1000 ml, on introduit 20,0 g (0,066 mole) de 4-amino 2-(3,4-diméthoxy-phényl)benzoate d'éthyle (composé 252) et 200 ml d'acide acétique. Après dissolution, on ajoute 60 ml d'acide bromhydrique à 47 %, refroidit à 0° C puis ajoute 150 ml d'eau distillée. On coule 4,6 g (0,066 mole) de nitrite de sodium dissous dans 30 ml d'eau. On poursuit l'agitation entre 0 et 5° C pendant une heure puis verse le mélange sur une solution de bromure cuivreux (0,066 mole, 9,5 g) dans 100 ml d'acide bromhydrique à 47 %. Le mélange réactionnel est chauffé à 65° C pendant une heure puis versé dans l'eau. Le précipité formé est filtré, rincé à l'eau puis séché à l'air. On obtient ainsi 18,4 g (rendement : 76,3 %) de 4-bromo 2-(3,4-diméthoxy phényl) benzoate d'éthyle fondant à 53° C (composé 250).

On a obtenu de façon identique les composés de formule (III) dans laquelle $R_{23}$, $R_{24}$ sont des groupes méthoxy, $R_{25}$, $R_{26}$ $R_{28}$, $R_{29}$ sont l'atome d'hydrogène ; $Z_2$ a la signification donnée ci-après et $R_{27}$ est l'atome de brome.

| Composé N° | $Z_2$ | $R_{27}$ | PF |
|---|---|---|---|
| 250 | $OC_2H_5$ | Br | 53 |
| 251 | OH | Br | 181,5 |

Exemple 252 : 4-amino 2-(3,4-diméthoxy phényl) benzoate d'éthyle (composé 252) :

Dans un ballon de 500 ml, on introduit 200 ml de 1,2-diméthoxy-1,2 éthane, puis 17 g (0,07 mole) de 4-amino 2-bromo benzoate d'éthyle (composé 218), 0,3 g de tetrakis (triphényl) phosphine et 100 ml de carbonate de sodium 2M. Après 8 heures d'ébulition à reflux le mélange réactionnel est versé sur 600 ml d'eau. On filtre, rince à l'eau et sèche à l'air le solide obtenu. On rince par 50 ml de pentane. On obtient ainsi 20,0 g (rendement : 95 %) de 4-amino 2-(3,4-diméthoxy phényl) benzoate d'éthyle fondant à 127° C (composé 252).

On a obtenu de façon identique les produits de formule (III) dans laquelle $R_{23}$, $R_{24}$ sont des groupes méthoxy, $R_{25}$, $R_{28}$ et $R_{29}$ sont l'atome d'hydrogène ; $Z_2$, $R_{27}$ et $R_{26}$ ont les significations données ci-après.

| Composé N° | $Z_2$ | $R_{27}$ | $R_{26}$ | PF |
|---|---|---|---|---|
| 252 | $OC_2H_5$ | $NH_2$ | H | 127 |
| 253 | OH | H | $NO_2$ | 182 |

Exemple 254 : 4-amino 2-bromo benzoate d'éthyle (composé 254) :

Dans un ballon de 1 litre, on introduit successivement 46 g (0,17 mole) de 2-bromo 4-nitro benzoate d'éthyle, 250 ml d'éthanol, 28 g (0,5 mole) de poudre de fer et 20 ml d'acide chlorhydrique concentré. Le mélange est porté au reflux pendant quatre heures. Après refroidissement le mélange réactionnel est traité par 500 ml d'une solution saturée de bicarbonate de sodium et extrait en 5 fois par 300 ml d'acétate d'éthyle chaque fois. Après séchage sur sulfate de magnésium, filtration et évaporation, on isole 35,2 g (rendement : 86 %) de 4-amino 2-bromo benzoate d'éthyle fondant à 93° C (composé n° 254).

Les exemples 301 à 399 et 3100 à 3119 illustrent la préparation de composés de formule (IV).

Exemple n° 301 : N-(2-(3,4-diméthoxyphényl)-6-phényl nicotinoyl) morpholine (composé n° 301) procédé (b) :

A une solution de 1,9 g (0,005 mole) du chlorure de 2-(3,4-diméthoxy phényl 6-phényl) nicotinoyle (composé n° 2) dans 60 ml de tétrahydrofurane, on coule goutte à goutte, à 25-30° C, 1,8 g (0,002 mole) de morpholine. Le chlorhydrate précipite dès le début de coulée. Après la fin d'addition on laisse réagir 10 mn, on dilue le milieu avec 200 ml d'acétate d'éthyle. On lave le milieu 2 fois avec 100 ml d'eau. La solution est séchée sur sulfate de magnésium anhydre puis concentrée sous pression réduite. On obtient une huile qui cristallise dans l'éther. Après filtration et séchage on obtient 1,9 g d'une poudre beige (rendement : 95 %) fondant à 185,5° C.

Exemples n° 304-308 : chlorure de 2-(3,4-diméthoxy-phényl) 6-phényl nicotinoyle (composé n° 302) :

On chauffe un mélange de 1,8 g (0,0054 mole) de l'acide 2-(3,4-diméthoxyphényl) 6-phényl nicotinique (composé n° 303) et 20 ml de chlorure de thionyle 1 h à l'ébullition à reflux. Le milieu est concentré sous pression réduite. On obtient 1,9 g (rendement = 100 %) d'un miel jaune.

En opérant ainsi on obtient les chlorures de nicotinoyle de formule (IV'''') dans laquelle $Z_3$ est l'atome de chlore et $R_{371}$ a les significations indiquées ci-après.

| Composé N° | $R_{371}$ | PF |
|---|---|---|
| 304 | H | miel |
| 305 | Cl | 114 |
| 306 | F | 114 |
| 307 | Br | 106 |
| 308 | NC | 160 |

Exemple n° 303 et 310 à 313 : acide 2-(3,4-diméthoxyphényl) 6-phényl nicotinique (composé n° 303) :

A une solution de 3,6 g (0,01 mole) du 2-(3,4-diméthoxyphényl) 6-phényl nicotinate d'éthyle (composé n° 309) dans 100 ml d'alcool à 95 % on ajoute 2 ml de soude à 30 %. On chauffe le milieu 2 h à 60-70° C. On concentre le milieu sous pression réduite et dissout le résidu dans 100 ml d'eau. On acidifie le milieu avec 2 ml d'acide chlorhydrique concentré : une gomme relargue. On extrait avec 100 ml d'acétate d'éthyle. La solution est lavée 2 fois avec 100 ml d'eau, séchée sur sulfate de magnésium anhydre puis concentrée sous pression réduite. On obtient une meringue qui cristallise par trituration dans 50 ml d'éther. Après filtration et séchage on obtient 2,5 g (rendement = 75 %), d'une poudre beige fondant à 178° C. En opérant de la même façon, on obtient les composés de formule (IV'''') dans laquelle $Z_3$ est le groupe hydroxy et $R_{361}$ a les significations indiquées ci-après.

| Composé N° | $R_{371}$ | PF | Log P |
|---|---|---|---|
| 303 | H | 178 | |
| 310 | Cl | 145 | |
| 311 | F | 170 | |
| 312 | Br | 162 | |
| 313 | CN | 163 | |

Exemple 309 et 315 à 317 : 2-(3,4-diméthoxyphényl) 6-phényl nicotinate d'éthyle (composé n° 309) : procédé a' :

On chauffe à reflux pendant 4 heures, un mélange de 7,7 g (0,02 mole) de 2-(3,4-diméthoxybenzoyl) 5-oxo 5-phénylvalérate d'éthyle (composé n° 314), 3,1 g (0,04 mole) d'acétate d'ammonium et 30 ml d'acide acétique. On verse le milieu réactionnel dans 300 ml d'eau : une huile relargue. Celle-ci est extraite avec 200 ml d'acétate d'éthyle. La solution organique est lavée 3 fois avec 100 ml d'eau puis séchée sur sulfate de magnésium anhydre et concentrée sous pression réduite. On obtient 4,5 g d'une huile brun clair (rendement : 62 %) correspondant au 2-(3,4-diméthoxyphényl) 6-phényl nicotinate d'éthyle.

En opérant de la même façon on obtient les composés de formule (IV'''') dans laquelle $Z_3$ est éthoxy et $R_{371}$ a les significations suivantes :

| Composé N° | $R_{371}$ | Caractéristiques physiques |
|---|---|---|
| 315 | phényl | miel |
| 316 | 4-chloro phényl | miel |
| 317 | 4-fluoro phényl | miel ; indice de réfraction = 1,611 |

Exemple 318 à 320 :

En opérant selon le procédé a, comme à l'exemple précédent, à l'aide des intermédiaires correspondants convenablement substitués, on a obtenu les composés ci-dessous de formule (IV'''') avec $Z_3$ = éthoxy.

| Composé N° | $R_{371}$ | Caractéristiques physiques |
|---|---|---|
| 318 | 4-bromo phényl | miel |
| 319 | 4-cyano phényl | PF = 118°C |
| 320 | 3-thienyl | miel |

Exemple 321 à 344 : 2-(3,4-diméthoxybenzoyle) 5-oxo 5-phényl valérate d'éthyle (composé n° 314) :

A une solution alcoolique de 13,7 g (0,05 mole) du sel de sodium du (3,4-diméthoxybenzoyl) acétate d'éthyle on ajoute 8,4 g (0,05 mole) de 3-chloro propiophénone : la réaction est exothermique et la température du milieu s'élève progressivement de 24°C à 35°C. On laisse réagir sous agitation pendant 3/4 heure (retour à température ambiante). On verse le milieu réactionnel dans 400 ml d'eau ; le produit relargue sous forme de gomme. Celle-ci est extraite avec 300 ml de chloroforme.

La solution chloroformique est lavée 5 fois avec 200 ml d'eau puis séchée sur sulfate de magnésium anhydre et concentrée sous pression réduite. On obtient 19,5 g d'un miel brun. Le produit cristallise après dissolution dans 100 ml d'éther. Après filtration du précipité et séchage du gâteau on obtient 15 g, (rendement : 77 %) d'une poudre beige fondant à 70°C correspondant au 2-(3,4-diméthoxybenzoyle) 5-oxo 5-phényl valérate d'éthyle.

En opérant de la même façon, on obtient les composés rassemblés dans les tableaux suivants et de formule :

```
(3,4-diméthoxyphényl) - CO - CH - CO - Z3
                             |
                             CH2 - CH - CO - R37
                                   |
                                   R38
```

| Composé N° | $R_{37}$ | $R_{38}$ | $Z_3$ | PF |
|---|---|---|---|---|
| 321 | $C_6H_5$ | H | $OC_2H_5$ | 70 |
| 322 | 4-Cl $C_6H_4$ | H | $OC_2H_5$ | 109 |
| 323 | 4-F $C_6H_4$ | H | $OC_2H_5$ | 100 |
| 324 | 4-Br $C_6H_4$ | H | $OC_2H_5$ | 98 |
| 325 | t-$C_4H_9$-$C_6H_4$ | H | morpholino | 134 |
| 326 | 4-Cl $C_6H_4$ | $CH_3$ | morpholino | 98 |
| 327 | 4-F $C_6H_4$ | $CH_3$ | morpholino | 88 |
| 328 | t-$C_4H_9$- | H | morpholino | 145 |
| 329 | $(CH_3)_2$ = CH- | H | morpholino | 114 |
| 330 | 4-Br $C_6H_4$ | H | $C_2H_5O$- | 98 |
| 331 | 4-Cl $C_6H_4$ | H | morpholino | 145 |
| 332 | 4-$C_6H_5$-$C_6H_4$ | H | morpholino | 178 |
| 333 | 4-$CH_3$ $C_6H_4$ | H | morpholino | 148 |

| Composé | $R_{37}$ | $R_{38}$ | $Z_3$ | PF |
|---|---|---|---|---|
| 334 | 4-Br $C_6H_4$ | H | morpholino | 164 |
| 335 | 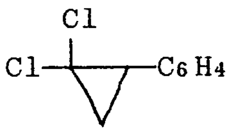 | H | morpholino | 90 |
| 336 | 2-Cl $C_6H_4$ | H | morpholino | 104 |
| 337 | 4-iso $C_3H_7$-$C_6H_4$ | H | morpholino | 100 |
| 338 | 4-$CH_3O$ $C_6H_4$ | H | morpholino | 130 |
| 339 | 2-thienyl | H | morpholino | 146 |
| 340 | 3-thienyl | H | morpholino | 148 |
| 341 | 1-naphthyl | H | morpholino | 160 |
| 342 | 2-naphthyl | H | morpholino | 150 |
| 343 | 2-furyl | H | morpholino | 150 |
| 344 | 2-$CH_3$-$C_6H_4$ | H | morpholino | 132 |

Exemple 345 : Préparation de la N-[5-(4-chlorophényl) 2-(3,4 diméthoxybenzoyl) 5-nicotinoyl] morpholine (Composé 345) (procédé a) :

On dissout 46g (0,1 mole) de N-[5-(4-chlorophényl) 2-(3,4-diméthoxybenzoyl) 5-oxo valéryl] morpholine (Composé 346) et 15,4 g (O,2 mole) d'acétate d'ammonium dans 150 ml d'acide acétique. On chauffe 4 h à l'ébulition à reflux tout en faisant barbotter un courant d'air dans le milieu. On refroidit à température ambiante, on verse le milieu dans 0,5 l d'eau : une gomme relargue. On extrait celle-ci avec 200 ml de chlorure de méthylène. On lave à l'eau la solution chlorométhylènique, puis on traite la solution au charbon actif. On concentre la solution. On obtient un miel brun qui cristallise par trituration dans 200 ml d'éther. Le produit cristallisé est filtré, le gâteau est lavé à l'éther puis séché sous pression réduite. On obtient ainsi 21,2 g d'une poudre beige fondant à 152°C (rendement : 48 %).

Exemple 345 à 394 :

En opérant selon l'un des modes opératoires décrit aux exemples 345 (procédé a) et 301 (procédé b) à partir des intermédiaires convenablement substitués, on obtient le dérivé de formule (IV'''') dont les substituants, le procédé utilisé et les caractéristiques physiques figurent dans le tableau qui suit.

Les radicaux morpholino substitués sont numérotés à partir de l'atome d'oxygène, la valence libre étant

portée par l'atome d'azote. L'astérisque signifie que le composé est un intermédiaire de synthèse.

| Composé N° | Xn | $R_{38}$ | $NR_{31}R_{32}$ | procédé | PF | Log P |
|---|---|---|---|---|---|---|
| 301 | - | H | morpholino | a | 185,5 | 3,3 |
| 345 | 4-Cl | H | morpholino | b | 148 | 4,0 |
| 347 | 4-Cl | H | $NCH_3(C_2H_5)$ | b | 156 | 4,4 |
| 348 | 4-Cl | H | $(CH_3OCH_2CH_2)_2N$ | b | miel | 4,6 |
| 349 | 4-F | H | morpholino | b | 180 | 3,5 |
| 350 | 4-F | H | $NCH_3\ (C_2H_5)$ | b | 117 | 4,4 |
| 351 | 4-F | H | $(CH_3OCH_2CH_2)_2N$ | b | indice de réfraction 1,587 | 4,0 |
| 352 | 4-F | H | $CH_3OCH_2CH_2NH$ | b | 138 | * |
| 353 | 4-F | H | $CH_3OCH_2CH_2N$(–$CH_3$) | b | miel | 3,7 |
| 354 | - | H | $CH_3OCH_2CH_2N$(–$CH_3$) | b | miel | 3,5 |

| Composé | Xn | $R_{38}$ | $NR_{31}R_{32}$ | Procédé | PF | Log P |
|---|---|---|---|---|---|---|
| 355 | 4-CH-CH<br>Cl Cl | H | morpholino | a | 159 | 4,7 |
| 356 | 4-$iC_3H_7$ | H | morpholino | a | 179 | 4,9 |
| 357 | 4-Cl | H | 2-méthoxy pyrrolidino | b | 60 | 4,5 |
| 358 | 4-Cl | H | $NCH_3(nC_3H_7)$ | b | miel | 5,0 |
| 359 | - | H | $CH_3$<br>\|<br>$N-CH_2-CH=CH_2$ | b | verre | 3,7 |
| 360 | - | H | $N\ CH_3(i-C_3H_7)$ | b | verre | 4,0 |
| 361 | - | H | $N\ CH_3(n-C_4H_9)$ | b | verre | 4,7 |
| 362 | 4-Cl | H | $NCH_3(n-C_4H_9)$ | b | miel | 5,5 |
| 363 | 4-Cl | H | $NCH_3(CH_2-CN)$ | b | miel | 3,6 |
| 364 | 4-Cl | H | $(C_2H_5)_2N-$ | b | miel | 5,0 |
| 365 | - | H | $NCH_3(n-C_3H_7)$ | b | miel | 4,2 |

| Composé N° | Xn | $R_{38}$ | $NR_1R_2$ | procédé | PF | Log P |
|---|---|---|---|---|---|---|
| 367 | 4-Cl | $CH_3$ | morpholino | a | 186 | 4,70 |
| 368 | 4-F | $CH_3$ | morpholino | a | 180 | 4,1 |
| 369 | H | H | $NCH_3(C_2H_5)$ | b | 102 | 3,7 |
| 370 | H | H | $(CH_3)_2N$ | b | 70 | 3,2 |
| 371 | H | H | $CH_3O(CH_2)_2$-NH | b | 101 | * |
| 372 | H | H | tetrahydro furfurylamino | b | 143 | * |
| 373 | 4-Cl | H | pyrrolidino | b | 150 | 4,6 |
| 374 | 4-Cl | H | 2,6-diméthyl morpholino | b | 100 | 5,0 |
| 375 | 4-Cl | H | tetrahydro furfurylamino | b | 128 | * |
| 376 | 4-Cl | H | pipéridino | b | 144 | 5,1 |
| 377 | 4-Cl | H | 4-hydroxy piperidino | b | 150 | 3,0 |

| Composé N° | Xn | $R_{38}$ | $NR_{31}R_{32}$ | procédé | PF | Log P |
|---|---|---|---|---|---|---|
| 378 | 4-Cl | H | $CH_3$<br>furfuryl-N | b | miel | 4,9 |
| 379 | 4-Cl | H | $CH_3$<br>$CH_3O(CH_2)_2$-N | b | miel | 4,2 |
| 381 | 4-Cl | H | tetrahydro-<br>furfuryl-<br>méthylamino | b | 100 | 4,6 |
| 384 | 4-$CH_3$ | H | morpholino | a | 178 | 4,0 |
| 385 | 4-Br | H | morpholino | a | 152 | 4,2 |
| 386 | 4-Cl | H | $CH_3$-N<br>$CH_2$ = $CHCH_2$ | b | miel | 4,44 |

| Composé N° | Xn | $R_{38}$ | $NR_{31}R_{32}$ | Procédé | PF | Log P |
|---|---|---|---|---|---|---|
| 387 | - | H | $NC-CH_2CH_2-N(CH_3)$ | b | miel | 3,9 |
| 388 | - | H | $(C_2H_5)_2N$ | b | miel | 4,3 |
| 389 | 4-$CH_3O$ | H | morpholino | a | 210 | 3,3 |
| 390 | 4-Br | H | $NCH_3(C_2H_5)$ | a | miel | 4,6 |
| 391 | 2-$CH_3$ | H | morpholino | a | 158 | 4,0 |
| 392 | 2-Cl | H | morpholino | a | 186 | 4,0 |
| 393 | 4-Br | H | $CH_3O-CH_2CH_2-N(CH_3)-$ | b | miel | 4,4 |
| 394 | 4-Cl | H | $NCH_3(CH_3O)$ | b | verre | 4,6 |

| Composé N° | Xn | $R_{38}$ | $NR_1R_2$ | Procédé | PF | Log P |
|---|---|---|---|---|---|---|
| 366 | 4-Br | H | $CH_3$ C-$OCH_2CH_2$-N<br>‖          \|<br>O        $H_3C$ | b | miel | 5,0 |
| 380 | 4-Br | H | HO-$CH_2CH_2$-N<br>\|<br>$CH_3$ | b | 178 | 3,8 |
| 382 | 4-CN | H | morpholino | b | 197 | 2,8 |
| 383 | 4-Cl | H | HO-$CH_2CH_2$-N<br>\|<br>$CH_3$ | b | 160 | 3,6 |
| 395 | | H | $HOCH_2CH_2$-N<br>\|<br>$CH_3$ | b | 164 | 2,9 |

| Composé N° | Xn | $R_{38}$ | $NR_1R_2$ | Procédé | PF | Log P |
|---|---|---|---|---|---|---|
| 396 | | H | $CH_3C-OCH_2CH_2-N$<br>‖ \|<br>O $H_3C$ | b | miel | 4,3 |
| 397 | 4-Cl | H | $CH_3C-OCH_2CH_2-N$<br>‖ \|<br>O $CH_3$ | b | miel | 5,0 |
| 398 | | H | $CH_3$ $CH_3$<br>\| \|<br>$N-CH_2-CH_2-N$<br>\|<br>$CH_3$ | b | 229 | 3,70 |
| 399 | 4-CN | H | $CH_3$<br>\|<br>$C_2H_5-N$ - | b | 139 | 3,2 |
| 3100 | 4-CN | H | $CH_3$<br>\|<br>$CH_3O-CH_2CH_2-N-$ | b | miel | 3 |

Produits dont les résultats biologiques ne seront pas disponibles.

| Composé N° | Xn | $R_{38}$ | $NR_1R_2$ | Procédé | PF | Log P |
|---|---|---|---|---|---|---|
| 3101 | 4-$CH_3O$ | H | $CH_3$<br>\|<br>$CH_3OCH_2CH_2-N$ | b | miel | 3,5 |
| 3102 | 4-$CH_3O$ | H | $CH_3$<br>\|<br>$HO-CH_2-CH_2-N$ | b | miel | 2,9 |
| 3103 | 4-$CH_3O$ | H | $CH_3$<br>\|<br>$CH_3C-OCH_2CH_2-N$<br>‖<br>O | b | miel | 4,3 |

| Composé N° | Xn | $R_{38}$ | $NR_1R_2$ | Procédé | PF | Log P |
|---|---|---|---|---|---|---|
| 3104 | 3-Cl | H | morpholino | b | 100 | 4,0 |
| 3105 | 3-Cl | H | $CH_3$<br>\|<br>$C_2H_5$ - N | b | miel | 4,4 |
| 3106 | 3-Cl | H | $(C_2H_5)_2$ N - | b | miel | 5,0 |
| 3107 | 4-$CF_3$ | H | morpholino | a | 144 | 4,20 |
| 3108 | 4-$CH_3O$ | H | $(C_2H_5)_2$ N- | b | miel | 4,3 |
| 3109 | 4-$CH_3O$ | H | $CH_3$<br>\|<br>$C_2H_5-N-$ | b | miel | 3,7 |

Exemples 3110 à 3119 :

En opérant selon le mode opératoire de l'exemple 345 (procédé a) à partir des intermédiaires convenablement substitués, on obtient les dérivés de formule (IV') dans laquelle $NR_{31}R_{32}$ est un radical morpholino et $R_{37}$ a les significations indiquées au tableau suivant en même temps que les caractéristiques physiques des produits.

| Composé N° | $R_{37}$ | Procédé | PF | Log P |
|---|---|---|---|---|
| 3110 | t-Butyl | a | 163 | 3,2 |
| 3111 | $(CH_3)_2C=CH-$ | a | 163 | 2,9 |
| 3112 | thiényl-3 | a | 194 | 3,0 |
| 3113 | thiényl-2 | a | 190 | 3,20 |
| 3114 | naphtyl-1 | a | 148 | 4,5 |
| 3115 | naphtyl-2 | a | 112 | 4,5 |
| 3116 | furyl-2 | a | 174 | 2,70 |

| Composé N° | $R_{37}$ | $Z_3$ [formule (XIV)] | Procédé | PF | Log P |
|---|---|---|---|---|---|
| 3117 | thiényl-3 | $CH_3O-(CH_2)_2-N(CH_3)-$ | b | miel | 3,20 |
| 3118 | thiényl-3 | $C_2H_5-N(CH_3)-$ | b | miel | 3,40 |
| 3119 | thiényl-3 | $(C_2H_5)_2N$ | b | miel | 3,9 |

Les exemples 401 à 458 illustrent la préparation de dérivés de composés de formule (V).

Exemple 401 : 3-(3,4-diméthoxyphényl) 3-oxo 2-(diméthylaminométhylène) propionate d'éthyle (composé n° 401) :

On place dans un réacteur 20 g de 3,4-diméthoxy benzoyl-acétate d'éthyle, 80 ml de toluène et 20 g de diméthylformamide-diméthylacétal. On chauffe à reflux pendant deux heures et laisse une nuit à température ambiante. On évapore le solvant et l'excès de réactif. Le produit est purifié par passage sur silice (éluant : acétate d'éthyle). On obtient un miel jaune avec 94 % de rendement.

Exemple 402 : 1-(3,4-diméthoxyphényl) 3-diméthylamino 2-morpholinocarbonyl propènone (composé n° 402) :

Dans un réacteur, on charge 20 g de 3,4-diméthoxy-benzoyl-acétomorpholide, 28 g de diméthylformamide diméthylacétal et 20 ml de diméthylformamide. On chauffe 7 heures à 60° C et on laisse une nuit à température ambiante. On verse dans l'eau et filtre le précipité obtenu.
On obtient de cette façon un solide jaune fondant à 156,8°C avec 84 % de rendement.

Exemple 403 :

En opérant comme aux exemples 401 et 402, mais en faisant des intermédiaires convenablement substitués, on a obtenu les dérivés de formule (XV') dans laquelle $Ar_4$ a la signification précédente et dans laquelle $R_{43}$ et $R_{44}$ sont des groupes méthoxy, $R_{45}$ et $R_{49}$ sont l'atome d'hydrogène. $X_4$ et $Z_4$ ont la signification indiquée ci-dessous :

| Composé n° | $X_4$ | $Z_4$ | caractéristiques |
|---|---|---|---|
| 403 | $N(CH_3)_2$ | $N(CH_3)(C_2H_5)$ | miel |
| 404 | $N(CH_3)_2$ | $N(C_2H_5)_2$ | miel |

Exemple 405 : 5-carbéthoxy 2(4-chloro-phényl) 4-(3,4-diméthoxyphényl) pyrimidine (composé n° 405) :

Dans un réacteur on place 20 ml d'éthanol absolu et 0,3 g de sodium. Après dissolution, on ajoute 3,7 g d'hydroiodure de 4-chloro benzamidine. On chauffe à reflux une heure et ajoute 4,06 g de l'énaminone synthétisée dans l'exemple 401. On chauffe à reflux pendant 2,5 heures, évapore le solvant, lave le précipité obtenu à l'eau et sèche. On obtient 4,2 g de solide blanc fondant à 115,4°C.

Exemple 406 : 5-carboxy 2-(4-chlorophényl) 2-(3,4-diméthoxyphényl) pyrimidine (composé n° 406) :

Le produit obtenu dans l'exemple 404 (3,3 g) est dissout dans 100 ml d'éthanol et traité par 2 ml de soude 10N. On chauffe le mélange une heure à 80° C et évapore. On reprend le résidu dans l'eau et acidifie par HCl. On obtient un précipité jaune que l'on filtre et lave à l'eau.
Rendement 95 % - PF : 258,1° C.

Exemple 407 : 2-(4-chlorophényl) 4-(3,4-diméthoxyphényl) 5-morpholinocarbonyl pyrimidine (composé n° 407) :

On chauffe 1,5 g de l'acide obtenu dans l'exemple 406 avec 35 ml de chlorure de thionyle à 75° C pendant 1,5 heure. On concentre et rajoute du dichlorométhane et évapore de nouveau pour éliminer complètement le réactif en excès. On obtient de cette façon un solide orange.

Ce chlorure d'acide est dissous dans 100 ml d'éther éthylique et refroidi à 0° C. On ajoute goutte à goutte une solution de 0,9 g de morpholine et de 0,4 g de pyridine dans 40 ml d'éther. Après l'addition, on agite 1,5 heure à température ambiante. On verse dans l'eau et extrait à l'éther, sèche sur sulfate de magnésium et concentre. Après recristallisation dans l'éther, on obtient 1 g de solide fondant à 168° C (rendement 48 %).

Exemple 408 : 2-(3,4-dichlorophényl) 4-(3,4-diméthoxyphényl) 5-morpholinocarbonyl pyrimidine (composé n° 408) :

Dans un réacteur, on place 10 ml d'éthanol absolu et 0,1 g de sodium. On agite jusqu'à dissolution et ajoute 1,35 g de benzènesulfonate de 3,4-dichlorobenzamidine. On chauffe à 60° C pendant 30 minutes et ajoute l'énaminone préparée suivant l'exemple 402 et 15 ml d'éthanol absolu. On chauffe 5 heures à reflux, on filtre et lave le précipité à l'éthanol froid et à l'heptane. On sèche. On obtient un solide fondant à 181° C (83 % de rendement).

En opérant respectivement comme aux exemples 405 à 408, mais en partant d'intermédiaires convenablement substitués, on a obtenu les dérivés de formule (V) dans laquelle $R_{43}$ à $R_{44}$ sont des groupes méthoxy et $R_{45}$ et $R_{49}$ sont des atomes d'hydrogène ; $Z_4$ et $R_{47}$ ont les significations indiquées ci-dessous.

| composé N° | Procédé de l'-exemple N° | $R_{47}$ | $Z_4$ | caractéristiques | Log P |
|---|---|---|---|---|---|
| 409 | 405 | 3-nitro-phényl | $OC_2H_5$ | PF = 141°C | * |
| 410 | 406 | " | OH | PF = 226°C | * |
| 412 | 405 | phényl | $OC_2H_5$ | PF = 98°C | * |
| 413 | 406 | " | OH | PF = 215°C | * |
| 414 | 407 | " | morpho-lino | PF = 172°C | 2,3 |
| 415 | 407 | " | $N(CH_3)(C_2H_5)$ | PF = 100°C | 2,7 |
| 416 | 407 | " | $N(CH_3)(CH_3OC_2H_4)$ | huile orange | 2,5 |
| 405 | 405 | 4-chloro-phényl | $OC_2H_5$ | PF = 115°C | * |
| 407 | 407 | " | morpho-lino | PF = 168°C | 3,0 |
| 417 | 405 | 3,4-dichlo-rophényl | $OC_2H_5$ | PF = 74°C | * |
| 418 | 406 | " | OH | PF = 267°C | * |

| | | | | | |
|---|---|---|---|---|---|
| 408 | 408 | " | morpho-lino | PF = 181°C | 3,7 |
| 420 | 407 | " | $N(C_2H_5)_2$ | PF = 122°C | 3,6 |
| 421 | 407 | " | $N(CH_3)(C_2H_5)$ | PF = 146°C | 4,1 |
| 422 | 405 ou 423 | méthyl thio | $OC_2H_5$ | PF = 81°C | * |
| 423 | 407 | " | morpho-lino | PF = 151°C | * |
| 424 | 405 | 3-$CF_3$ phényl | $OC_2H_5$ | huile | * |
| 425 | 408 | " | morpho-lino | PF = 149°C | 3,2 |
| 426 | 406 | 3-$CF_3$ phényl | OH | PF = 162°C | * |
| 427 | 408 | 4-bromo-phényl | morpho-lino | PF = 169°C | 3,2 |
| 428 | 407 | 3-$CF_3$ phényl | $N(CH_3)(C_2H_5)$ | miel | 3,6 |
| 429 | 407 | " | $N(C_2H_5)_2$ | PF = 98°C | 4,1 |
| 430 | 408 | 4-bromo-phényl | $N(C_2H_5)_2$ | miel | 4,1 |
| 431 | 408 | 4-chloro-benzyl | morpho-lino | PF = 116°C | 3,2 |

Exemple 423 : 4-(3,4-diméthoxyphényl) 2-méthylthio 5-morpholinocarbonyl pyrimidine (composé n° 423) :

Dans un réacteur on dissout 6,6 g de KOH dans 60 ml d'eau, on ajoute ensuite 16,3 g de sulfate de S-méthyl isothiourée et tout de suite après 13,6 g de l'énaminone synthétisée dans l'exemple 402.

Un précipité se forme après reflux de 1,5 heure. On filtre, lave à l'eau et sèche le solide obtenu avec 58 % de rendement. PF = 151°C.

Exemple 432 : 4-(3,4-diméthoxyphényl) 2-méthylsulfonyl 5-morpholinocarbonyl pyrimidine (composé n° 432) :

Dans 10 ml de dichlorométhane, on dissout 9,5 g de la pyrimidine obtenue dans l'exemple 423 et à la température de 0°C, on ajoute par portions 11 g d'acide métachloroperoxybenzoïque. On laisse agiter 2,5 heures à température ambiante. On détruit l'excès de peracide au bisulfite de sodium et lave ensuite la solution au bicarbonate. On lave et sèche au sulfate de magnésium. Après évaporation du solvant, on obtient 9,9 g de solide blanc fondant à 77°C.

Exemple 433 : 4-(3,4-diméthoxyphényl) 2-(4-méthyl-phénoxy) 5-morpholinocarbonyl pyrimidine (composé n° 433) :

Dans un réacteur, on place 10 ml d'acétonitrile, 0,5 g de carbonate de potassium, 2,7 g de paracrésol et 1 g de la pyrimidine obtenue dans l'exemple 432, ainsi que trois gouttes de TDA-1[tris (3,6-dioxa-heptyl) amine] comme catalyseur.

On chauffe une heure à 65°C et évapore le solvant. On reprend l'eau et extrait à l'éther, sèche sur $MgSO_4$ et évapore. On obtient un solide blanc avec 75 % de rendement fondant à 152°C.

En opérant respectivement comme aux exemples 423, 432 et 433, mais en partant d'intermédiaires convenablement substitués, on obtient les dérivés de formule (V) dans laquelle $R_{43}$ à $R_{44}$ sont des groupes méthoxy et $R_{45}$ et $R_{49}$ sont des atomes d'hydrogène ; $Z_4$ et $R_{47}$ ont les significations indiquées dans le tableau ci-dessous.

| Composé N° | Procédé de l'exemple N° | $R_{47}$ | $Z_4$ | PF | Log P |
|---|---|---|---|---|---|
| 434 | 423 | benzylthio | morpholino | 53 | 2,9 |
| 435 | 423 | 4-chloro benzylthio | morpholino | 134 | 3,6 |
| 436 | 423 | 4-chloro phénylthio | morpholino | 147 | 3,7 |
| 437 | 422+432+ 433 | 4-chloro phénoxy | " | 174 | 3,7 |
| 438 | " | 4-bromo phénoxy | " | 170 | 3,9 |
| 439 | " | 4-fluoro phénoxy | " | 189 | 3,1 |
| 440 | " | 2-méthyl phénoxy | " | 136 | 3,6 |
| 441 | " | 4-Cl-3-méthyl-phénoxy | " | 176 | 4,3 |
| 433 | " | 4-$CH_3$ phénoxy | " | 152 | 3,6 |
| 442 | " | 4-$CF_3$ phénoxy | " | 54 | 3,9 |

| | | | | | |
|---|---|---|---|---|---|
| 444 | " | 2-chloro phénylthio | " | 179 | 3,1 |
| 445 | " | 3-chloro phénylthio | " | 58 | 3,6 |
| 446 | " | 2-bromo phénylthio | " | 169 | 3,8 |
| 447 | " | 4-bromo phénylthio | " | 158 | 3,8 |
| 448 | " | 3-fluoro phénylthio | " | 67 | 3,1 |

| Composé N° | Procédé de l'exemple N° | R47 | Z4 | PF | Log P |
|---|---|---|---|---|---|
| 449 | " | 4-fluoro phénylthio | " | 116 | 3,1 |
| 450 | " | 3,4-dichloro phénylthio | " | 135 | 4,4 |
| 451 | " | 2,6-dichloro phénylthio | " | 187 | 3,2 |
| 453 | 422 + 432 + 432 | anilino | morpholino | 210 | 3,2 |
| 454 | " | 4-chloro anilino | morpholino | 184 | 3,9 |
| 455 | " | 3,4-dichloro anilino | " | 152 | 4,6 |
| 456 | " | 2-chloro 4-bromo anilino | " | 122 | 4,8 |
| 457 | " | 3-chloro 4-fluoro anilino | " | 178 | 4,1 |
| 458 | " | pipéridino | " | 150,6 | 2,6 |

Exemple 501 : Préparation du 2-acétyl 3-(3,4-diméthoxyphényl) 5-phényl 5-oxo valérate d'éthyle (composé n° 501).

On dissout 26,8 g (0,1 mole) de 3,4-diméthoxy chalcone (m,p-$(CH_3O)_2$ $C_6$ $H_3$-CH=CH-CO-$C_6H_5$) et 13 g (0,1 mole) d'acétoacétate d'éthyle dans 300 ml d'éthanol. On ajoute 1 g de baryte activée et agite 20 heures à 20°C. Le précipité d'oxovalérate est filtré, lavé à l'éthanol et séché. On obtient 27 g (rendement : 68 %) du

composé n° 501 fondant à 135°C.

Exemple 502 : Préparation du 4-(3,4-diméthoxyphényl) 2-méthyl 6-phényl nicotinate d'éthyle (composé n° 502).

On chauffe 5 heures à l'ébullition à reflux un mélange de 20 g (0,05 mole) du composé n° 501, 7,7 g (0,1 mole) d'acétate d'ammonium et 80 ml d'acide acétique. On évapore, redissout dans 200 ml de chlorure de méthylène, lave avec une solution aqueuse de bicarbonate de sodium, puis à l'eau. On concentre, sèche, purifie par chromatographie sur silice.

On obtient 8,5 g d'un solide beige fondant à 82°C (rendement : 45 % ; composé n° 502).

Exemple 503 : Préparation de l'acide 4-(3,4-diméthoxyphényl) 2-méthyl 6-phényl nicotinique (composé n° 503).

On chauffe 30 heures à l'ébullition à reflux un mélange de 10 ml de soude aqueuse 10N avec une solution de 8,4 g (0,022 mole) de composé n° 502 dans 100 ml d'éthanol. On concentre, dissout dans l'eau, acidifie, extrait à l'acétate d'éthyle, sèche, concentre. On obtient 4,7 g (rendement : 61 %) du composé n° 503 fondant à 208°C.

Exemple 504 : Préparation de chlorure de 4-(3,4-diméthoxyphényl) 2-méthyl 6-phényl nicotinoyle (composé n° 504).

On chauffe pendant 1 heure à l'ébullition à reflux un mélange de 3,5 g (0,01 mole) du composé n° 503 avec 20 ml de chlorure de thionyle. On concentre. Le produit n° 504, rouge orangé, cristallise. Rendement : 100 %. PF = 140°C.

Exemple 505 : Préparation de N-[4-(3,4-diméthoxyphényl) 2-méthyl 6-phényl nicotinoyl] morpholine (composé n° 505).

Il s'agit du produit de formule (VI) avec $R_{59}$ = méthyle, $Z_5$ = morpholino, $R_{53}$ = $R_{54}$ = méthoxy, $R_{55}$ = $R_{56}$ = H, $R_{57}$ = $C_6H_5$.

A une solution de 1,2 g (0,033 mole) du composé n° 503 dans 50 ml de chloroforme, on ajoute 2 ml de morpholine. On laisse réagir 3 heures à 20°C, lave à l'eau, concentre on cristallise avec de l'éther, filtre, sèche. On obtient 0,9 g du composé n° 505 (poudre blanche ; rendement : 65 %) fondant à 176°C. log P = 3,8.

Exemples 506 et 507 :

Les composés 506 et 507 sont préparés comme à l'exemple 505 en remplaçant la morpholine respectivement par $CH_3$-NH-$C_2H_5$ et par $CH_3$-NH-$CH_2$-$CH_2$-O-$CH_3$. Le composé n° 506 fond à 76°C (log P = 4,2). Le composé n° 507 est un miel (log P = 4,0).

Exemple 601 : Préparation de 2-(3,4-diméthoxyphényl) 5-phényl 3-furane carboxylate d'éthyle :

On chauffe 12 heures à reflux un mélange de 10 g (0,027 mole) de 2-(3,4-diméthoxybenzoyl 4-phényl) 4-oxo butyrate d'éthyle 2,75 g (0,027 mole) d'anhydride acétique, 2 gouttes d'acide sulfurique concentré et 50 ml d'acide acétique. On verse le milieu réactionnel dans 500 ml d'eau. On extrait le produit avec 300 ml d'acétate d'éthyle, lave la solution organique à l'eau bicarbonatée puis à l'eau pure. On sèche la solution sur sulfate de magnésium anhydre, concentre sous pression réduite ; on obtient une gomme qui cristallise en la triturant dans 50 ml d'heptane. Après filtration et séchage, on obtient 6,5 g (rendement : 68 %) du produit attendu sous forme d'une poudre beige fondant 68°C (composé n° 601).

Exemple 602 : Acide 2-(3,4-diméthoxyphényl) 6-phényl 3-furoïque :

On chauffe une heure à reflux un mélange contenant 5 g (0,0142 mole) du composé de l'exemple 601, 6 ml de soude 10 N et 100 ml d'alcool éthylique. On concentre le milieu sous pression réduite, dissout le résidu dans 100 ml d'eau, et acidifie jusqu'à pH = 4 la solution aqueuse par ajout d'acide chlorhydrique concentré : un solide blanc précipite. On filtre le précipité, lave le gâteau à l'eau, sèche le produit sous pression réduite en présence de $P_2O_5$ comme déshydratant. On obtient 4,3 g (rendement = 93 %) du produit attendu sous forme d'une poudre blanche fondant à 177°C (composé n° 602).

Exemple 603 : Chlorure de 2-(3,4-diméthoxyphényl) 6-phényl 3-furoyle :

On chauffe 45 mn à reflux un mélange de 2,8 g du produit de l'exemple 2 et 20 ml de chlorure de thionyle. On concentre le milieu sous pression réduite : un solide vert cristallise. On obtient 3 g (rendement quantitatif) du produit fondant à 114°C (composé n° 603).

Exemple 604 : 2-(3,4-diméthoxyphényl) 5-phényl N-éthyl N-méthyl 3-furane carboxamide :

On dissout 1,51 g (4,4 mmole) du chlorure de l'exemple 603 dans 20 ml de THF. On ajoute sous agitation 0,59 g (10 mmole) de méthyl éthyl amine : le chlorhydrate de l'amine précipite immédiatement. On agite 1/4 d'heure à température ordinaire, filtre le chlorhydrate, concentre le filtrat. On reprend le miel résiduel avec 100 ml de chloroforme. On lave la solution chloroformique à l'eau, concentre la solution, fait cristalliser le produit dans un mélange d'éther diisopropylique et d'heptane. Après filtration et séchage, on récupère 0,6 g (rendement = 37 %) du composé attendu sous forme d'une poudre beige à 74°C (composé n° 604).

Exemple 605 : N-[2-(3,4-diméthoxyphényl) 6-(4-fluorophényl) 3-furoyl] morpholine :

On chauffe 30 heures à reflux un mélange contenant 1 g (2,3 mmole) de N-[2-(3,4-diméthoxybenzoyl) 4-(4-fluorophényl) 4-oxo butyroyl] morpholine, 20 ml d'éthanol et 1 ml d'acide chlorhydrique concentré. On verse le milieu réactionnel dans 200 ml d'eau, on extrait le produit avec 150 ml de chloroforme. On lave la solution chloroformique à l'eau, concentre la solution chloroformique, fait cristalliser le produit en le triturant dans 20 ml d'éther. Après filtration et séchage, on obtient 0,8 g (rendement = 83 %) du produit attendu sous forme d'une poudre blanche fondant à 168°C (composé n° 605).

Exemple 606 : Préparation de 2-(-3,4-diméthoxyphényl) 5-(4-fluorophényl) 3-(morpholino carbonyl) pyrrole :

On chauffe 12 heures à reflux un mélange de :

- 5g (11,6 mmole) de N-[2-(3,4-diméthoxy-benzoyl) 4-(4-fluorophényl) 4-oxo butyryl)] morpholine,
- 1,8 g (23,2 mmole) d'acétate d'ammonium,
- 30 ml d'acide acétique.

On verse le milieu réactionnel dans 30 ml d'eau, extrait le produit avec 200 ml d'acétate d'éthyle, lave la solution organique à l'eau bicarbonatée, puis à l'eau pure, sèche la solution sur sulftate de magnésium anhydre. On concentre la solution, recristallise le produit dans 300 ml de toluène. On obtient 1,4 g (rendement = 28 %) du produit attendu sous forme d'une poudre jaune fondant à 190°C (composé 606).

Exemple 607 : Préparation de 5-(4-fluorophényl 2-(3,4-diméthoxyphényl) 1-méthyl 3-(morpholinocarbonyl) pyrrole :

On dissout dans 30 ml d'acide acétique :

- 0,72 g (0,023 mole) de monométhylamine,
- 5 g (0,00115 mole) de N-[2-(3,4-diméthoxybenzoyl) 4-(4-phényl) 4-oxo butyroyl] morpholine.

On porte le milieu à reflux pendant 10 heures. Le milieu réactionnel est ensuite versé dans 300 ml d'eau. Le produit est extrait avec 200 ml d'acétate d'éthyle. La solution est lavée au bicarbonate puis à l'eau. Elle est ensuite séchée sur sulfate de magnésium puis concentrée. On obtient un solide marron que l'on recristallise dans l'éther.

On récupère 1,3 g de produit attendu (rendement = 26 %). PF = 148°C (composé 607).

Exemple 608 : 2-(4-chloro phényl) 3-(diméthoxy-3,4 phényl) 4-(morpholinocarbonyl) pyrazole :

On introduit dans un réacteur 2,5 g de 1-(3,4-diméthoxy phényl) 3-(diméthylamino) 2-morpholinocarbonyl propénone (exemple 402), 2,1 g de chlorohydrate de 4-chlorophényl hydrazine, 1,2 g de triéthylamine et 100 ml d'éthanol. On chauffe à reflux pendant 8 heures 30.

On évapore le solvant, on verse dans l'eau, on extrait à l'acétate d'éthyle, on sèche sur sulfate de sodium et on concentre. Après filtration sur silice (éluant acétate d'éthyle/heptane : 90/10), on obtient 2,1 g de solide jaune fondant à 125°C (rendement : 60 %) (composé 608).

Exemple 609 : 4-carbéthoxy-2-(4-chlorophényl)-3-(3,4-diméthoxy phényl) pyrazole :

On introduit dans un réacteur 2,5 g de 3-(3,4-diméthoxy phényl)-3-oxo-2-(diméthylaminométhylène) propionate d'éthyle (exemple 401), 1,49 g de chlorhydrate de 4-chloro phényl hydrazine, 0,82 g de triéthylamine et 100 ml d'éthanol. On chauffe à reflux pendant 8 heures. On évapore le solvant, on verse dans l'eau et on reprend à l'acétate d'éthyle. Après séchage sur sulfate de sodium et évaporation du solvant, on obtient 3 g d'un solide marron (rendement : 95 %) qu'on utilise sans purification (composé n° 609).

Exemple 610 : 4-carboxy-2-(4-chlorophényl) 3-(3,4-diméthoxy phényl) pyrazole :

L'ester obtenu dans l'exemple 611 (3 g) est introduit dans un réacteur contenant 150 ml d'éthanol et traité par 1,01 g de potasse pilée à 86 %. On chauffe à reflux pendant 4 heures. On évapore le solvant, on verse dans l'eau et on acidifie avec une solution d'acide chlorhydrique 1N jusqu'à pH 1. Le précipité obtenu est filtré, lavé à l'eau puis à l'heptane, on obtient 2,6 g de poudre marron (93,5 %) qu'on utilise sans purification (composé n° 610).

Exemple 611 : 4-(N-méthyl N-éthyl aminocarbonyl)-2-(4-chlorophényl)-3-(3,4-diméthoxy phényl) pyrazole :

On chauffe 2,6 g d'acide préparé dans l'exemple 610 avec 50 ml de chlorure de thionyle à 75 % pendant 8 heures. On reprend avec du dichlorométhane et on concentre. L'huile obtenue (1,4 g) est dissoute dans 30 ml de chlorure de méthylène et refroidie vers 3°C. On ajoute une solution de 0,54 g de N-méthyl N-éthylamine et 0,3 g de pyridine, 30 ml de chlorure de méthylène en maintenant la température inférieure à 5°C. On agite pendant 6 heures 20 minutes à température ambiante. On verse dans l'eau et on extrait au chlorure de méthylène. On sèche sur sulfate de sodium et on concentre. On obtient 1,2 g de précipité orange fondant à 110°C (rendement = 82,2 %) (composé n° 611).

Exemple 612 : 4-(N,N-diéthyl aminocarbonyl)-2-(4-chloro phényl) 3-(3,4-diméthoxy phényl) pyrazole :

On utilise le même opératoire que dans l'exemple 611 en utilisant la diéthylamine. Rendement : 80 % en un miel orange (composé n° 612).

Exemple 613 : 1-benzyl 3-(3,4-diméthoxy phényl)-4-(morpholinocarbonyl) pyrazole :

On utilise le même mode opératoire que dans l'exemple 608 en utilisant le chlorhydrate de benzylhydrazine. On obtient 22,8 % d'une huile jaune à côté de l'isomère normalement attendu (45,7 %) (composé n° 613).

Exemple 614 : 1-(4-bromo benzyl)-3-(3,4-diméthoxy phényl)-4-(morpholinocarbonyl) pyrazole :

On introduit dans un réacteur, 0,9 g de 3-(3,4-diméthoxy phényl)-4(morpholinocarbonyl) pyrazole (obtenu en utilisant le même mode opératoire que dans l'exemple 608 avec l'hydrate d'hydrazine), 0,7 g de bromo-4 benzyle, 0,4 g de carbonate de potassium et 70 ml de DMF (diméthylformamide). On chauffe à 75° C pendant 5 heures 30. On verse dans l'eau, on extrait à l'acétate d'éthyle, on sèche et on concentre. Après filtration sur silice, on isole 0,6 g (rendement : 43,5 %) d'un précipité fondant à 122° C (composé n° 614).

En opérant respectivement comme aux exemples 601 à 614, mais en partant d'intermédiaires convenablement substitués, on a obtenu les dérivés de formule (VII) dans laquelle $R_{610}$ est -CH=, $R_{63}$ et $R_{64}$ sont des groupes méthoxy, $R_{65}$ est un atome d'hydrogène, $NR_{61}R_{62}$, $K_{66}$ et $K_{67}$ ont les significations indiquées ci-dessous dans le tableau.

| Composé N° | Procédé décrit dans l'exemple | $K_{66}$ | $K_{67}$ | $NR_{61}R_{62}$ | PF | log P |
|---|---|---|---|---|---|---|
| 615 | 604 | O | -C(4-chlorophényl) = | $N(CH_3)C_2H_5$ | 102 | 4,7 |
| 616 | 605 | O | -C(4-chlorophényl) = | morpholino | 139 | 4,3 |
| 617 | 605 | O | -C(phényl) = | morpholino | 177 | 3,6 |
| 604 | 604 | O | -C(phényl) = | $N(CH_3)C_2H_5$ | 74 | 4,0 |
| 605 | 605 | O | -C(4-fluorophényl) = | morpholino | 168 | 3,7 |
| 606 | 606 | -NH- | -C(4-fluorophényl) = | morpholino | 190 | 3,1 |
| 607 | 607 | $-N(CH_3)-$ | -C(4-fluorophényl) = | morpholino | 148 | 3,9 |
| 618 | 606 | -NH- | -C(4-chlorophényl) = | morpholino | 225 | 3,7 |
| 619 | 607 | $-N(CH_3)-$ | -C(4-chlorophényl) = | morpholino | 155 | 4,5 |
| 620 | 606 | -N(H)- | -C(phényl) = | morpholino | 160 | 3,0 |
| 621 | 607 | $-N(CH_3)-$ | -C(phényl) = | morpholino | 173 | 3,8 |
| 622 | 606 | -N(H)- | -C(4-tbutylphényl) = | morpholino | 156 | 4,9 |
| 623 | 608 | $-N(C_6H_5)-$ | -N = | morpholino | 110 | 2,5 |
| 624 | 611 | $-N(C_6H_5)-$ | -N = | $N(CH_3)_2C2H_5$ | miel | 2,9 |
| 625 | 612 | $-N(C_6H_5)-$ | -N = | $N(C_2H_5)_2$ | 111 | 3,4 |
| 608 | 608 | -N(4-chlorophényl) | -N = | morpholino | 125 | 3,2 |
| 611 | 611 | -N(4-chlorophényl) | -N = | $N(CH_3)C_2H_5$ | 110 | 3,6 |
| 612 | 612 | -N(4-chlorophényl) | -N = | $N(C_2H_5)_2$ | miel | 4,0 |
| 626 | 608 | -N(benzyl) | -N = | morpholino | miel | 2,5 |
| 627 | 611 | -N(benzyl) | -N = | $N(C_2H_5)_2$ | 105 | 2,9 |
| 628 | 612 | -N(Benzyl) | -N = | $N(C_2H_5)_2$ | miel | 3,3 |
| 619 | 613 | -N = | -N(benzyl)- | morpholino | miel | 2,5 |
| 614 | 614 | -N = | -N(4-bromo benzyl)- | morpholino | 122 | 3,4 |

Exemples biologiques :

Les exemples ci-après montrent les bonnes propriétés fongicides des composés selon l'invention.

Exemple B1: Traitement du mildiou de la tomate (Phytophthora infestans) :

Des plants de tomate (Lycopersicon esculentum), de variété Marmande, sont cultivés dans des godets. Lorsque ces plants sont âgés d'un mois (stade 5 à 6 feuilles, hauteur 12 à 15 cm), ils sont traités par pulvérisation au moyen d'une suspension ou solution aqueuse de la matière à tester, à la concentration désirée et contenant un tensioactif qui est un condensat de monooléate de sorbitan et de 20 molécules d'oxyde d'éthylène. Ce tensioactif est à une concentration égale à la moitié de la concentration de la matière active. Chaque plant de tomate reçoit environ 5 ml de la solution ou dispersion. Pour chaque concentration de matière active à tester, le traitement est effectué sur deux plants. Des plants utilisés comme témoins sont traités par une solution ne contenant pas de matière active, mais contenant le même tensioactif à concentration identique.

Après séchage pendant 24 heures, on contamine chaque plant par pulvérisation au moyen d'une suspension aqueuse de spores de Phytophthora infestans, responsable du mildiou de la tomate, à raison d'environ 5 ml/plant (soit environ 50.000 spores par plant).

Après cette contamination, les plants de tomate sont mis en incubation pendant un jour à 15° C environ en atmosphère saturée d'humidité, puis pendant cinq jours à 17° C environ sous 70 % à 90 % d'humidité relative.

Sept jours après la contamination, on compare les résultats obtenus dans le cas des plants traités par la matière active à tester à ceux obtenus dans le cas des plants utilisés comme témoins.

Dans ces conditions, on a observé que, à la dose de 1 000 ppm(1 g/l) les composés 104, 110, 111, 112, 113, 117, 118, 129, 133, 148, 149, 150, 164, 166, 167, 169, 201 à 203, 205, 206, 208, 209, 211, 212, 214, 222, 223, 235, 236, 237, 238, 301, 345, 347, 349, 350, 353, 356, 359, 364, 369, 370, 379, 381, 382, 384 à 386, 388, 391 et 393, 396, 3113, 3118, 3119, 407, 414 à 416, 433, 434, 435, 440, 445, 458, 505 à 507, 607, 619, 621, 608, 6081 entraînaient au moins 80 % d'inhibition du développement du champignon.

Exemple B2 : Traitement du mildiou de la vigne (Plasmopara viticola) :

Des boutures de vigne (Vitis vinifera), de variété Chardonnay sont cultivées dans des godets. Lorsque ces plants sont âgés de 2 mois (stade 8 à 10 feuilles, hauteur 20 à 30 cm), ils sont traités de la même manière qu'à l'exemple B1.

Après séchage pendant 24 heures, on contamine chaque plant par pulvérisation au moyen d'une suspension aqueuse de spores de Plasmopara viticola, responsable du mildiou de la vigne, à raison d'environ 5 ml/plant (soit environ 100.000 spores par plant).

Après cette contamination, les plants de vigne sont mis en incubation pendant deux jours à 18° C environ en atmosphère saturée d'humidité, puis pendant cinq jours à 20-22° C environ sous 90-100 % d'humidité relative.

Huit jours après la contamination, on compare les résultats obtenus dans le cas des plants traités par la matière active à tester à ceux obtenus dans le cas des plants utilisés comme témoin.

Dans ces conditions, on a observé que, à la dose de 330 ppm (0,33 g/l) les composés 101, 104, 105, 107, 110 à 118, 121 à 125, 127 à 134, 136, 137, 141, 148 à 150, 152 à 160, 162 à 164, 166 à 170, 201 à 203, 204, 205, 206, 208, 209, 210, 211, 212, 213, 214, 215, 217, 222, 223, 229, 234 à 239, 301, 345, 347, 349 à 351, 353 à 356, 358, 359, 361 à 370, 373 à 380, 382 à 391, 393 à 394, 396, 397, 399, 3100, 3110, 3112 à 3119, 407, 414 à 416, 420, 421, 433 à 442, 445, 450, 458, 505 à 507, 616, 607, 608, 612, 613, 619, 621, 623, 626 entraînaient au moins 80 % d'inhibition du développement du champignon.

Exemple B3 : Action sur les champignons phytopathogènes du sol :

On fait agir des composés selon l'invention sur le champignon suivant :

Phytophthora citrophtora, parasite des racines d'agrumes, se traduisant par des micoses et déformations des parties aériennes.

Pour chaque essai, on opère de la manière suivante : un milieu nutritif à base d'extrait de pomme de terre solidifié par de la gélose, est introduit en surfusion dans une série de boîtes de Pétri (20 ml par boîte) après stérilisation à l'autoclave à 120° C.

Au cours du remplissage des boîtes, on injecte, dans le milieu en surfusion, une solution acétonique de la matière active, pour obtenir la concentration finale désirée.

On prend comme témoin des boîtes de Pétri analogues aux précédentes, dans lesquelles on coule des quantités similaires d'un milieu nutritif ne contenant pas de matière active.

Après solidification, chaque boîte est ensemencée par dépôt d'un fragment de mycelium provenant d'une culture précédente du même champignon.

Les boîtes sont conservées pendant 5 jours à 22° C et on compare alors la croissance du champignon dans les boites contenant la matière active à tester, à celle du même champignon dans la boîte utilisée comme témoin.

Dans ces conditions, à la dose de 30 ppm (parties par million), les composés suivants ont provoqué 80 % au moins d'inhibition du développement du champignon : 101, 102, 104, 105, 107, 110 à 118, 121 à 129, 131 à 137, 141, 149 à 150, 153 à 158, 162 à 164, 166, 167, 169, 201 à 203, 204, 205, 206, 208, 209, 210, 211, 212, 213, 214, 215, 216, 217, 222, 223, 224, 228, 229, 230, 231, 234 à 239, 301, 345, 347, 349, 350, 353, 354, 355, 356, 358 à 360,

363 à 370, 373, 376 à 386, 388 à 397, 3110 à 3119, 407, 414 à 416, 420, 425 à 430, 433 à 442, 445, 447, 449, 450, 458, 505 à 507, 607, 608, 619 et 621.

Utilisation des composés de l'invention :

Les composés selon l'invention peuvent être utilisés comme matières actives fongicides, en particulier pour la lutte contre les maladies fongiques des plantes notamment celles dûes aux champignons pathogènes notamment ceux de la famille des oomycètes du type Phytophthora sp par exemple Phytophthora infestans (mildiou de la pomme de terre ou de la tomate), Phytophthora Citrophthora, Phytophthora capsici, Phytophthora cactorum, Phytophthora palmivora, Phytophthora cinnamoni, Phytophthora megasperma, Phytophthora parasitica, Peronospora sp (notamment mildiou du tabac), Plasmopara sp notamment Plasmopara viticola (mildiou de la vigne) et Plasmopara halstedei (mildiou du tournesol), Pseudoperonospora sp (notamment mildiou des cucurbitacées et du houblon), Bremia lactucae (Bremia de la laitue), ainsi que les champignons du sol.

Ils s'appliquent avantageusement à des doses de 0,01 à 5 kg/ha, et plus spécifiquement de 0,02 à 1,5 kg/ha environ.

Pour leur emploi pratique, les composés selon l'invention sont rarement utilisés seuls. Le plus souvent ils font partie de compositions. Ces compositions, utilisables pour la protection des végétaux contre les maladies fongiques, ou dans les compositions régulatrices de la croissance des plantes, contiennent comme matière active au moins un composé selon l'invention tel que décrit précédemment en association avec les supports solides ou liquides inertes, acceptables en agriculture et/ou les agents tensio-actifs compatibles avec la matière active, également acceptables en agriculture. En particulier sont utilisables les supports inertes et usuels et les agents tensio-actifs usuels.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés) ou gaz.

Les compositions selon l'invention (destinées à être commercialisées à l'état concentré soit à être appliquées directement à l'état dilué) comprennent généralement de 0,0001 à 95 % de matière active, de préférence de 0,0005 à 90 % ainsi que 0 à 20 % d'agent tensio actif, de préférence, 0,5 à 15 %. Les compositions concentrées, destinées à être commercialisées, comprennent généralement 0,1 à 95 % de matière active, de préférence 0,5 à 90 %.

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Les compositions utilisées dans l'invention peuvent être sous des formes assez diverses, fluides, liquides, ou solides.

Comme formes de compositions fluides, ou liquides, on peut citer notamment les concentrés émulsionnables, les émulsions, les suspensions aqueuses concentrées, les pâtes, les solutions, en particulier les concentrés solubles dans l'eau, les solutions concentrées dans un milieu organique, (solutions ULV = ultra low volume) et les aérosols.

Les concentrés émulsionnables ou solubles comprennent le plus souvent 10 à 80 % de matière active, les émulsions ou solutions prêtes à l'application contenant, quant à elles, 0,001 à 20 % de matière active. En plus de la matière active et du solvant, les concentrés émulsionnables peuvent contenir, quand c'est nécessaire, un co-solvant approprié et de 2 à 20 % d'additifs appropriés, comme des stabilisants, des agents de pénétration, des inhibiteurs de corrosion, des colorants, des adhésifs.

A partir de ces concentrés, on peut obtenir par dilution avec de l'eau des émulsions de toute concentration désirée, qui conviennent particulièrement à l'application sur les cultures.

A titre d'exemples, voici la composition de quelques concentrés émulsionnables :

Exemples CE 1

| | |
|---|---|
| - matière active (composé n° 102 ou 202 ou 301)..... | 250 g/l |
| - huile végétale epoxydée..... | 25 g/l |
| - mélange de sulfonate d'alcoylaryle et d'éther de polyglycol et d'alcools gras..... | 100 g/l |
| - diméthylformamide..... | 50 g/l |
| - xylène..... | 575 g/l |

Exemple CE 2

| | |
|---|---|
| - matière active (composé n° 105 ou 206 ou 345)..... | 400 g/l |
| - dodécylbenzene sulfonate alcalin..... | 24 g/l |
| - nonylphénol oxyéthylé à 10 molécules d'oxyde d'éthylène ..... | 16 g/l |
| - cyclohexanone..... | 200 g/l |
| - solvant aromatique..... | 1 l |

A partir de ces concentrés, on peut obtenir par dilution avec de l'eau des émulsions de toute concentration désirée, qui conviennent particulièrement à l'application sur les feuilles.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti- mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici la composition de plusieurs suspensions aqueuses concentrées selon l'invention :

Exemple SAC 1 :

| | |
|---|---|
| - matière active (composé n° 106 ou 206 ou 350)..... | 100 g/l |
| - agent mouillant (alkylphénol polyéthoxylé)..... | 5 g/l |
| - agent dispersant (Naphtalène sulfonate de Na)..... | 10 g/l |
| - antigel (Propylèneglycol)..... | 100 g/l |
| - épaississant (Polysaccharide)..... | 3 g/l |
| - biocide (Formaldéhyde)..... | 1 g/l |
| - eau.....Q.S.P. | 1 litre |

Exemple SAC 2 :

On prépare une suspension aqueuse comprenant :

| | |
|---|---|
| - matière active (composé n° 108 ou 208 ou 301)..... | 250 g/l |
| - agent mouillant (alcool synthétique en $C_{13}$ polyéthoxylé)..... | 10 g/l |
| - agent dispersant (lignosulfonate de sodium)..... | 15 g/l |
| - antigel (urée)..... | 50 g/l |
| - épaississant (Polysaccharide)..... | 2,5 g/l |
| - biocide (Formaldéhyde)..... | 1 g/l |
| - eau.....Q.S.P. | 1 litre |

Exemple SAC 3 :

On prépare une suspension aqueuse comprenant :

| | |
|---|---|
| - matière active (composé n° 109 ou 209 ou 345)..... | 500 g/l |
| - agent mouillant (alcool synthétique en $C_{13}$ polyéthoxylé)..... | 10 g/l |
| - agent dispersant (phosphate de polyaryl phénol éthoxylé salifié)..... | 50 g/l |
| - antigel (propylèneglycol)..... | 100 g/l |
| - épaississant (polysaccharide)..... | 1,6 g/l |
| - biocide (4-méthylhydroxy benzoate de sodium) ..... | 3,3 g/l |
| - eau.....Q.S.P. | 1 litre |

Comme formes de compositions solides, on peut citer les poudres pour poudrage (à teneur en matières actives pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 0,5 et 80 % pour ces derniers cas).

Les poudres mouillables (ou poudres à pulvériser) sont habituellement préparées de manière qu'elles contiennent 10 à 95 % de matière active, et elles contiennent habituellement, en plus du support solide, de 0 à 5 % d'un agent mouillant, de 3 à 10 % d'un agent dispersant, et, quand c'est nécessaire, de 0 à 1o % d'un ou plusieurs stabilisants et/ou autres additifs, comme des agents de pénétration, des adhésifs, ou des agents antimottants, colorants, etc...

A titre d'exemple, voici la composition de plusieurs poudres mouillables.

Exemple PM1 :

| | |
|---|---|
| - matière active (composé n° 111 ou 211 ou 350)..... | 10 % |
| - alcool synthétique oxo de type ramifié, en $C_{13}$, éthoxylé par 8 à 10 oxyde d'éthylène (agent mouillant)..... | 0,75 % |
| - lignosulfonate de calcium neutre (agent dispersant)..... | 12 % |
| - carbonate de calcium (charge inerte) Q.S.P..... | 100 % |

Exemple PM 2 :

| | |
|---|---|
| - matière active (composé n° 102 ou 202 ou 301) selon l'invention)..... | 50 % |
| - alcool gras éthoxylé (agent mouillant)..... | 2,5 % |
| - styrylphenol éthoxylé (agent dispersant)..... | 5 % |
| - craie (support inerte)..... | 42,5 % |

Exemple PM 3 : contenant les mêmes ingrédients que dans l'exemple précédent, dans les proportions ci-après :

| | |
|---|---|
| - matière active (composé n° 105 ou 206 ou 345)..... | 75 % |
| - agent mouillant..... | 1,5 % |
| - agent dispersant..... | 8 % |
| - carbonate de calcium (charge inerte).....Q.S.P. | 100 % |

Exemple PM4 :

| | |
|---|---|
| - matière active (composé n° 106 ou 206 ou 350)..... | 90 % |
| - alcool gras éthoxylé (agent mouillant)..... | 4 % |
| - styrylphenol éthoxylé (agent dispersant)..... | 6 % |

Pour obtenir ces poudres à pulvériser ou poudres mouillables, on mélange intimement la matière active dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles des végétaux.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Les granulés pour poudrage ont des dimensions comprises entre 0,1 et 2 mm et peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvants.

Voici deux exemples de composition de granulé :

Exemple G 1 et G 2 :

| | | |
|---|---|---|
| - matière active (composé n° 108 ou 208 ou 301) ..... | 50 g | 200 g |
| - propylène glycol..... | 50 g | 50 g |
| - ether de cétyle et de polyglycol..... | 2,5 g | 2,5 g |
| - polyéthylène glycol..... | 35 g | 35 g |
| - kaolin (granulométrie : 0,3 à 0,8 mm)..... | 910 g | 760 g |

Les composés selon l'invention peuvent être avantageusement formulés sous la forme de granulés dispersibles dans l'eau également compris dans le cadre de l'invention.

Ces granulés dispersibles, de densité apparente généralement comprise entre environ 0,3 et 0,6 ont une dimension de particules généralement comprise entre environ 150 et 2000 microns, et de préférence entre 300 et 1500 microns.

La teneur en matière active de ces granulés est généralement comprise entre environ 1 % et 90 % et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge est hydrosoluble ou non. Dans le cas d'une charge hydrosoluble, elle peut être minérale et de préférence organique. On a obtenu d'excellents résultats avec l'urée. Dans le cas d'une charge insoluble, celle-ci est de préférence minérale, comme par exemple le kaolin ou la bentonite. Elle est alors accompagnée d'agents tensio-actifs (à raison de 2 à 20 % en poids du granule), adjuvants tensio-actifs dont plus de la moitié est constituée par au moins un agent dispersant, essentiellement anionique, tel qu'un poly(naphtalène sufonate alcalin ou alcalino terreux) ou un lignosulfonate alcalin ou alcalino-terreux, le reste étant constitué par des mouillants non ioniques ou anioniques tel qu'un alcoylnaphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassage suivi d'un tamisage à la dimension de particule choisie dans les limites mentionnées ci-dessus.

De préférence, le granulé selon l'invention, est obtenu par extrusion. En opérant comme indiqué dans les exemples ci-après, on a préparé les compositions suivantes de granulés dispersibles.

Exemple GD1 :

Dans un mélangeur, on mélange 90 % en poids de matière active (composé n° 111 ou 211) et 10 % d'urée en perles. Le mélange est ensuite broyé dans un broyeur à broches. On obtient une poudre humide qui est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple GD2 :

Dans un mélangeur, on mélange les constituants suivants :

| | |
|---|---|
| - matière active (composé n° 105 ou 202 ou 345)..... | 75 % |
| - agent mouillant (alkylnaphtalène sulfonatedesodium..... | 2 % |
| - agent dispersant (polynaphtalène sulfonate desodium)..... | 8 % |
| - charge inerte insoluble dans l'eau (kaolin)..... | 15 % |

Exemple GD3 :

| | |
|---|---|
| - matière active (composé n° 105 ou 206 ou 350)..... | 20 % |
| - alkylnaphtalène sulfonate de sodium..... | 2 % |
| - méthylène bis naphtalène sulfonate de sodium ..... | 8 % |
| - kaolin..... | 70 % |

Ce mélange est granulé en lit fluide, en présence d'eau, puis séché, concassé et tamisé de manière à obtenir des granulés de dimension comprise entre 0,16 et o,40 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

Les composés selon l'invention peuvent être encore formulés sous forme de solutions organiques encapsulables, notamment par polymérisation interfaciale, dans des capsules à paroi polymériques, par exemple à base de polyamides de polyurées ou de polyamide urées. Ces capsules se trouvent à l'état de dispersion aqueuse concentrée que l'on peut diluer au moment de l'emploi pour obtenir une bouillie de pulvérisation.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général des compositions utilisables dans la présente invention. Les émulsions peuvent être du type eau-dans-l'huile ou huile-dans-l'eau et ils peuvent avoir une consistance épaisse comme celle d'une "mayonnaise".

L'invention concerne de plus un procédé de traitement des végétaux contre les maladies causées par les champignons phytopathogènes notamment ceux de la famille des oomycètes du type Phytophthora sp par exemple Phytophthora infestans (mildiou de la pomme de terre ou de la tomate), Phytophthora Cytrophthora, Phytophthora capsici, Phytophthora cactorum, Phytophthora palmivora, Phytophthora cinnamoni, Phytophthora megasperma, Phytophthora parasitica, Peronospora sp (notamment mildiou du tabac), Plasmopara sp notamment plasmopara viticola (mildiou de la vigne) et plasmopara halstedei (mildiou du tournesol), Pseudoperonospora sp (notamment mildiou des cucurbitacees et du houblon), Bremia lactucae (Bremia de la laitue), ainsi que les champignons du sol.

Ce procédé est caractérisé en ce qu'il consiste à appliquer sur ces végétaux une quantité efficace d'une composition contenant comme matière active un composé selon la formule (I). Par "quantité efficace" on entend une quantité suffisante pour permettre le contrôle et la destruction des champignons présents sur ces végétaux. les doses d'utilisation peuvent toutefois varier dans de larges limites selon le champignon à combattre, le type de culture, les conditions climatiques, et selon le composé utilisé.

En pratique des doses allant de 1 g/hl à 500 g/hl correspondant sensiblement à des doses de matière active par hectare de 10 g/ha à 5000 g/ha environ donnant généralement de bons résultats.

Comme exemples de procédés de traitement utilisables, on peut citer la pulvérisation foliaire ou au sol, le poudrage, le trempage, l'incorporation au sol de granulés, de poudres ou de bouillies, l'arrosage, l'injection dans les arbres, le badigeonnage et le traitement des semences.

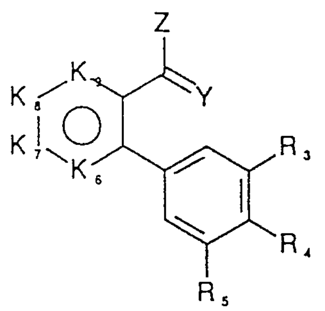

FORMULE I a

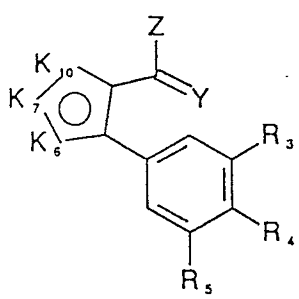

FORMULE I b

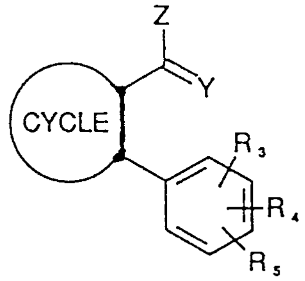

Formule I

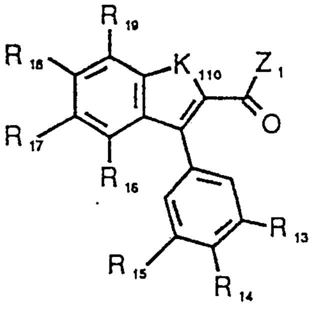

FORMULE II

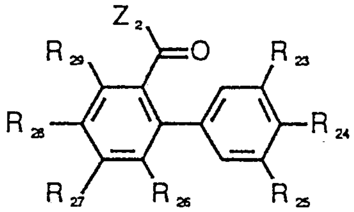

FORMULE III

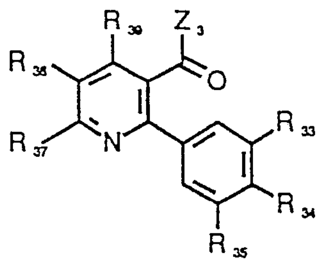

FORMULE IV

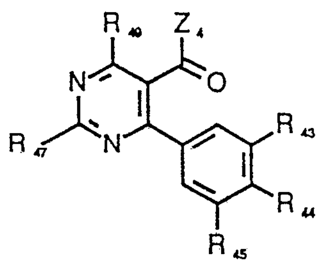

FORMULE V

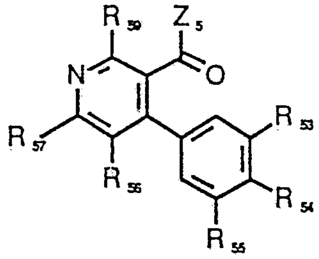

FORMULE VI

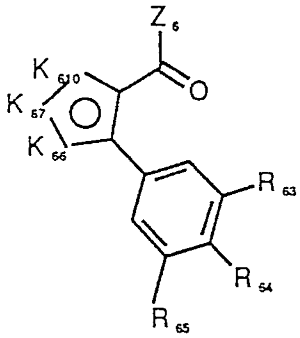

FORMULE VII

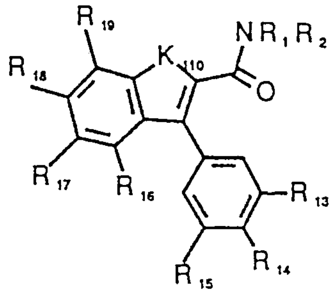

FORMULE II'

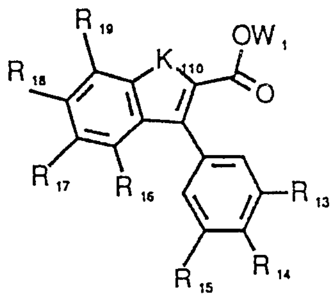

FORMULE II"

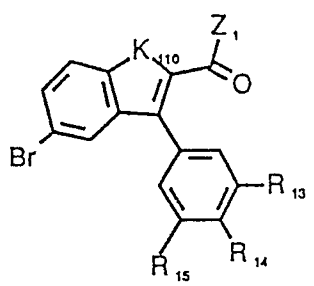

FORMULE II'''

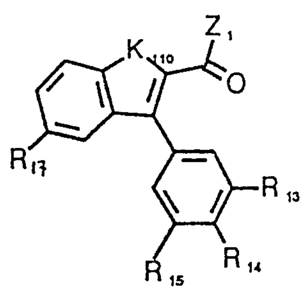

FORMULE II''''

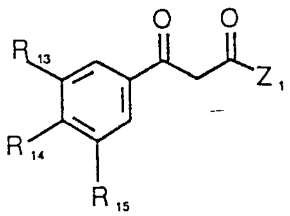

FORMULE XXXII'

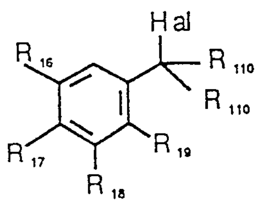

FORMULE XXXII"

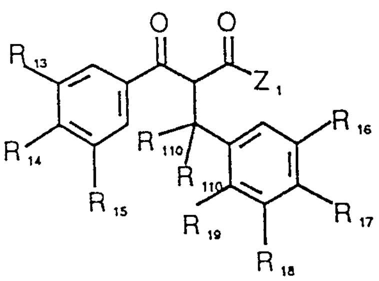

FORMULE XXXII'"

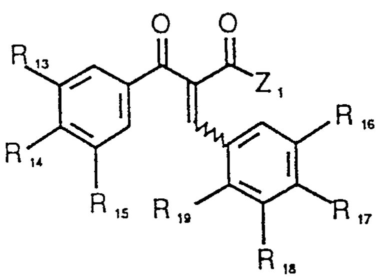

FORMULE XXXXII '

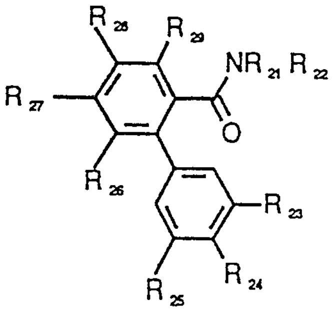

FORMULE III'

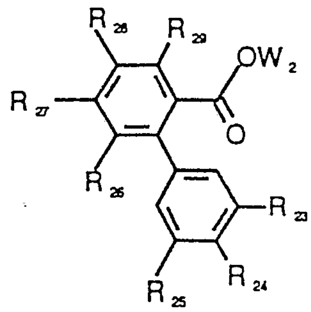

FORMULE III"

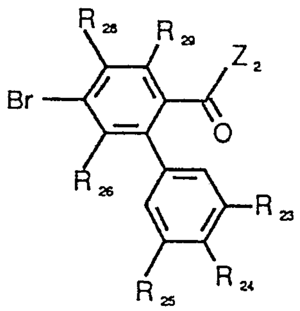

FORMULE XIII'

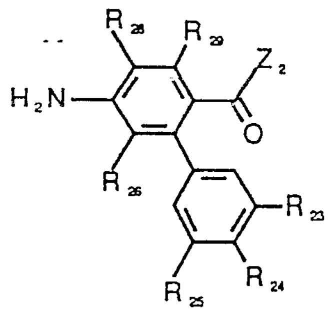

FORMULE XIII"

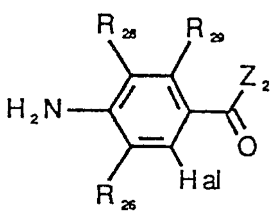

FORMULE XIII'''

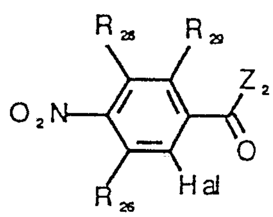

FORMULE XIII''''

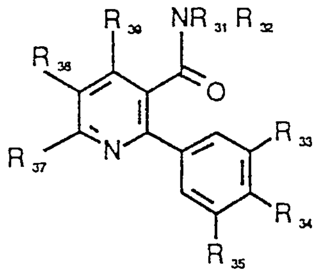

FORMULE IV '

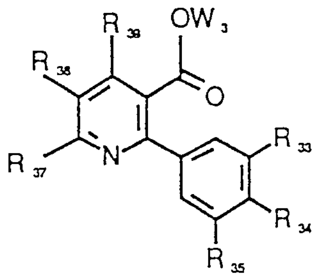

FORMULE IV "

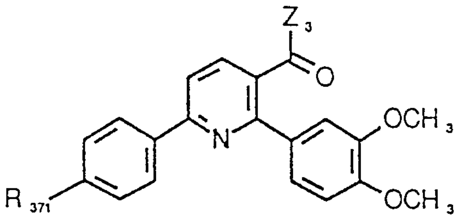

FORMULE IV '''

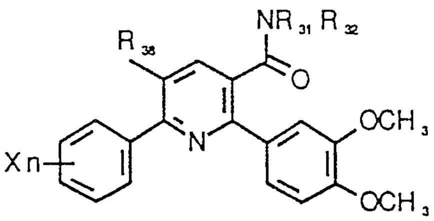

FORMULE IV ''''

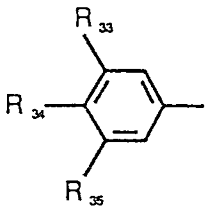

FORMULE XIV "

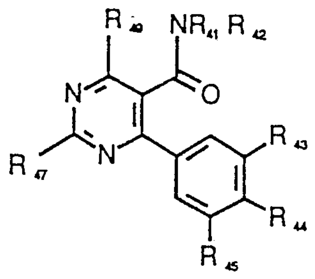

FORMULE V '

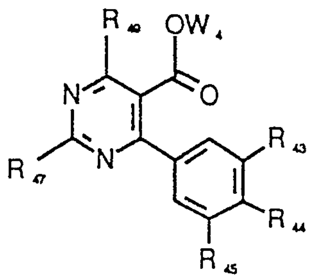

FORMULE V "

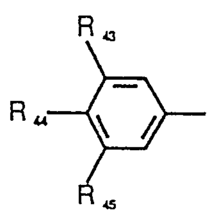

FORMULE XV "

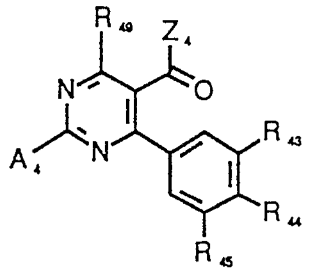

FORMULE XXV '

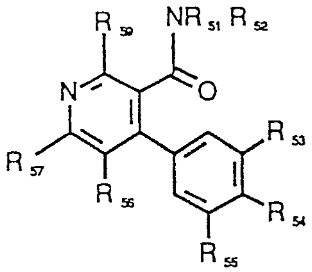

FORMULE VI '

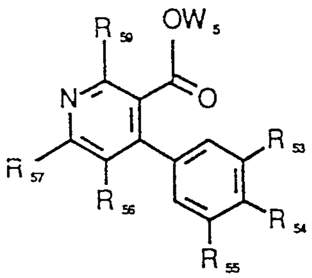

FORMULE VI "

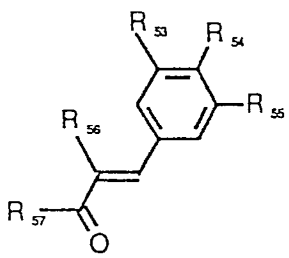

FORMULE XVI '

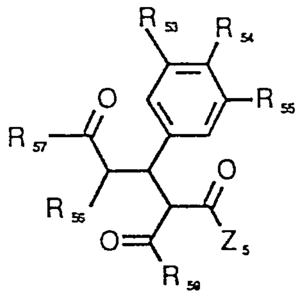

FORMULE XVI "

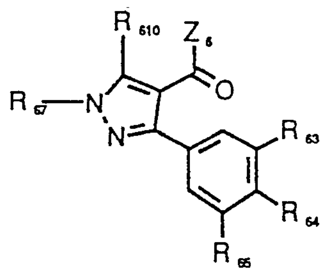

FORMULE VII'

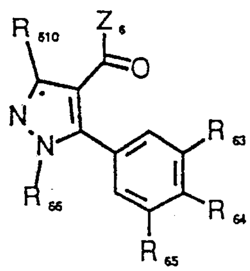

FORMULE VII"

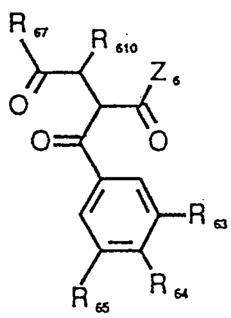

FORMULE XVII '

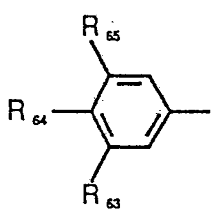

FORMULE XVII'"

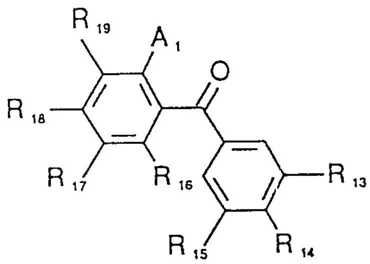

FORMULE XII'

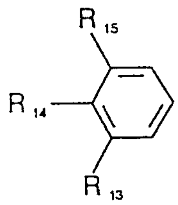

FORMULE XII"

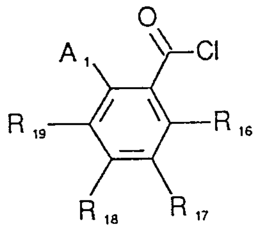

FORMULE XII'''

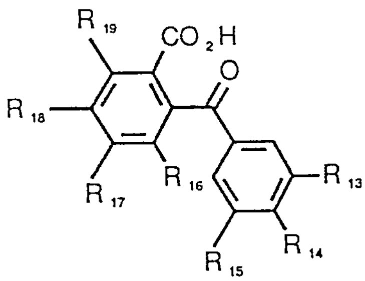

FORMULE XXII'

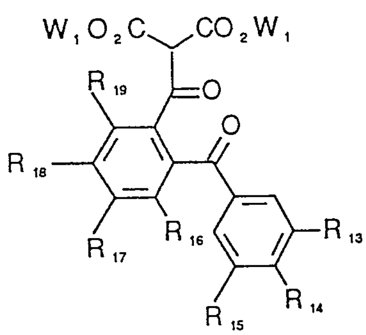

FORMULE XXII"

**Revendications**

1) Composés, utilisables notamment pour le traitement des atteintes fongiques des plantes, caractérisés en ce qu'ils ont la formule (I) dans laquelle :

* Le signe CYCLE entouré d'un demi-cercle, représente, avec les deux atomes de carbone auxquels ce

demi-cercle est relié, un cycle carboné ou hétérocyclique, ayant de 4 à 7 chaînons comportant au moins une liaison insaturée à caractère éthylènique ou aromatique entre les 2 atomes de carbone porteurs du groupe -C = Y et du
|
Z
groupe phényle, ledit cycle étant éventuellement substitué par des radicaux organiques ayant au plus 30 atomes de carbone,

* Y est un atome d'oxygène ou de soufre.

* Z est un groupe $NR_1 R_2$.

* $R_1$ et $R_2$ identiques ou différents représentent :

- un radical alkyle inférieur ou alkoxy inférieur (l'un au moins des radicaux $R_1$ et $R_2$ étant autre que alkoxy), ou cycloalkyle de 3 à 7 atomes de carbone, ces radicaux étant éventuellement substitués par au moins un atome d'halogène et/ou un groupe hydroxy ou alkoxy inférieur, ou alkoxy (inférieur)-alkyle (inférieur) ou acyloxy (inférieur) ou phényle ou un groupe hétérocyclique de 4 à 6 chaînons comprenant 1 ou 2 hétéroatomes (O, N ou S) ou un groupe amino mono-ou disubstitué par des alkyle inférieurs,
- un radical alkényle ou alkynyle ayant de 3 à 7 atomes de carbone.

* ou bien $R_1$ et $R_2$ forment ensemble, avec l'atome d'azote auquel ils sont rattachés, un hétérocycle, saturé ou insaturé, comprenant de 4 à 7 chaînons (de préférence 5 ou 6) et 1, 2 ou 3 hétéroatomes (O, N ou S), cet hétérocycle pouvant être substitué par des groupes alkyle inférieurs ou alkoxy inférieurs ou hydroxy ou un radical oxo ou un ou plusieurs atomes d'halogène ou un groupe alkyl (inférieur)-alkoxy (inférieur).

* $R_3$, $R_4$ et $R_5$ identiques ou différents, représentent:

* un atome d'hydrogène ou d'halogène, l'un au moins de ces radicaux $R_3$, $R_4$ et $R_5$ étant autre qu'un atome d'hydrogène ou d'halogène

* un groupe amino éventuellement substitué par un ou deux groupes alkyle inférieurs

* un groupe alkyle inférieur ou alkoxy inférieur ou alkyl (inférieur)-alkoxy inférieur ou alkylthio inférieur, éventuellement halogéné ou hydroxylé

* $R_3$ et $R_4$ (en position meta et para) peuvent former ensemble un radical unique divalent à 3 ou 4 chaînons comprenant 1 ou 2 atomes d'oxygène non vicinaux.

2) Composés selon la revendications 1 caractérisés en ce que le logarithme de leur coefficient de partage octanol/eau est compris entre 2 et 5.

3) Composés selon la revendication 2 caractérisés en ce que le logarithme de leur coefficient de partage octanol/eau est compris entre 2,5 et 4,5.

4) Composés selon l'une des revendications 1 à 3, caractérisés en ce que :

- $R_1$ et $R_2$ représentent :

* un radical alkyle inférieur ; un radical cycloalkyle ayant de 3 à 7 atomes de carbone ; un radical alkènyle ou alkynyle ayant de 3 à 7 atomes de carbone ; un radical alkoxyalkyle ayant de 3 à 8 atomes de carbone

* ou bien $R_1$ et $R_2$ peuvent former ensemble, avec l'atome d'azote auquel ils sont reliés, un hétérocycle saturé ou insaturé comprenant 4 à 7 chaînons et 1 ou 2 hétéroatomes (O, N, S), cet hétérocycle pouvant être substitué par des radicaux alkyle de 1 à 3 atomes de carbone ou des groupes alkoxyalkyle de 2 à 4 atomes de carbone.

- $R_3$, $R_4$ et $R_5$ sont en position respective méta, para et méta' sur le groupe phényle substituant le CYCLE ( ces positions étant repérées par rapport au lien valentiel reliant ledit groupe phényle avec ledit CYCLE), et

* $R_3$, $R_4$ et $R_5$ représentent un atome d'hydrogène ou d'halogène (l'un au moins de ces 3 radicaux étant autre que l'atome d'hydrogène ou d'halogène) ; ou un radical alkyle inférieur ou un alkoxy inférieur ; ou un alkoxyalkyle ayant de 2 à 4 atomes de carbone ; ou un alkylthio inférieur ; ou un hydroxy ; ou un groupe amino éventuellement mono-substitué ou disubstitué par un radical alkyle inférieur, ou bien

* $R_3$ et $R_4$ (en position meta et para) peuvent former ensemble un radical unique divalent à 3 ou 4 chaînons comprenant 1 ou 2 atomes d'oxygène non vicinaux,

- le signe CYCLE, entouré d'un demi-cercle, représente un radical divalent $-K_6-K_7-K_8-K_9-$ ou $-K_6-K_7-K_{10}-$ de manière à constituer, avec les deux atomes de carbone auxquels il est relié un cycle à 5 ou 6 chaînons [comme représenté graphiquement dans les formules (Ia) et (Ib)], les groupes $K_6$, $K_7$, $K_8$, $K_9$ et $K_{10}$ étant tels que :

$K_6$ représente -N= ou $-C(R_6)-$
$K_7$ représente -N= ou $-C(R_7)-$
$K_8$ représente -N= ou $-C(R_8)-$
$K_9$ représente -N= ou $-C(R_9)-$
$K_{10}$ représente -N= ou $-C(R_{10})-$

et, en outre, dans le cas où le signe CYCLE entouré d'un demi-cercle représente $K_6$, $K_7$, $K_{10}$, ces radicaux $K_6$, $K_7$ et $K_{10}$ peuvent encore être tels que :

$K_6$ représente $-N(R_6)-$ ou $-C(R_6)_2-$ ou -O- ou $-S(O)_n$
$K_7$ représente $-N(R_7)-$ ou $-C(R_7)_2-$ ou -O- ou $-S(O)_n$
$K_{10}$ représente $-N(R_{10})-$ ou $-C(R_{10})_2-$ ou -O- ou $-S(O)_n$
ou -CO-

n étant un nombre entier égal à 0, 1 ou 2,

étant entendu que, dans les deux cas où le signe CYCLE représente $-K_6-K_7-K_8-K_9$ et $-K_6-K_7-K_{10}-$, l'un au moins des groupes $K_6$ et $K_7$ représente $-C(R_6)=$ ou $-C(R_6)_2-$ ou $-C(R_7)=$ ou $-C(R_7)_2-$ et que, de plus, ces radicaux divalents $-K_6-K_7-K_8-K_9-$ et $-K_6-K_7-K_{10}-$ ne contiennent dans leur chaîne principale que 0, 1 ou 2 hétéroatomes (N, O ou S) et aucun enchaînement -O-O- ou -S-S-.

$-R_6$, $R_7$, $R_8$, $R_9$ ou $R_{10}$ chacun étant identique ou différent des autres, représentent :

* un atome d'hydrogène ou d'halogène, l'un au moins des radicaux $R_6$ ou $R_7$ étant autre que l'atome d'hydrogène,

* un groupe cyano, nitro, thiocyanato, hydroxy ou carboxy,

* un alkyle, cycloalkyle, alkényle, alkynyle, alkyl-$S(O)_n$ (avec n = 0, 1 ou 2), alkoxy, cycloalkoxy de 3 à 8 atomes de carbone, alkényloxy, alkynyloxy ; ces radicaux ont avantageusement de 1 à 8 atomes de carbone et peuvent éventuellement être substitués par un ou plusieurs atomes d'halogène,

* un radical phényle, naphtyle, phényl-$S(O)_n$ (avec n = 0, 1 ou 2), phénoxy, phénylalkyle (alkyle inférieur), phénylalkyl-$S(O)_n$ (alkyle inférieur ; n = 0, 1 ou 2), phénylalkoxy (alkoxy inférieur) ou encore un reste d'un hétérocycle éventuellement substitué, saturé ou insaturé, ayant dans le cycle 5 à 6 chaînons avec 1 à 3 hétéroatomes choisis dans le groupe comprenant l'oxygène, le soufre et l'azote, le noyau phényle ou hétérocyclique de chacun de ces radicaux pouvant être substitué par au moins un substituant choisi dans le groupe comprenant :

* un atome d'halogène,

* un groupe nitro, cyano, carboxy, hydroxy, mercapto, thiocyanato, alkoxy (inférieur)-carbonyle, -CO-NR′R″, NR′R″, -N(R′)-CO-R″, -O-CO-R′, -O-CO-NR′R″ ou,

* un radical alkyle inférieur, alkoxy inférieur, alkyl (inférieur)-$S(O)_n$ (avec n = 0, 1 ou 2), cycloalkyle (de 3 à 7 atomes de carbone), phényle, phénoxy, phényl-$S(O)_n$ (avec n = 0, 1 ou 2), phénylalkyle, phénylalkoxy, phénylalkyl-$S(O)_n$ (avec n = O, 1 ou 2), ces radicaux étant éventuellement halogénés et ayant 1 à 4 atomes de carbone pour la partie alkyle.

* Un groupe alkoxy (inférieur)-carbonyle, CO-NR′R″, NR′R″, N(R′)-CO-R″, O-CO-R′, O-CO-NR′R″.

* R′ et R″ identiques ou différents sont chacun un atome d'hydrogène ; un radical alkyle inférieur ; cycloalkyle de 3 à 7 atomes de carbone ; phényle éventuellement halogéné ; phénylalkyle (alkyle inférieur) éventuellement halogéné ; un alkényle ou alkynyle de 3 à 7 atomes de carbone ; alkoxyalkyle de 3 à 8 atomes de carbone.

* Lorsque $K_6$ et $K_7$ représentent $-C(R_6)=$ ou $-C(R_6)_2-$ou $-C(R_7)=$ ou $-C(R_7)_2-$, alors $R_6$ avec $R_7$, ou l'un des radicaux $R_6$ avec l'un des radicaux $R_7$, peuvent former, avec les deux atomes de carbone portant $R_6$ et $R_7$, un cycle carboné ou hétérocyclique, saturé ou insaturé, comprenant 5 ou 6 chaînons et O,1 ou 2 hétéroatomes (O, N ou S) non vicinaux.

* Lorsque $K_6$ ou $K_7$ ou $K_8$, $K_9$ ou $K_{10}$ ont l'une des significations $-N(R_6)-$, $-N(R_7)-$, $-N(R_8)-$, $-N(R_9)-$, $-N(R_{10})-$, alors le radical correspondant , $R_6$, $R_7$, $R_8$, $R_9$ ou $R_{10}$, a l'une des significations suivantes (pourvu que l'un au moins des radicaux $R_6$ ou $R_7$ soit autre que l'atome d'hydrogène) :

l'atome d'hydrogène ; un radical alkyle de 1 à 6 atomes de carbone ; un radical phényle ou phénylalkyle (alkyle inférieur) éventuellement substitués par un ou plusieurs atomes d'halogène ou par un groupe cyano ou nitro ou par un radical alkyle inférieur ou alkoxy inférieur.

5) Composés selon l'une des revendications 1 à 4, caractérisés en ce qu'ils ont la formule (Ia).

6) Composés selon l'une des revendications 1 à 4, caractérisés en ce qu'ils ont la formule (Ib).

7) Composés, selon l'une des revendications 1 à 6, caractérisés en ce qu'ils ont la formule (II) dans laquelle :

* $K_{110}$ est un atome d'oxygène, un groupe $S(O)_{n1}$ dans lequel $n_1$ est un entier égal à zéro, 1 ou 2, ou un groupe $NR_{110}$ ou $C(R_{110})_2$ ou un groupe oxo.

* $Z_1$ représente un groupe $NHR_{11}$ ou $NR_{11}R_{12}$, dans lequel $R_{11}$ et $R_{12}$, identiques ou différents, ont l'une des significations données respectivement pour $R_1$ et $R_2$ dans les revendications précédentes

* $R_{13}$ à $R_{15}$, identiques ou différents, ont l'une des significations données pour $R_3$, $R_4$ et $R_5$ dans les revendications précédentes

* $R_{13}$ et $R_{14}$ peuvent également former ensemble un groupe méthylènedioxy ou éthylènedioxy, les carbones de ces motifs pouvant eux-mêmes être substitués par des radicaux alkyle inférieur ou des atomes d'halogène

* $R_{16}$ à $R_{19}$, identiques ou différents, ont également l'une des significations données pour $R_6$ à $R_9$ dans les revendications précédentes

* si $K_{110}$ est $=C(R_{110})-$ ou $-C(R_{110})_2-$, alors $R_{110}$ représente un atome d'hydrogène ou un radical alkyle inférieur ou un radical benzyle ou un hydroxy, ou un radical alcanoyloxy inférieur (par exemple acétyloxy) ou un alkoxy inférieur,

* si $K_{110}$ est $-N(R_{110})-$, alors $R_{110}$ représente un atome d'hydrogène ou un radical alkyl inférieur ou un radical benzyle.

8) Composés, selon la revendication 7, caractérisés en ce qu'ils ont la formule (II) dans laquelle :

* $K_{110}$ est un atome d'oxygène, de soufre, ou représente $-N(R_{110})-$ ou $-C(R_{110})_2-$,

* $Z_1$ est un groupe $NHR_{11}$ ou $NR_{11}R_{12}$, où $R_{11}$ et $R_{12}$ sont des alkyle inférieurs tels que $R_{11}$ et $R_{12}$ comportent au plus six carbones au total, ou bien forment ensemble un groupe morpholino ou

thiomorpholino,

9) Composés, selon la revendication 7, caractérisés en ce qu'ils ont la formule (II) dans laquelle :

* $Z_1$ est un groupe $NR_{11}R_{12}$ qui est un groupe morpholino, thiomorpholino, N-méthyléthylamino ou N-diéthylamino,

* $R_{13}$ à $R_{15}$, identiques ou différents, sont un atome d'hydrogène ou d'halogène, un alkyle inférieur, un alkoxy inférieur, un alkylthio inférieur,

*$R_{16}$, $R_{18}$, $R_{19}$ sont un atome d'hydrogène ou de fluor,

* $R_{17}$ est un atome d'hydrogène ou d'halogène ; un radical alkyle inférieur, alkoxy inférieur, alkylthio inférieur, halogénoalkyle inférieur, halogénoalkoxy inférieur, halogénoalkylthio inférieur ; un groupe nitro, cyano ; un radical alkényle inférieur, un alkynyle inférieur ou un alkoxy (inférieur)-carbonyle.

10) Composés, selon l'une des revendications 7 à 9, caractérisés en ce que le logarithme deleur coefficient de partage octanol-eau (log P) est compris entre 2,5 et 4.

11) Composés, selon l'une des revendication 1 à 6, caractérisés en ce qu'ils ont pour formule la formule (III) dans laquelle :

* $Z_2$ représente un groupe $NR_{21}R_{22}$ dans lequel $R_{21}$ et $R_{22}$, identiques ou différents, ont l'une des significations données respectivement pour $R_1$ et $R_2$ dans l'une des revendications précédentes

*$R_{23}$ à $R_{25}$ identiques ou différents ont l'une des significations données respectivement pour $R_3$, $R_4$ et $R_5$ dans l'une des revendications précédentes

* $R_{23}$ et $R_{24}$ peuvent également former ensemble un groupe méthylènedioxy ou éthylènedioxy, les carbones de ces motifs pouvant eux-mêmes être substitués par des radicaux alkyle inférieur ou des atomes d'halogène.

* $R_{26}$ et $R_{27}$, identiques ou différents, ont l'une des significations données respectivement pour $R_6$ et $R_7$ dans l'une des revendications précédentes sous réserve que $R_{26}$ et $R_{27}$ ne sont pas simultanément un atome d'hydrogène.

* $R_{28}$ et $R_{29}$, identiques ou différents, représentent un atome, d'hydrogène ou d'halogène ; le groupe hydroxy ; un radical alkyle inférieur ; alkoxy inférieur ; alkoxy (inférieur)-alkyle (inférieur); alkoxy (inférieur)-carbonyle.

12) Composés, selon la revendication 11, caractérisés en ce que

*$Z_2$ est un groupe $NR_{21}$ $R_{22}$ où $R_{21}$ et $R_{22}$ sont des alkyle inférieurs tels que $R_{21}$ et $R_{22}$ comportant au plus six carbones au total, ou bien forment ensemble un groupe morpholino ou thiomorpholino.

* $R_8$ et $R_9$, identiques ou différents, représentent un atome d'hydrogène ou d'halogène ou un groupe hydroxy.

13) Composés, selon l'une des revendications 11 ou 12, caractérisés en ce que

* $Z_2$ est un groupe $NR_{21}$ $R_{22}$ qui est un groupe morpholino, thiomorpholino, N-méthyl éthylamino ou N-diéthylamino.

* $R_{23}$ à $R_{25}$, identiques ou différents, sont un atome d'hydrogène ou d'halogène un alkyle inférieur, un alkoxy inférieur, un alkylthio inférieur.

* $R_{26}$ à $R_{27}$, identiques ou différents, sont un atome d'hydrogène ou d'halogène, un groupe alkyle inférieur, un groupe alkényle inférieur, un groupe alkoxy inférieur, un groupe alkylthio inférieur, un groupe halogénoalkyle inférieur, un groupe halogénoalkoxy inférieur ou halogénoalkylthio inférieur.

* $R_{28}$ et $R_{29}$, identiques ou différents, sont un atome d'hydrogène ou de fluor.

14) Composés, selon l'une des revendications 11 à 13, caractérisés en ce que le logarithme de leur coefficient de partage octanol-eau (log P) est compris entre 2,5 et 4.

15) Composés, selon l'une des revendications 1 à 6, caractérisés en ce qu'ils ont pour formule la formule (IV) dans laquelle :

* $Z_3$ représente un groupe $NR_{31}$ $R_{32}$, dans lequel $R_{31}$ et $R_{32}$, identiques ou différents, ont l'une des significations données respectivement pour $R_1$ et $R_2$, dans les revendications précédentes.

* $R_{33}$ à $R_{35}$, identiques ou différents, ont l'une des significations données respectivement pour $R_3$, $R_4$ et $R_5$ dans les revendications précédentes.

* $R_{33}$ et $R_{34}$ peuvent également former ensemble un groupe méthylènedioxy ou éthylènedioxy, les carbones de ces groupes pouvant eux-mêmes être substitués par des alkyle inférieurs ou des atomes d'halogène.

* $R_{37}$ a l'une des significations données pour $R_7$, dans les revendications précédentes, sous réserve que $R_7$ ne soit pas un atome d'hydrogène.

* $R_{38}$ et $R_{39}$, identiques ou différents, peuvent être un atome d'hydrogène ou d'halogène, un radical alkyle inférieur, alkoxy inférieur, alkoxy (inférieur)-alkyle (inférieur) ou alkoxycarbonyle inférieur ou un groupe cyano. De plus, lorsque $R_{37}$ est un phényle, $R_{38}$ peut former, avec l'atome de carbone de $R_{37}$ en ortho de celui relié au noyau pyridinique, une chaîne $Y_3$-$(CH_2)_{n3}$, dans laquelle $Y_3$ est un atome d'oxygène ou de soufre ou un groupe méthylène et $n_3$ un nombre égal à 0, 1 ou 2.

16) Composés, selon la revendication 15, caractérisés, en ce qu'ils ont pour formule la formule (IV) dans laquelle $R_{33}$ à $R_{35}$, identiques ou différents, sont un atome d'hydrogène ou d'halogène (l'un au moins de ces trois radicaux étant autre que l'atome d'hydrogène ou d'halogène) ou un alkyle inférieur, un alkoxy inférieur ou un alkylthio inférieur.

17) Composés, selon l'une des revendications 1 à 6, caractérisés en ce qu'ils ont pour formule la (V) dans laquelle:

* $Z_4$ représente un groupe $NR_{41}$ $R_{42}$ dans lequel $R_{41}$ et $R_{42}$, identiques ou différents, ont l'une des significations données respectivement pour $R_1$ et $R_2$ dans les revendications précédentes.

* $R_{43}$, $R_{44}$, $R_{45}$, identiques ou différents, ont l'une des significations données respectivement pour $R_3$, $R_4$ et $R_5$ dans les revendications précédentes.

* $R_{47}$ a une des significations données pour $R_7$, dans les revendications précédentes, mais est autre que l'atome d'hydrogène.

* $R_{49}$ peut être un atome d'hydrogène ou un radical alkyle inférieur.

18) Composés, selon la revendication 17, caractérisés en ce qu'ils ont pour formule la (V) dans laquelle :

* $Z_4$ est un groupe $NR_{41}$ $R_{42}$, dans lesquels $R_{41}$ et $R_{42}$ sont des alkyle inférieurs tels que $R_{41}$ et $R_{42}$ comportent au plus six carbones au total, ou bien forment ensemble un groupe morpholino ou thiomorpholino.

* $R_{43}$ à $R_{45}$, identiques ou différents, sont un atome d'hydrogène ou d'halogène, un alkyle inférieur, un alkoxy inférieur, un alkylthio inférieur.

* $R_{49}$ est un atome d'hydrogène.

19) Composés, selon l'une des revendications 1 à 6, caractérisés en ce qu'ils ont pour formule la formule (VI) dans laquelle :

* $Z_5$ représente un groupe $NR_{51}R_{52}$ dans lequel $R_{51}$ et $R_{52}$, identiques ou différents, ont l'une des significations données respectivement pour $R_1$ et $R_2$ dans les revendications précédentes.

* $R_{53}$, $R_{54}$, $R_{55}$, identiques ou différents, ont l'une des significations données respectivement pour $R_3$, $R_4$, et $R_5$ dans les revendications précédentes.

* $R_{53}$ et $R_{54}$ peuvent également former ensemble en groupe méthylènedioxy ou éthylènedioxy, les carbones de ces groupes pouvant eux-mêmes être substitués par des alkyle inférieurs ou des atomes d'halogène.

* $R_{56}$ et $R_{57}$ ont l'une des significations données pour $R_6$ et $R_7$, dans les revendications précédentes, sous réserve que $R_6$ et $R_7$ ne sont pas simultanément un atome d'hydrogène.

* $R_{59}$ peut être un atome d'hydrogène ou d'halogène un radical alkyle inférieur, un radical hydroxy, alkoxy inférieur ou alkoxyalkyle inférieur, ces radicaux pouvant être halogénés.

20) Composés, selon la revendication 19, caractérisés en ce qu'ils ont pour formule la formule (VI), dans laquelle $R_{53}$ à $R_{55}$, identiques ou différents, sont un atome d'hydrogène ou d'halogène ou un alkyle inférieur, un alkyloxy inférieur ou un alkylthio inférieur.

$R_{59}$ est un atome d'hydrogène ou un alkyle inférieur.

21) Composés, selon l'une des revendications 1 à 6 caractérisés en ce qu'ils ont pour formule la formule (VII) dans laquelle :

* $Z_6$ représente :

- un groupe $NR_{61}$ $R_{62}$ dans lequel $R_{61}$ et $R_{62}$, identiques ou différents, ont l'une des significations données respectivement pour $R_1$ et $R_2$ dans les revendications précédentes.

* $K_{66}$ est un atome d'azote substitué $-N(R_{66})-$, ou $-N=$, ou un atome de soufre ou d'oxygène ou un atome de carbone substitué $=C(R_{66})-$.

*$K_{67}$ est un atome de soufre ou d'oxygène ou un atome d'azote substitué $-N(R_{67})-$, ou $-N=$, ou un atome de carbone substitué $=C(R_{67})-$, étant entendu que $K_{66}$ et $K_{67}$ ne peuvent pas simultanément être $-N(R_{66})$-et $-N(R_{67})-$.

* $K_{610}$ est un atome d'azote substitué $-N(R_{610})-$, ou $-N=$, ou un atome de soufre ou d'oxygène, ou un atome de carbone substitué $=C(R_{610})-$, étant entendu que $K_{610}$ et $K_{67}$ ne peuvent pas simultanément être l'un $-N(R_{610})$ et l'autre $N(R_{67})-$.

* $R_{63}$, $R_{64}$, $R_{65}$, identiques ou différents, ont l'une des significations données respectivement pour $R_3$, $R_4$, $R_5$ dans les revendications précédentes.

* $R_{66}$, $R_{67}$ et $R_{610}$, identiques ou différents, ont l'une des significations données respectivement pour $R_6$, $R_7$ et $R_{10}$ dans les revendications précédentes.

22) Composés, selon la revendication 21, caractérisés en ce qu'ils ont pour formule la formule (VII) dans laquelle :

* $Z_6$ est un groupe $NR_{61}$ $R_{62}$ dans lesquels $R_{61}$ et $R_{62}$ comportent au plus six carbones au total, ou bien forment ensemble un groupe morpholino ou thiomorpholino.

* $K_{66}$ est un atome d'azote substitué $-N(R_{66})-$.

* $K_{67}$ est un atome d'azote $-N=$ ou un atome de carbone substitué $=C(R_{67})-$.

* $K_{610}$ est un atome de carbone $=CH-$.

* $R_{63}$, $R_{64}$, $R_{65}$, identiques ou différents, sont un atome d'hydrogène ou d'halogène, ou un alkyle inférieur, un alkoxy inférieur, un alkylthio inférieur.

* $R_{66}$ et $R_{67}$ sont un atome d'hydrogène, un groupe alkyle, phényle, phénylalkyle éventuellement substitués par des halogènes ou des groupements halogénoaikyles étant bien entendu que $R_{66}$ et $R_{67}$ ne peuvent pas simultanément être un atome d'hydrogène.

23) Compositions utiles pour la lutte contre les atteintes fongiques des plantes, caractérisées en ce qu'elles contiennent une quantité efficace d'une matière active selon l'une des revendications 1 à 22.

24) Compositions, selon la revendication 23, caractérisée en ce qu'elles comprennent un support en plus de la matière active.

25) Compositions, selon l'une des revendications 23 ou 24, caractérisées en ce qu'elles comprennent, en outre, un tensio actif.

26) Compositions, selon l'une des revendications 23 à 25, caractérisées en ce qu'elles comprennent 0,0001 à 95 % de matière active et 0 à 20 % d'agent tensio actif.

27) Compositions, selon la revendication 26, caractérisés en ce qu'elles comprennent entre 0,0005 et 90 % de matière active.

28) Compositions commercialisables, selon la revendication 27, caractérisées en ce qu'elles comprennent entre 0,1 et 95 % de matière active.

29) Compositions, selon la revendication 27, caractérisées en ce qu'elles comprennent entre 0,5 et 90 % de matière active.

30) Compositions, selon l'une des revendications 27 à 29, caractérisées en ce qu'elles comprennent 0,5 et 15 % d'agent(s) tensioactif(s).

31) Compositions, selon l'une des revendications 23 à 30, caractérisées en ce qu'elles sont sous forme de concentrés émulsionnables comprenant 10 à 80 % de matière active.

32) Compositions, selon l'une des revendications 23 à 30, caractérisés en ce qu'elles sont sous forme de suspensions aqeuses concentrées comprenant 10 à 75 % de matière active.

33) Composition, selon l'une des revendications 23 à 26 ou 28, caractérisés en ce qu'elles sont sous forme de poudres mouillables concentrées comprenant 10 à 95 % de matières actives.

34) Compositions, selon l'une des revendications 23 à 30, caractérisés en ce qu'elles sont sous forme de granulés comprenant 0,5 à 25 % de matière active.

35) Compositions, selon l'une des revendications 23 à 30, caractérisés en ce qu'elles sont sous forme de granulés dispersibles comprenant 1 à 90 % de matière active.

36) Procédé pour lutter contre les atteintes fongiques des végétaux caractérisés en ce qu'on applique une quantité efficace de matière active selon l'une des revendications 1 à 22 ou une composition selon l'une des revendications 23 à 35.

37) Procédé selon la revendication 36 caractérisé en ce qu'on traite une culture infestée ou susceptible d'être infestée par des champignons de la famille des mildious.

38) Procédé selon l'une des revendications 36 ou 37 caractérisé en ce que la matière active est appliquée à raison de 0,01 à 5 kg/ha.

39) Procédé selon la revendications 38 caractérisé en ce qu'on applique la matière active à raison de 0,02 à 1,5 kg/ha.

40) Procédé de préparation de composés selon l'une des revendications 1 à 16 caractérisé en ce qu'on opère selon l'une ou l'autre des caractéristiques suivantes :

a) On prépare un composé de formule (II) dans laquelle $K_{110}$ est -O-, -S- ou $NHR_{110}$, et que l'on part d'une benzophénone de formule (XII') dans laquelle $R_{13}$ à $R_{19}$ ont les significations indiquées dans les revendications précédentes, et $A_1$ est un atome d'halogène substituable (tel que le brome ou, de préférence, le chlore ou le fluor) ou un groupe nitro, et que l'on fait réagir cette benzophénone avec un dérivé de formule :

$H\text{-}K_{110}\text{-}CH_2\text{-}CO\text{-}Z_1$

dans laquelle $K_{110}$ et $Z_1$ ont la signification donnée dans les revendications précédentes.

b) Des dérivés de formule (II") sont transformés (procédé G) en dérivés de formule (II') par chauffage, à une température comprise entre 100° C et 200° C et éventuellement sous pression, avec un excès d'amine $HNR_{11}R_{12}$.

c) Un dérivé de formule (II') est préparé à partir d'autres dérivés de formule (II') par aménagement fonctionnel.

d) Un dérivé nitré (formule (II) dans laquelle $R_{16}$ ou $R_{17}$ ou $R_{18}$ ou $R_{19}$ est $NO_2$) est réduit (procédé B) en dérivé aminé par un réducteur.

e) Un dérivé aminé (formule (II) dans laquelle $R_{16}$ ou $R_{17}$ ou $R_{18}$ ou $R_{19}$ sont $NH_2$) est soumis à des réactions d'acylation (procédé C) ou de diazotation.

f) Une réaction d'acylation est effectuée à l'aide de dérivés d'acide carboxylique (chlorure ou anhydride d'acide) en présence d'une base organique ou minérale au sein d'un solvant.

g) Une diazotation (procédé D) est réalisée en traitant une solution du dérivé aminé (formule (II) dans laquelle $R_{16}$ ou $R_{17}$ ou $R_{18}$ ou $R_{19}$ sont $NH_2$) dans un acide minéral fort (acide chlorhydrique, bromhydrique ou sulfurique) par un nitrite alcalin (nitrite de sodium), à une température comprise entre -10° C et +15° C et que le sel de diazonium ainsi obtenu est alors décomposé à des températures comprises entre + 5° C et +100° C.

h) On réalise une réaction de couplage arylique (procédé E) avec un acide boronique $R_{17}B(OH)_2$ dans lequel $R_{17}$ peut être un hétérocycle ou un phényl ou un groupe vinyle (ces groupes peuvent être substitués comme indiqué dans la définition de $R_7$) pour conduire à d'autres dérivés selon les revendications précédentes.

i) On fait réagir à une température comprise entre 40° C et la température d'ébullition du solvant un composé de formule (II) dans lequel un des substituants $R_{16}$ à $R_{19}$ est un groupe amino non substitué et $Z_1$ et $R_{13}$ à $R_{15}$ ont la signification déjà indiquée avec un nitrite d'alkyle inférieur au sein d'un solvant pour obtenir un dérivé de formule (II) dans lequel le groupe amino est remplacé par un atome d'hydrogène et où $Z_1$ et $R_{13}$ à $R_{19}$ ont la signification déjà indiquée

j) Les dérivés de formule (XII′) sont préparés par une réaction de Friedel-Crafts selon le schéma :
(XII‴) + (XII″) → (XII′)
dans lequel $A_1$ et $R_{13}$ à $R_{19}$ ont la même signification que dans les revendications précédentes.

k) On prépare des composés de formule (II) dans laquelle $K_{110}$ est un groupe $C(R_{110})_2$ et où $R_{13}$ à $R_{19}$ ont les mêmes significations que dans les revendications précédentes, et l'on fait réagir une benzophénone de formule (XXII′) dans laquelle $Z_{11}$ = OH et $R_{13}$ à $R_{19}$ ont les mêmes significations que dans les revendications précédentes, avec du chlorure de thionyle ($SOCl_2$), du chlorure de phosphoryle ($POCl_3$), du trichlorure de phosphore ou du pentachlorure de phosphore ($PCl_3$ ou $PCl_5$).

l) Un composé de formule (XXII′), préparé selon k, est mis à réagir avec un malonate d'alkyle inférieur en présence d'une base qui est préférentiellement un alkanoate de magnésium, et le composé obtenu est un composé traité à chaud à une température comprise entre 100° C et 200° C éventuellement sous pression avec un excès d'une amine $HNR_{11}R_{12}$, en présence ou non d'un solvant, pour fournir un composé de formule (II) dans lequel $K_{110}$ est un groupe oxo et où $R_{13}$ à $R_{19}$ ont la même signification que dans les revendications précédentes, et $Z_1$ est un groupe $NR_{11}$ $R_{12}$ tel que défini et que ce composé est éventuellement réduit par un réducteur approprié.

m) Un composé de formule (II) dans laquelle $K_{110}$ est un groupe $C(R_{110})_2$ et où $R_{13}$ à $R_{19}$ ont les mêmes significations que dans les revendications précédentes, est préparé en faisant réagir un dérivé de formule (XXXII′), dans lequel $Z_1$ est un groupe $NR_{11}$ $R_{12}$ avec un halogénure de benzyle de formule (XXXII′) où $R_{16}$ à $R_{110}$ ont la même signification que dans les revendications précédentes, et où Hal désigne un atome d'halogène tel que le chlore ou le brome, et ce composé est traité par un acide organique ou minéral, de préférence l'acide polyphosphorique en présence ou non d'un solvant à une température comprise entre 0° C et 150° C.

n) Un composé de formule (II) dans laquelle $K_{110}$ est un groupe $C(R_{110})_2$ et où $R_{13}$ à $R_{19}$ ont les mêmes significations que dans les revendications précédentes, en préparé en faisant réagir un dérivé de formule (XXXII′) dans lequel $Z_1$ est un groupe $OW_1$ ou $NR_{11}$ $R_{12}$ avec un benzaldéhyde substitué par des radicaux $R_{16}$, $R_{17}$, $R_{18}$ ou $R_{19}$, puis ce composé est ensuite réduit par un réducteur, puis l'alcool allylique obtenu est cyclisé par traitement par un acide organique ou minéral en quantité catalytique ou supérieure à la stoéchiométrie en présence ou non d'un solvant à une température comprise entre 0° C et 150° C.

o) La préparation de composés de formule (III) s'effectue, selon un procédé, par une réaction de couplage arylique entre un acide boronique $R_{27}B(OH)_2$ [dans lequel $R_{27}$ peut être un hétérocycle, un phényle ou un groupe vinyle ( ces groupes pouvant être substitués comme indiqué dans la définition de $R_7$)] et un dérivé bromé de formule (XIII′), étant entendu qu'aucun des autres substituants ne peut être simultanément un atome de brome ou d'iode, le schéma réactionnel de ce couplage arylique étant :

base

(XIII') + $R_{27}$ $B(OH)_2$ ——————> (III)

catalyseur

p) Le dérivé bromé de formule (XIII′) est obtenu par diazotation de l'amine de formule (XIII″)

$NaNO_2$ , HBr

(XIII'') ——————————————> (XIII')

CuBr

et le sel de diazonium ainsi obtenu est alors décomposé à des températures comprises entre + 5° C et + 90° C, en présence d'acide bromhydrique et de bromure cuivreux.

q) L'amine (XIII″) est obtenue par une réaction de couplage arylique entre un acide phénylboronique de formule ($A_2$) convenablement substitué par $R_{23}$, $R_{24}$, $R_{25}$ et un dérivé halogéné (bromé ou iodé) de formule (XIII‴), selon le schéma:

base

(XIII''') + ($A_2$ ) ——————> (XII'')

catalyeur

dans lesquelles formules les radicaux $Z_2$, $R_{26}$, $R_{28}$, et $R_{29}$, sous réserve que $R_{26}$, $R_{28}$ et $R_{29}$ ne soient pas simultanément un atome de brome ou d'iode, ont la même signification que celles indiquées que dans les revendications précédentes, et Hal représente un atome d'halogène, en

particulier brome ou iode.

r) La préparation de composés de formule (IV) s'effectue par cyclisation d'un dérivé de formule (XIV')

```
                  R38    R39    CO - Z3
                   |      |      |
R37 - CO - CH - CH - CH - CO - Ar3          (XIV')
```

dans laquelle $Ar_3$ est un groupe de formule (XIV") c'est-à-dire phényle substitué par $R_{33}$ $R_{34}$ et $R_{35}$, et $Z_3$, $R_{33}$ à $R_{39}$ ont la même signification que dans les revendications précédentes, à chaud en présence d'un donneur d'ammoniac, en milieu acide.

s) Les dérivés intermédiaires de formule (XIV') sont préparés selon un procédé caractérisé en ce qu'on effectue la réaction d'une 3-propiocétone et d'une benzoyl-acétate ou acétamide convenablement substitué selon le schéma suivant :

```
                  R38    R39
                   |      |
R37 - CO - CH - CH - T3 + Z3 - CO - CH2 - CO - Ar3
——> (XIV')
```

dans lesquelles formules $Z_3$ et $R_{33}$ à $R_{39}$ ont les mêmes significations que pour la formule (XIV'), $T_3$ désigne un groupe partant tel qu'un atome d'halogène ou un ammonium quaternaire, en présence d'une base minérale forte et d'un solvant.

t) On passe des dérivés de formule (IV") avec $W_3$ = alkyl inférieur aux dérivés de formule (IV'), dans laquelle $Z_3$ est le groupe $NR_{31}R_{32}$ par saponification, comme indiqué au paragraphe précédent, puis halogénation de ces acides en halogénures d'acides correspondants et réaction de ces halogénures avec une amine de formule $NHR_{31}R_{32}$ dans laquelle $R_{31}$ et $R_{32}$ ont la signification indiquée dans les revendications précédentes.

u) On fait réagir une énone de formule (XV')

```
            X4
            |
O = C - C = C - R49     (XV')
    |   |
    Z4  CO
        |
        Ar4
```

dans laquelle

. $Ar_4$ a la formule (XV")

. $R_{43}$, $R_{44}$, $R_{45}$, $R_{49}$ et $Z_4$ ont la même signification que dans la formule (V) et

. $X_4$ est un groupe dialkyl (inférieur)-amino ou alkoxy (inférieur),

avec une amidine de formule

```
R47 - C = NH
      |
      NH2
```

ou un de ses sels de formule

```
R47 - C = N+H2        anion-
      |
      NH2
```

dans lesquelles $R_{47}$ a la même signification que dans les revendications précédentes, en présence

d'un solvant en milieu basique.

v) Les dérivés de formule (XV') sont préparés selon un procédé, dans lequel on fait réagir un dérivé de formule (XV''')

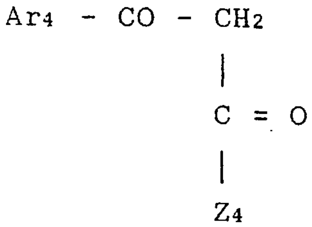

(XV"')

dans laquelle $Ar_4$, $R_{43}$, $R_{44}$ et $R_{45}$, et $Z_4$ ont les significations indiquées ci-dessus avec un dérivé de formule (XV"").

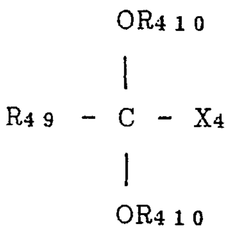

(XV"")

dans lequel $R_{49}$ et $X_4$ ont la même signification que ci-dessus, et $R_{410}$ est un radical alkyle inférieur.

w) Selon un second procédé de préparation des dérivés de formule (V), dans le cas où $R_{47}$ n'est pas un radical alkyl-ou benzyl-sulfinyle ou sulfonyle, on fait réagir un dérivé de formule (XXV') dans laquelle $R_{43}$ à $R_{45}$, $R_{49}$ et $Z_4$ ont les significations indiquées ci-dessus et $A_4$ est un groupe alkyl(inférieur)sulfonyle ou benzylsulfonyle, avec un dérivé de formule $R_{47}H$ dans lequel $R_{47}$ est un radical ayant même signification que $R_7$ sous réserve que le lien valentiel le reliant à l'atome d'hydrogène soit également lié dans $R_{47}$ à un hétéroatome tel que O, S ou $NR_{47}$.

x) Selon ce procédé, on fait réagir (Réaction de Michael) une phénylcétone 2-éthylénique de formule (XVI') avec un ester ou un amide β-cétonique, de formule $R_{59}$-CO-$CH_2$-$COZ_5$, dans lesquels $R_{53}$ à $R_{59}$ et $Z_5$ ont la signification précédente, en présence d'une base organique ou minérale, comme par exemple la baryte partiellement deshydratée au sein d'un alkanol inférieur tel que l'alcool éthylique et à une température comprise entre 0° C et la température d'ébullition du solvant utilisé, et Le composé de formule (XVI") obtenu, dans la formule duquel $R_{53}$ à $R_{59}$ et $Z_5$ ont la signification précédente, est éventuellement cyclisé par simple chauffage de 40° C au reflux du solvant en milieu acide carboxylique.

y) Les composés selon la formule (VII) dans laquelle $K_{66}$ est 0 et $K_{67}$ est -C($R_{67}$)=, et $K_{610}$ est -C($R_{610}$)= se préparent à partir d'esters ou d'amides β,gamma-dicétoniques de formule (XVII') par cyclisation dans un alcool ou un acide carboxylique, tel que l'acide acétique, par simple chauffage du réactif en présence d'une quantité catalytique (0,1 à 5 %) d'acide fort.

z) Les composés selon la formule (VII) dans laquelle $K_{66}$ est $NR_{66}$, $K_{67}$ est -C($R_{67}$)=, $K_{610}$ est -C($R_{610}$)= se préparent à partir d'esters ou d'amides β,gamma-dicétoniques de formule (XVII') par cyclisation en milieu acide à chaud en présence d'une amine de formule $NHR_{66}$ dans laquelle $R_{66}$ a la signification précédente.

aa) Les esters et amides dicétoniques de formule (XVII') sont préparés selon un procédé caractérisé en ce qu'on effectue la réaction d'une halogénocétone (éventuellement substituée par des radicaux $R_{67}$ et $R_{610}$) de formule (XVII") avec un benzoylacétate ou acétamide convenablement substitué selon le schéma suivant :

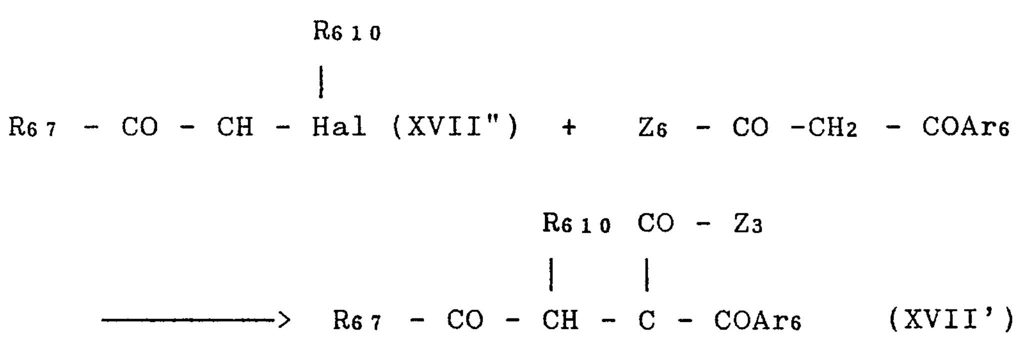

(XVII") (XVII')

dans laquelle $Ar_6$ est un groupe de formule (XVII''''), c'est-à-dire un phényle substitué par $R_{63}$, $R_{64}$ et $R_{65}$ dans lesquelles formules $Z_6$ et $R_{63}$ à $R_{610}$ ont les mêmes significations que dans les revendications précédentes, Hal désigne un groupe partant tel qu'un atome d'halogène.

ab) Les composés selon la formule (VII) dans laquelle $K_{66}$ est -N = ou-N($R_{66}$)- et $K_{67}$ est -N = ou -N($R_{67}$)-, sous réserve que $K_{66}$ et $K_{67}$ ne soient pas simultanément -N($R_{66}$)- et N($R_{67}$)-, se préparent par réaction d'une énone de formule (XXVII')

```
                 X6
                 |
O = C - C = C - R610      (XXVII')
    |   |
    Z6  CO
        |
        Ar6
```

dans laquelle $Ar_6$ est un groupe de formule (XVII'''') c'est-à-dire un phényl substitué par $R_{63}$, $R_{64}$ et $R_{65}$ ; $Z_6$, $R_{610}$ ont la même signification que dans les revendications précédentes, et $X_6$ est un groupe dialkyl(inférieur) amino ou alkoxy avec une hydrazine éventuellement substituée par au plus un radical $R_{66}$. La réaction s'effectue au sein d'un alkanol tel que l'alcool éthylique en présence d'une base minérale ou organique telle que la triéthylamine.

ac) On prépare un composé à partir de la formule (VII), si le dérivé dans la formule desquels $Z_6$ = $OW_6$ est obtenu, en le transformant en dérivé dans la formule desquels $Z_6$ = $NR_{61}$ $R_{62}$, par chauffage à une température comprise entre 100° C et 200° C, éventuellement sous pression, avec un excès d'une amine $HNR_{61}R_{62}$.

ad) A partir de composés dans la formule (VII) desquels $Z_6$ est O-alkyle, on obtient des composés dans la formule desquels $Z_6$ est $OM_6$ où $M_6$ est un métal alcalin, par une réaction de saponification avec une base minérale.

ae) A partir du dérivé dans la formule (VII) duquel $Z_6$ est OH, on obtient le composé dans la formule duquel $Z_6$ est $NR_{61}$ $R_{62}$ après activation de la fonction acide carboxylique par un agent tel que le chlorure de thionyle ($SOCl_2$), le chlorure de phosphoryle ($POCl_3$), le trichlorure ou le pentachlorure de phosphore ($PCl_3$, $PCl_5$), le dicyclohexylcarbodiimide, le diimidazole carbonyle, les chloroformiates d'alcoyle ou l'anhydride trifluoracétique puis une réaction avec l'amine $HNR_{61}$ $R_{62}$ en présence d'une base organique ou minérale au sein d'un solvant organique tel qu'un solvant chloré ou aromatique (notamment un hydrocarbure) ou un éther tel que le THF.

41) Composés caractérisés en ce qu'ils ont la formule (I) dans laquelle :

* Le signe CYCLE entouré d'un demi-cercle, représente, avec les deux atomes de carbone auxquels ce demi-cercle est relié, un cycle carboné ou hétérocyclique, ayant de 4 à 7 chaînons (de préférence 5 ou 6) comportant au moins une liaison insaturée à caractère éthylènique ou aromatique entre les 2 atomes de carbone porteurs du groupe

```
-C = Y
 |
 Z
```

et du groupe phényle, ledit cycle étant éventuellement substitué par des radicaux organiques ayant au plus 30 atomes de carbone,

* Y est un atome d'oxygène ou de soufre.

* Z est atome de chlore, un groupe OW', $NHR_1$ ou $NR_1$ R (les composés dans la formule desquels Z est $NR_1$ $R_2$ sont préférés lorsqu'on cherche à combattre les maladies des plantes et les composés dans lesquels Z est un atome de chlore, un groupe OW' ou un groupe $NHR_1$ sont particulièrement utiles comme intermédiaires chimiques).

* W' est un atome d'hydrogène ou un radical alkyle inférieur ou un métal alcalin ou alcalinoterreux.

* $R_1$ et $R_2$, identiques ou différents représentent :

- un radical alkyle inférieur ou alkoxy inférieur (l'un au moins des radicaux $R_1$ et $R_2$ étant autre que alkoxy), ou cycloalkyle de 3 à 7 atomes de carbone, ces radicaux étant éventuellement substitués par au moins un atome d'halogène et/ou un groupe hydroxy ou alkoxy inférieur, ou alkoxy (inférieur)-alkyle (inférieur) ou acyloxy (inférieur) ou phényle ou un groupe hétérocyclique de 4 à 6 chaînons comprenant 1 ou 2 hétéroatomes (O, N ou S) ou un groupe amino mono-ou disubstitué par des alkyle inférieurs,
- un radical alkényle ou alkynyle ayant de 3 à 7 atomes de carbone.

* ou bien $R_1$ et $R_2$ forment ensemble, avec l'atome d'azote auquel ils sont rattachés, un hétérocycle, saturé ou insaturé, comprenant de 4 à 7 chaînons (de préférence 5 ou 6) et 1, 2 ou 3 hétéroatomes (O, N ou S), cet hétérocycle pouvant être substitué par des groupes alkyle inférieurs ou alkoxy inférieurs ou hydroxy ou un radical oxo ou un ou plusieurs atomes d'halogène ou un groupe alkyl (inférieur)-alkoxy (inférieur).

* $R_3$, $R_4$ et $R_5$ identiques ou différents, représentent :
* un atome d'hydrogène ou d'halogène, l'un au moins de ces radicaux $R_3$, $R_4$ et $R_5$ étant autre qu'un atome d'hydrogène ou d'halogène
* un groupe amino éventuellement substitué par un ou deux groupes alkyle inférieurs
* un groupe alkyle inférieur ou alkoxy inférieur ou alkyl (inférieur)-alkoxy inférieur ou alkylthio inférieur, éventuellement halogéné ou hydroxylé
* $R_3$ et $R_4$ (en position meta et para) peuvent former ensemble un radical unique divalent à 3 ou 4 chaînons comprenant 1 ou 2 atomes d'oxygène non vicinaux.

42) Composés, selon la revendication 41, caractérisés en ce que le radical Z est un atome de chlore ou un groupe OW′ ou $NHR_1$.

43) Composés, selon l'une des revendications 41 ou 42, caractérisés en ce que les radicaux autres que Z ont l'une des significations données dans les revendications 4 à 22.

44) Composés, selon l'une des revendications 1 à 22, caractérisés en ce que les produits suivants sont exclus des revendications :
* les produits de formule (Ia) dans laquelle Y est l'atome d'oxygène, et en même temps $K_8$ et $K_9$ sont, ou bien -CH=, ou bien l'un est -N= et l'autre -CH=, et en même temps $K_6$ et $K_7$ forment ensemble un cycle carboné insaturé accolé au cycle à 5 chaînons comprenant $K_{10}$,
* les produits de formule (Ib) dans laquelle Y est l'atome d'oxygène et en même temps $K_{10}$ est O, S ou SO ou $SO_2$ ou $NR_{10}$ ($R_{10}$ ayant la signification donnée ci-avant) ou $CH_2$ ou CO et en même temps $K_6$ et $K_7$ forment ensemble un cycle carboné insaturé, accolé au cycle à 5 chaînons comprenant $K_{10}$.

45) Composés, selon l'une des revendications 41 à 44, caractérisés en ce que les produits suivants sont exclus des revendications :
* les produits de formule (Ia) dans laquelle Y est l'atome d'oxygène, et en même temps $K_8$ et $K_9$ sont, ou bien -CH=, ou bien l'un est -N= et l'autre -CH=, et en même temps $K_6$ et $K_7$ forment ensemble un cycle carboné insaturé accolé au cycle à 5 chaînons comprenant $K_{10}$,
* les produits de formule (Ib) dans laquelle Y est l'atome d'oxygène et en même temps $K_{10}$ est O, S ou SO ou $SO_2$ ou $NR_{10}$ ($R_{10}$ ayant la signification donnée ci-avant) ou $CH_2$ ou CO et en même temps $K_6$ et $K_7$ forment ensemble un cycle carboné insaturé, accolé au cycle à 5 chaînons comprenant $K_{10}$.

Office européen des brevets

**RAPPORT PARTIEL DE RECHERCHE EUROPEENNE**
qui selon la règle 45 de la Convention sur le brevet européen est considéré, aux fins de la procédure ultérieure, comme le rapport de recherche européenne

Numéro de la demande

EP 89 42 0320

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | EP-A-0 146 243 (MERCK FROSST CANADA INC.)<br>* Revendications *<br>-- | 1-3 | C 07 D 333/70<br>C 07 D 307/85<br>C 07 D 209/42<br>C 07 D 213/78<br>C 07 D 213/80<br>C 07 D 213/82<br>C 07 D 307/68<br>C 07 D 207/34<br>C 07 D 231/14<br>C 07 D 239/28<br>C 07 D 295/18<br>C 07 C 62/38<br>A 01 N 43/00 |
| X | FR-A-2 272 657 (SCHERICO)<br>* Revendications *<br>-- | 1-4 | |
| X | FR-A-2 151 576 (ANVAR)<br>* Page 4, exemple no. 33; revendications *<br>-- | 1,2 | |
| X | EP-A-0 135 770 (HOFFMANN-LA ROCHE)<br>* Pages 31-33, exemples 11,12,13 *<br>-- | 1 | |
| X | FR-A-1 603 791 (SUMITOMO)<br>* Résumé *<br>-- ./. | 1,40-43 | **DOMAINES TECHNIQUES RECHERCHES (Int. Cl.4)**<br>C 07 D 307/00<br>C 07 D 333/00<br>C 07 D 207/00<br>C 07 D 209/00<br>C 07 D 213/00<br>C 07 D 231/00<br>C 07 D 239/00<br>C 07 D 295/00<br>C 07 C 62/00<br>A 01 N 43/00 |

## RECHERCHE INCOMPLETE

La division de la recherche estime que la presente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches completes:
Revendications ayant fait l'objet de recherches incompletes: 1-45
Revendications n'ayant pas fait l'objet de recherches:
Raison pour la limitation de la recherche:

Recherche limitée aux exemples décrits (Article 84 de la Convention sur le Brevet Européen)

| Lieu de la recherche | Date d'achèvement de la recherche | Examinateur |
|---|---|---|
| La Haye | 07-12-1989 | CHOULY |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1505.1 03 82

Office européen des brevets

**RAPPORT PARTIEL DE RECHERCHE EUROPEENNE**

Numéro de la demande

EP 89 42 0320

-2-

| DOCUMENTS CONSIDERES COMME PERTINENTS | | | CLASSEMENT DE LA DEMANDE (Int Cl 4) |
|---|---|---|---|
| Catégorie | Citation du document avec indication, en cas de besoin, des parties pertinentes | Revendication concernee | |
| X | CHEMICAL ABSTRACTS, vol. 75, no. 1, 5 juillet 1971, page 474, no. 5700m, Columbus, Ohio, US & JP-A-70 39 538 (SUMITOMO CHEMICAL CO., LTD) 12-12-1970<br>* Résumé *<br>-- | 1,40-43 | |
| X | CHEMICAL ABSTRACTS, vol. 89, no. 19, 6 novembre 1978, page 560, no. 163396k, Columbus, Ohio, US & JP-A-78 71 066 (SUMITOMO CHEMICAL CO., LTD.) 24-06-1978<br>* Résumé *<br>-- | 1,41-43 | DOMAINES TECHNIQUES RECHERCHES (Int. Cl.4) |
| X | CHEMICAL ABSTRACTS, vol. 90, no. 9, 25 février 1979, page 503, no. 72053a, Columbus, Ohio, US & JP-A-78 112 872 (SUMITOMO CHEMICAL CO., LTD.) 02-10-1978<br>* Résumé *<br>-- | 1,40-43 | |
| X | BULLETIN DE LA SOCIETE CHIMIQUE DE FRANCE, mars-avril 1974, BSCFAS (3-4), deuxième partie, 'Chimie organique - Biochimie', pages 475-476, Masson et Cie dépositaires, Paris, FR<br>L. RENE et al.: "No. 87. - Réactions induites par le chlorhydrate de pyridine. XVI. - Sur la désalcoylation des dérivés Bz-méthoxylés de nitriles et amides coumariliques (*) (Note de Laboratoire)"<br>* En entier *<br>-- | 1,2,41-43 | |
| X | CHIMIE THERAPEUTIQUE, vol. 8, no. 4, juillet-août 1973, pages 398-411;<br>C. GOLDENBERG et al.: "Recherches dans la série des benzofurannes. LII. Synthèse de dérivés d'hydroxy-5 ou -6 p-hydroxyphényl-2 ou -3 benzofurannes"<br>./. | | |

Office européen des brevets

**RAPPORT PARTIEL DE RECHERCHE EUROPEENNE**

Numero de la demande

EP 89 42 0320

-3-

| DOCUMENTS CONSIDERES COMME PERTINENTS | | | CLASSEMENT DE LA DEMANDE (Int. Cl 4) |
|---|---|---|---|
| Catégorie | Citation du document avec indication, en cas de besoin, des parties pertinentes | Revendication concernee | |
| | * En entier * | 1,2, 40-31 | |
| | -- | | |
| Y | FR-A-2 043 479 (THE DOW CHEMICAL CO.) | | |
| | * Revendications * | 23-39 | |
| | -- | | |
| Y | FR-A-2 206 908 (CONS. ELEKT. IND.) | | |
| | * Revendications * | 23-39 | |
| | -- | | DOMAINES TECHNIQUES RECHERCHES (Int. Cl.4) |
| Y | US-A-3 927 037 (R.A. SCHERRER) | | |
| | * Colonne 1 * | 23-39 | |
| | -- | | |
| P,X | DE-A-3 710 717 (CELAMERCK GmbH & CO KG) | | |
| | * En entier * | 1-45 | |
| | -------- | | |